# EUROPEAN PATENT APPLICATION

(11) **EP 4 700 024 A1**
(43) Date of publication of application: **25.02.2026**
(21) Application number: 24792016.8
(22) Date of filing: 17.04.2024
(51) Int. Cl.: C07D 409/12, C07D 231/38, A61K 31/4155, A61P 35/00

(54) **CYCLOPENTYLPYRAZOLAMINE DERIVATIVE AND USE THEREOF**

(30) Priority: 17.04.2023 CN 202310411874; 04.07.2023 CN 202310815737; 28.09.2023 CN 202311288133; 10.04.2024 CN 202410431164; 13.04.2024 CN 202410445541
(71) Applicant: Chia Tai Tianqing Pharmaceutical Group Co., Ltd., Lianyungang, Jiangsu 222062 (CN)
(72) Inventor: GAO, Yong, Lianyungang, Jiangsu 222062 (CN); JI, Yanpeng, Lianyungang, Jiangsu 222062 (CN); ZHAO, Damin, Lianyungang, Jiangsu 222062 (CN); FAN, Lu, Lianyungang, Jiangsu 222062 (CN); WANG, Jinan, Lianyungang, Jiangsu 222062 (CN); CAO, Bin, Shanghai 200131 (CN); ZHANG, Yinsheng, Lianyungang, Jiangsu 222062 (CN)
(74) Representative: Grünecker Patent- und Rechtsanwälte PartG mbB
(86) International application number: PCT/CN2024/088161
(87) International publication number: WO 2024/217431

(57) **Abstract**

The present application belongs to the field of medicinal chemistry, relates to a cyclopentylpyrazolamine derivative and the use thereof, and specifically relates to a compound of formula (I), an isomer thereof, or a pharmaceutically acceptable salt thereof.

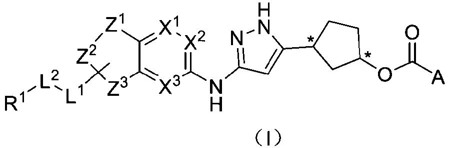

## Description

### CROSS-REFERENCE TO RELATED APPLICATIONS

The present application claims the priority and benefit to 1) Chinese Patent Application No. 202310411874.5 filed with the China National Intellectual Property Administration on Apr. 17, 2023, 2) Chinese Patent Application No. 202310815737.8 filed with the China National Intellectual Property Administration on Jul. 04, 2023, 3) Chinese Patent Application No. 202311288133.9 filed with the China National Intellectual Property Administration on Sep. 28, 2023, 4) Chinese Patent Application No. 202410431164.3 filed with the China National Intellectual Property Administration on Apr. 10, 2024, and 5) Chinese Patent Application No. 202410445541.9 filed with the China National Intellectual Property Administration on Apr. 13, 2024, which are incorporated herein in their entirety.

### TECHNICAL FIELD

The present disclosure relates to the field of pharmaceutical chemistry, particularly, to a cyclopentylpyrazolamine derivative and use thereof, and more particularly, to a compound of formula (I), an isomer thereof, or a pharmaceutically acceptable salt thereof.

### BACKGROUND

Cyclin-dependent kinases (CDKs) belong to the serine/threonine protein kinase family, and are directly involved in the regulation of the cell cycle to promote the ordered growth, proliferation, and apoptosis of cells. The cell division cycle is divided into 4 phases: G1, S, G2, and M phases, with the G1-S regulatory point being the most important, and its regulation being closely related to CDKs. CDKs form protein kinase complexes through binding to cyclins, and the complexes catalyze substrate phosphorylation, control cell cycle progression, and complete DNA replication and mitosis in sequence, leading to cell division and proliferation. The cell division cycle is regulated by two modes: inhibition and promotion, both of which are normally in a dynamic balance. However, when the signal for promoting cell proliferation is enhanced or the signal for inhibiting cell proliferation is weakened, the balance is lost, and cell proliferation is out of control, resulting in the development of tumors. Studies have shown that the overexpression of CDKs occurs in many malignancies.

Studies have shown that the CDKs directly involved in the cell cycle regulation mainly include CDK1, CDK2, CDK4, and CDK6, which play a key role in regulating the cell cycle. CDK2 is a member of the CDK family. It is a cyclin-dependent kinase essential for the completion of the G1 phase and the transition from the G1 phase to the S phase in cell mitosis. In the late G1 phase, CDK2 binds to Cyclin E and becomes activated, promoting the continuous phosphorylation of pRb, and ensuring that cells smoothly pass through the G1 phase and enter the S phase. The inactivation of E2F is a primary condition for the completion of the S phase. At the early stage of the S phase, CDK2 binds to Cyclin A to inactivate the E2F transcription factor, thereby facilitating the smooth completion of the S phase in cells. However, the sustained activity of E2F will lead to cell apoptosis. Therefore, selectively inhibiting the activity of CDK2/Cyclin A to increase the concentration of E2F can cause the cell cycle to stall in the S phase or trigger apoptosis, thereby achieving the purpose of treating tumor cells.

### SUMMARY

The present disclosure provides a compound of formula (I), an isomer thereof, or a pharmaceutically acceptable salt thereof, 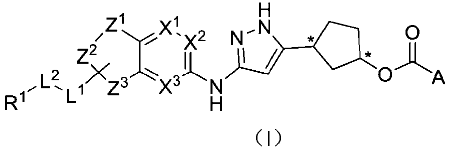 wherein
X¹, X², and X³ are each independently selected from the group consisting of N and C(R^{a});
Z¹, Z², and Z³ are each independently selected from the group consisting of O, S, N(R^{b}), S(O), S(O)₂, C(O), and C(R^{a}R^{a});
L¹ and L² are each independently selected from the group consisting of a single bond, -N(R^{b})-, -N=, -O-, -S-, - (CH₂)ₘ-, -(CD₂)ₘ-, -(CHD)ₘ-, -C(=O)-, -S(=O) -, -S(=O)₂ -, -S(=O)(=N(R^{b}))-, -S(=O)(R^{c})-, -P(=O)(R^{c})-, and - P(=O)(NR^{b}R^{b})-;
R¹ is selected from the group consisting of H, deuterium, C₁₋₃ alkyl, C₁₋₃ alkoxy, -NH₂, -NH(C₁₋₃ alkyl), -N(C₁₋₃ alkyl)₂, C₃₋₆ cycloalkyl, C₃₋₆ cycloalkenyl, 3- to 6-membered heterocyclyl, phenyl, and 5- to 6-membered heteroaryl, the C₁₋₃ alkyl or C₁₋₃ alkoxy being optionally independently substituted with 1, 2, or 3 R^{d}, and the C₃₋₆ cycloalkyl, C₃₋₆ cycloalkenyl, 3- to 6-membered heterocyclyl, phenyl, or 5- to 6-membered heteroaryl being optionally independently substituted with 1 or more R^{e};
A is selected from the group consisting of 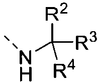 and 3- to 6-membered heterocyclyl containing 1 or 2 N atoms, the 3- to 6-membered heterocyclyl being optionally independently substituted with 1 or more R^{z};
R², R³, and R⁴ are each independently selected from the group consisting of H, deuterium, halogen, OH, CN, NH₂, C₁₋₃ alkyl, C₁₋₃ alkoxy, C₃₋₆ cycloalkyl, C₃₋₆ cycloalkenyl, and 3- to 6-membered heterocyclyl, the C₁₋₃ alkyl, C₁₋₃ alkoxy, C₃₋₆ cycloalkyl, C₃₋₆ cycloalkenyl, or 3- to 6-membered heterocyclyl being optionally independently substituted with 1 or more R^{z};
or, R² and R³, together with the carbon atom linked thereto, form C₃₋₆ cycloalkyl, C₃₋₆ cycloalkenyl, or 3- to 6-membered heterocyclyl, and R⁴ is selected from the group consisting of H, deuterium, halogen, OH, CN, NH₂, C₁₋₃ alkyl, and C₁₋₃ alkoxy, the C₁₋₃ alkyl, C₁₋₃ alkoxy, C₃₋₆ cycloalkyl, C₃₋₆ cycloalkenyl, or 3- to 6-membered heterocyclyl being optionally independently substituted with 1 or more R^{z};
or, R², R³, and R⁴, together with the carbon atom linked thereto, form C₅₋₈ bicycloalkyl or 5- to 8-membered heterobicycloalkyl, the C₅₋₈ bicycloalkyl or 5- to 8-membered heterobicycloalkyl being optionally independently substituted with 1 or more R^{z};
R^{a} is each independently selected from the group consisting of hydrogen, deuterium, halogen, OH, CN, NH₂, COOH, NO₂, C₁₋₃ alkyl, C₁₋₃ alkoxy, C₁₋₃ alkoxy C₁₋₃ alkylene, C₁₋₃ alkylamino, and (C₁₋₃ alkyl)₂ amino, the C₁₋₃ alkyl or C₁₋₃ alkoxy being optionally independently substituted with 1 or more R^{x};
or, the two R^{a} in C(R^{a}R^{a}), together with the carbon atom linked thereto, form C₃₋₆ cycloalkyl, C₃₋₆ cycloalkenyl, or 3- to 6-membered heterocyclyl, the C₃₋₆ cycloalkyl, C₃₋₆ cycloalkenyl, or 3- to 6-membered heterocyclyl being optionally independently substituted with 1 or more R^{y};
R^{b} is each independently selected from the group consisting of H, C₁₋₃ alkyl, C₁₋₃ alkoxy, -C(=O)H, -S(=O)₂C₁₋₃ alkyl, C₃₋₆ cycloalkyl, C₃₋₆ cycloalkenyl, and 3- to 6-membered heterocyclyl, the C₁₋₃ alkyl or C₁₋₃ alkoxy being optionally independently substituted with 1, 2, or 3 R^{x}, and the C₃₋₆ cycloalkyl, C₃₋₆ cycloalkenyl, or 3- to 6-membered heterocyclyl being optionally independently substituted with 1 or more R^{y};
R^{c} is selected from the group consisting of halogen, OH, CN, and C₁₋₃ alkyl, the C₁₋₃ alkyl being optionally independently substituted with 1 or more R^{x};
R^{d} is each independently selected from the group consisting of deuterium, halogen, OH, CN, and NH₂;
R^{e} is each independently selected from the group consisting of deuterium, halogen, OH, CN, NH₂, =O, C₁₋₃ alkyl, and C₁₋₃ alkoxy, the C₁₋₃ alkyl or C₁₋₃ alkoxy being optionally independently substituted with 1 or more R^{x};
R^{x} is each independently selected from the group consisting of deuterium, halogen, OH, CN, and NH₂;
R^{y} is each independently selected from the group consisting of deuterium, halogen, OH, CN, NH₂, =O, C₁₋₃ alkyl, and C₁₋₃ alkoxy, the C₁₋₃ alkyl or C₁₋₃ alkoxy being optionally independently substituted with 1 or more substituents selected from the group consisting of deuterium, halogen, OH, CN, and NH₂;
R^{z} is each independently selected from the group consisting of deuterium, halogen, OH, CN, NH₂, C₁₋₃ alkyl, and C₁₋₃ alkoxy, the C₁₋₃ alkyl or C₁₋₃ alkoxy being optionally independently substituted with 1 or more substituents selected from the group consisting of deuterium, halogen, OH, CN, and NH₂;
m is selected from the group consisting of 1, 2, and 3;
the carbon atom with "*" is a chiral carbon atom present in a form of a single (R) or (S) enantiomer or in a form enriched with one enantiomer;
or, L¹, L², R¹, R², R³, R⁴, R^{a}, R^{b}, R^{c}, R^{d}, R^{e}, R^{x}, R^{y}, or R^{z} is each independently optionally substituted with one or more substituents.

In some embodiments of the present disclosure, R^{a}, R^{b}, R^{c}, R^{d}, R^{e}, R^{x}, R^{y}, or R^{z} is each independently optionally substituted with one or more substituents.

In some embodiments of the present disclosure, R^{d}, R^{x}, R^{y}, or R^{z} is each independently optionally substituted with one or more substituents.

In some other embodiments of the present disclosure, R¹, R², R³, R⁴, R^{a}, R^{d}, R^{e}, R^{x}, R^{y}, or R^{z} is each independently optionally substituted with one or more deuterium.

In some other embodiments of the present disclosure, R¹, R², R³, R⁴, R^{a}, R^{d}, R^{e}, R^{x}, R^{y}, or R^{z} is each independently optionally substituted with 1, 2, 3, 4, or 5 deuterium, or is each independently optionally substituted with 3 or 5 deuterium.

In some embodiments of the present disclosure, at least one of R¹, R², R³, R⁴, R^{a}, R^{d}, R^{e}, R^{x}, R^{y}, and R^{z} is a deuterium atom-containing group.

In some embodiments of the present disclosure, the "substituted with 1 or more" is each independently selected from a substitution with 1, 2, 3, 4, 5, or 6.

In some embodiments of the present disclosure, the "substituted with 1 or more" is each independently selected from a substitution with 1, 2, 3, 4, or 5.

In some embodiments of the present disclosure, the "substituted with 1 or more" is each independently selected from a substitution with 1, 2, 3, or 4.

In some embodiments of the present disclosure, the "substituted with 1 or more" is each independently selected from a substitution with 1, 2, or 3.

In some embodiments of the present disclosure, each "heterocyclyl" described herein may be independently selected from the group consisting of "heterocycloalkyl" and "heterocycloalkenyl".

In some embodiments of the present disclosure, each "3- to 6-membered heterocyclyl" described herein may be independently selected from the group consisting of "3- to 6-membered heterocycloalkyl" and "3- to 6-membered heterocycloalkenyl".

In some embodiments of the present disclosure, each "3- to 6-membered heterocyclyl" described herein may be independently selected from "3- to 6-membered heterocycloalkyl".

In some embodiments of the present disclosure, L¹, L², R¹, R², R³, R⁴, R^{a}, R^{b}, R^{c}, R^{d}, R^{e}, R^{x}, R^{y}, or R^{z} is each independently optionally substituted with one or more of the following substituents: deuterium, -OH, -SH, halogen, -NH₂, nitro, nitroso, -CN, an azido group, a sulfoxide group, a sulfone group, a sulfonamide group, carboxyl, a carboxaldehyde group, an imine group, alkyl, haloalkyl, cycloalkyl, halocycloalkyl, alkenyl, haloalkenyl, cycloalkenyl, halocycloalkenyl, alkynyl, haloalkynyl, cycloalkynyl, halocycloalkynyl, heteroalkyl, haloheteroalkyl, alkoxy, alkylthio, aryl, aryloxy, arylthio, arylalkyl, arylalkyloxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkyl, heterocyclylalkoxy, heterocyclylalkylthio, acyl, acyloxy, a carbamate group, an amide group, a ureido group, an epoxy group, an ester group, or the like, the group being optionally substituted with one or more substituents selected from the group consisting of: oxo, hydroxy, amino, nitro, halogen, cyano, alkyl, alkenyl, alkynyl, alkoxy, haloalkoxy, alkylamino, dialkylamino, haloalkylamino, halodialkylamino, carboxyl, - C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkyl, cycloalkyloxy, heterocyclyl, heterocyclylalkyl, heterocyclyloxy, heterocycloalkyl, heterocycloalkylalkyl, heterocycloalkyloxy, heteroaryl, heteroarylalkyl, heteroaryloxy, aryl, arylalkyl, and aryloxy.

In some embodiments of the present disclosure, X¹, X², and X³ are selected from C(R^{a}).

In some embodiments of the present disclosure, one of X¹, X², and X³ is selected from N.

In some embodiments of the present disclosure, X¹ is selected from N, and X² and X³ are selected from C(R^{a}).

In some embodiments of the present disclosure, X² is selected from N, and X¹ and X³ are selected from C(R^{a}).

In some embodiments of the present disclosure, X³ is selected from N, and X¹ and X² are selected from C(R^{a}).

In some embodiments of the present disclosure, R^{a} is each independently selected from the group consisting of hydrogen, deuterium, halogen, OH, CN, NH₂, COOH, NO₂, C₁₋₃ alkyl, C₁₋₃ alkoxy, C₁₋₃ alkoxy C₁₋₃ alkylene, C₁₋₃ alkylamino, and (C₁₋₃ alkyl)₂ amino, the C₁₋₃ alkyl or C₁₋₃ alkoxy being optionally independently substituted with 1 or more R^{x};
or, the two R^{a} in C(R^{a}R^{a}), together with the carbon atom linked thereto, form C₃₋₆ cycloalkyl or 3- to 6-membered heterocycloalkyl, the C₃₋₆ cycloalkyl or 3- to 6-membered heterocycloalkyl being optionally independently substituted with 1 or more R^{y}.

In some embodiments of the present disclosure, R^{a} is each independently selected from the group consisting of hydrogen, F, Cl, Br, I, CN, C₁₋₃ alkyl, C₁₋₃ alkoxy, and C₁₋₃ alkoxy C₁₋₃ alkylene, the C₁₋₃ alkyl or C₁₋₃ alkoxy being optionally independently substituted with 1, 2, or 3 R^{x}.

In some other embodiments of the present disclosure, R^{a} is each independently selected from the group consisting of hydrogen, F, C₁₋₃ alkyl, C₁₋₃ alkoxy, and C₁₋₃ alkoxy C₁₋₃ alkylene, the C₁₋₃ alkyl or C₁₋₃ alkoxy being optionally independently substituted with 1, 2, or 3 R^{x}.

In some other embodiments of the present disclosure, R^{a} is each independently selected from the group consisting of hydrogen, F, methyl, ethyl, methoxy, ethoxy, methoxymethyl, methoxyethyl, ethoxymethyl, and ethoxyethyl, the methyl, ethyl, methoxy, ethoxy, methoxymethyl, methoxyethyl, ethoxymethyl, or ethoxyethyl being optionally independently substituted with 1, 2, or 3 R^{x}.

In some embodiments of the present disclosure, R^{a} is each independently selected from the group consisting of hydrogen, F, methyl, and methoxymethyl, the methyl or methoxymethyl being optionally independently substituted with 1, 2, or 3 R^{x}.

In some embodiments of the present disclosure, R^{a} is each independently selected from the group consisting of hydrogen, F, methyl, and methoxymethyl.

In some embodiments of the present disclosure, R^{a} is each independently selected from the group consisting of hydrogen, F, and methoxymethyl.

In some embodiments of the present disclosure, R^{a} is each independently selected from the group consisting of hydrogen and methyl.

In some embodiments of the present disclosure, R^{a} is each independently selected from hydrogen.

In some embodiments of the present disclosure, X¹, X², and X³ are selected from CH.

In some embodiments of the present disclosure, X¹ is selected from C(R^{a}), and X² and X³ are selected from CH.

In some embodiments of the present disclosure, X¹ is selected from C(F), and X² and X³ are selected from CH.

In some embodiments of the present disclosure, X¹ is selected from C(CH₂OCH₃), and X² and X³ are selected from CH.

In some embodiments of the present disclosure, Z¹, Z², and Z³ are each independently selected from the group consisting of S(O), S(O)₂, and C(R^{a}R^{a}).

In some embodiments of the present disclosure, Z² is selected from the group consisting of S(O) and S(O)₂.

In some embodiments of the present disclosure, Z² is selected from the group consisting of S(O) and S(O)₂, and Z¹ and Z³ are each independently selected from C(R^{a}R^{a}).

In some embodiments of the present disclosure, Z² is selected from the group consisting of S(O) and S(O)₂, and Z¹ and Z³ are each independently selected from the group consisting of CH₂ and CH(CH₃).

In some embodiments of the present disclosure, Z² is selected from S(O)₂, and Z¹ and Z³ are selected from CH₂.

In some embodiments of the present disclosure, Z² is selected from S(O)₂, Z¹ is selected from CH(CH₃), and Z³ is selected from CH₂.

In some embodiments of the present disclosure, Z² is selected from S(O)₂, and Z¹ and Z³ are selected from CH(CH₃). In some embodiments of the present disclosure, Z² is selected from S(O), and Z¹ and Z³ are selected from CH₂.

In some embodiments of the present disclosure, the two R^{a} in C(R^{a}R^{a}), together with the carbon atom linked thereto, form cyclopropyl, cyclobutyl, oxiranyl, aziridinyl, oxetanyl, thietanyl, or azetidinyl, the cyclopropyl, cyclobutyl, oxiranyl, aziridinyl, oxetanyl, thietanyl, or azetidinyl being optionally independently substituted with 1 or more R^{y}. In some embodiments of the present disclosure, the two R^{a} in C(R^{a}R^{a}), together with the carbon atom linked thereto, form cyclopropyl or azetidinyl.

In some embodiments of the present disclosure, L¹ is selected from the group consisting of a single bond, -N(R_{b})-, -O-, -S(=O)₂-, -S-, -(CH₂)ₘ-, and -C(=O)-.

In some embodiments of the present disclosure, L¹ is selected from the group consisting of a single bond, -NH-, - N(C₁₋₃ alkyl)-, -S(=O)₂-, -CH₂-, -CH₂-CH₂-, -CH₂-CH₂-CH₂-, and -C(=O)-.

In some embodiments of the present disclosure, L¹ is selected from the group consisting of a single bond, -NH-, - N(CH₃)-, -S(=O)₂-, -CH₂-, -CH₂-CH₂-, -CH₂-CH₂-CH₂-, and -C(=O)-.

In some embodiments of the present disclosure, L¹ is selected from the group consisting of a single bond, -NH-, - N(CH₃)-, -S(=O)₂-, -CH₂-, and -C(=O)-.

In some embodiments of the present disclosure, L¹ is selected from the group consisting of a single bond and -NH-. In some embodiments of the present disclosure, L¹ is selected from a single bond.

In some embodiments of the present disclosure, L² is selected from the group consisting of a single bond, -NH-, - N(C₁₋₃ alkyl)-, -CH₂-, -CH₂-CH₂-, -CH₂-CH₂-CH₂-, -C(=O)-, -S(=O)-, -S(=O)₂-, and -S(=O)(C₁₋₃ alkyl)-.

In some embodiments of the present disclosure, L² is selected from the group consisting of a single bond, -NH-, - N(CH₃)-, -CH₂-, -CH₂-CH₂-, -CH₂-CH₂-CH₂-, -C(=O)-, -S(=O)-, -S(=O)₂-, and -S(=O)(CH₃)-.

In some embodiments of the present disclosure, L² is selected from the group consisting of a single bond, -NH-, and -S(=O)₂-.

In some embodiments of the present disclosure, L² is selected from the group consisting of a single bond and - S(=O)₂-.

In some embodiments of the present disclosure, L² is selected from a single bond.

In some embodiments of the present disclosure, -L²-L¹- is selected from the group consisting of a single bond, - NH-, -N(CH₃)-, -C(=O)-, -CH₂-, -CH₂-CH₂-, -S(=O)₂-, -CH₂-NH-, -NH-CH₂-, -CH₂-N(CH₃)-, -N(CH₃)-CH₂-, - C(=O)-CH₂-, -CH₂-C(=O)-, -C(=O)-NH-, -NH-C(=O)-, -NH-S(=O)₂-, -S(=O)₂-NH-, -N(CH₃)-S(=O)₂-, -S(=O)₂-N(CH₃)-, -N(CH₃)-C(=O)-, -C(=O)-N(CH₃)-, -N(CH₃)-CH₂-CH₂-, -CH₂-CH₂-N(CH₃)-, -NH-CH₂-CH₂-, and -CH₂-CH₂-NH-.

In some embodiments of the present disclosure, -L²-L¹- is selected from the group consisting of a single bond, - NH-, -CH₂-, -NH-S(=O)₂-, and -S(=O)₂-NH-.

In some embodiments of the present disclosure, -L²-L¹- is selected from the group consisting of a single bond, - NH-, -CH₂-, and -S(=O)₂-NH-.

In some embodiments of the present disclosure, -L²-L¹- is selected from the group consisting of a single bond and -S(=O)₂-NH-.

In some embodiments of the present disclosure, -L²-L¹- is selected from a single bond.

In some embodiments of the present disclosure, R¹ is selected from the group consisting of H, deuterium, C₁₋₃ alkyl, C₁₋₃ alkoxy, -NH₂, -NH(C₁₋₃ alkyl), -N(C₁₋₃ alkyl)₂, C₃₋₆ cycloalkyl, 3- to 6-membered heterocycloalkyl, phenyl, and 5- to 6-membered heteroaryl, the C₁₋₃ alkyl or C₁₋₃ alkoxy being optionally independently substituted with 1, 2, or 3 R^{d}, and the C₃₋₆ cycloalkyl, 3- to 6-membered heterocycloalkyl, phenyl, or 5- to 6-membered heteroaryl being optionally independently substituted with 1 or more R^{e}.

In some embodiments of the present disclosure, R¹ is selected from the group consisting of H, C₁₋₃ alkyl, C₁₋₃ alkoxy, -NH₂, -NH(C₁₋₃ alkyl), cyclopropyl, cyclobutyl, cyclopentyl, 4- to 6-membered heterocycloalkyl containing 1 or 2 atoms selected from the group consisting of N, O, and S, and 5- to 6-membered heteroaryl containing 1 or 2 N atoms, the C₁₋₃ alkyl or C₁₋₃ alkoxy being optionally independently substituted with 1, 2, or 3 R^{d}, and the 4- to 6-membered heterocycloalkyl or 5- to 6-membered heteroaryl being optionally independently substituted with 1, 2, or 3 R^{e}.

In some embodiments of the present disclosure, R¹ is selected from the group consisting of H, methyl, ethyl, methoxy, -NH₂, -NH(CH₃), -NH(CH₂CH₃), cyclopropyl, cyclobutyl, cyclopentyl, 4- to 6-membered heterocycloalkyl containing 1 or 2 atoms selected from the group consisting of N, O, and S, and 5- to 6-membered heteroaryl containing 1 or 2 N atoms, the methyl, ethyl, or methoxy being optionally independently substituted with 1, 2, or 3 R^{d}, and the 4- to 6-membered heterocycloalkyl or 5- to 6-membered heteroaryl being optionally independently substituted with 1, 2, or 3 R^{e}.

In some embodiments of the present disclosure, R¹ is selected from the group consisting of H, methyl, methoxy, - NH₂, -NH(CH₃), -NH(CH₂CH₃), cyclopropyl, oxetanyl, pyrazolidinyl, isothiazolidinyl, isoxazolidinyl, imidazolyl, oxazolyl, and pyrimidinyl, the methyl, ethyl, or methoxy being optionally independently substituted with 1, 2, or 3 R^{d}, and the cyclopropyl, oxetanyl, pyrazolidinyl, isothiazolidinyl, isoxazolidinyl, imidazolyl, oxazolyl, or pyrimidinyl being optionally independently substituted with 1, 2, or 3 R^{e}.

In some embodiments of the present disclosure, R¹ is selected from the group consisting of H and methyl.

In some embodiments of the present disclosure, R¹ is selected from H.

In some embodiments of the present disclosure, the structural unit 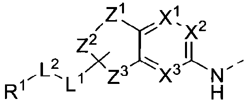 is selected from the group consisting of

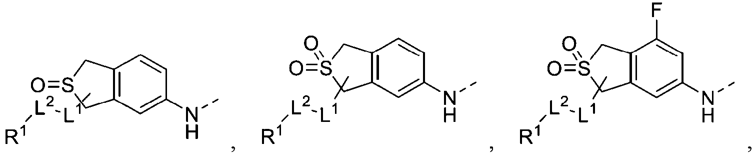

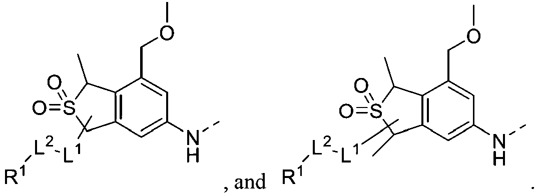

In some embodiments of the present disclosure, the structural unit 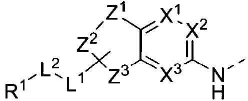 is selected from the group consisting of

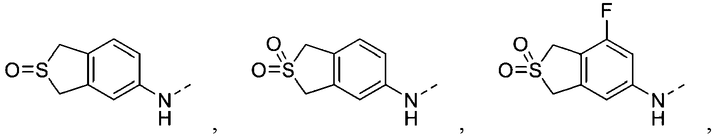

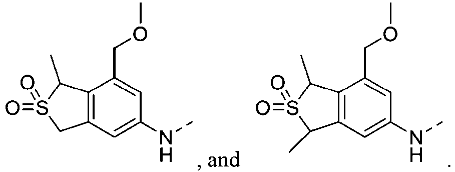

In some embodiments of the present disclosure, the structural unit 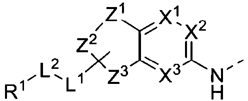 is selected from the group consisting of

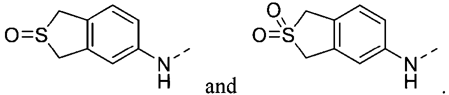

In some embodiments of the present disclosure, R^{d} is each independently selected from the group consisting of F, Cl, Br, I, and OH.

In some embodiments of the present disclosure, R^{d} is each independently selected from the group consisting of F and OH.

In some embodiments of the present disclosure, R^{e} is each independently selected from the group consisting of F, Cl, Br, I, =O, and C₁₋₃ alkyl, the C₁₋₃ alkyl being optionally independently substituted with 1, 2, or 3 R^{x}.

In some embodiments of the present disclosure, R^{x} is each independently selected from halogen.

In some embodiments of the present disclosure, R^{x} is each independently selected from F.

In some embodiments of the present disclosure, R^{e} is each independently selected from the group consisting of F, Cl, Br, I, =O, and C₁₋₃ alkyl.

In some other embodiments of the present disclosure, R^{e} is each independently selected from the group consisting of F and methyl.

In some other embodiments of the present disclosure, R^{y} is each independently selected from halogen.

In some other embodiments of the present disclosure, R^{y} is each independently selected from F.

In some embodiments of the present disclosure, R^{b} is each independently selected from the group consisting of H, C₁₋₃ alkyl, C₁₋₃ alkoxy, -C(=O)H, -S(=O)₂C₁₋₃ alkyl, C₃₋₆ cycloalkyl, and 3- to 6-membered heterocycloalkyl, the C₁₋₃ alkyl or C₁₋₃ alkoxy being optionally independently substituted with 1, 2, or 3 R^{x}, and the C₃₋₆ cycloalkyl or 3- to 6-membered heterocycloalkyl being optionally independently substituted with 1 or more R^{y}.

In some embodiments of the present disclosure, R^{b} is each independently selected from the group consisting of H, C₁₋₃ alkyl, C₁₋₃ alkoxy, C₃₋₆ cycloalkyl, and 3- to 6-membered heterocycloalkyl, the C₁₋₃ alkyl or C₁₋₃ alkoxy being optionally independently substituted with 1, 2, or 3 R^{x}, and the C₃₋₆ cycloalkyl or 3- to 6-membered heterocycloalkyl being optionally independently substituted with 1, 2, or 3 R^{y}.

In some other embodiments of the present disclosure, R^{b} is each independently selected from the group consisting of H, -C(=O)H, -S(=O)₂CH₃, and methyl, the methyl being optionally independently substituted with 1, 2, or 3 R^{x}. In some embodiments of the present disclosure, R^{b} is each independently selected from the group consisting of H and C₁₋₃ alkyl, the C₁₋₃ alkyl being optionally independently substituted with 1, 2, or 3 R^{x}.

In some embodiments of the present disclosure, R^{b} is each independently selected from the group consisting of H and methyl, the methyl being optionally independently substituted with 1, 2, or 3 R^{x}.

In some embodiments of the present disclosure, R^{b} is each independently selected from the group consisting of H and methyl.

In some embodiments of the present disclosure, R^{c} is selected from C₁₋₃ alkyl, the C₁₋₃ alkyl being optionally independently substituted with 1, 2, or 3 R^{x}.

In some embodiments of the present disclosure, R^{c} is selected from methyl, the methyl being optionally independently substituted with 1, 2, or 3 R^{x}.

In some embodiments of the present disclosure, R^{c} is selected from methyl.

In some embodiments of the present disclosure, m is selected from the group consisting of 1 and 2.

In some embodiments of the present disclosure, A is selected from

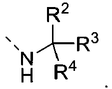

In some embodiments of the present disclosure, R², R³, and R⁴ are each independently selected from the group consisting of H, deuterium, halogen, OH, CN, NH₂, C₁₋₃ alkyl, C₁₋₃ alkoxy, C₃₋₆ cycloalkyl, and 3- to 6-membered heterocycloalkyl, the C₁₋₃ alkyl, C₁₋₃ alkoxy, C₃₋₆ cycloalkyl, or 3- to 6-membered heterocycloalkyl being optionally independently substituted with 1 or more R^{z};
or, R² and R³, together with the carbon atom linked thereto, form C₃₋₆ cycloalkyl or 3- to 6-membered heterocycloalkyl, and R⁴ is selected from the group consisting of H, deuterium, halogen, OH, CN, NH₂, C₁₋₃ alkyl, and C₁₋₃ alkoxy, the C₁₋₃ alkyl, C₁₋₃ alkoxy, C₃₋₆ cycloalkyl, or 3- to 6-membered heterocycloalkyl being optionally independently substituted with 1 or more R^{z};
or, R², R³, and R⁴, together with the carbon atom linked thereto, form C₅₋₈ bicycloalkyl or 5- to 8-membered heterobicycloalkyl, the C₅₋₈ bicycloalkyl or 5- to 8-membered heterobicycloalkyl being optionally independently substituted with 1 or more R^{z}.

In some embodiments of the present disclosure, R², R³, and R⁴ are each independently selected from the group consisting of H, deuterium, C₁₋₃ alkyl, and C₃₋₆ cycloalkyl, the C₁₋₃ alkyl or C₃₋₆ cycloalkyl being optionally independently substituted with 1, 2, or 3 R^{z}.

In some embodiments of the present disclosure, R², R³, and R⁴ are each independently selected from the group consisting of H, deuterium, methyl, ethyl, and cyclopropyl, the methyl, ethyl, or cyclopropyl being optionally independently substituted with 1, 2, or 3 R².

In some other embodiments of the present disclosure, R^{z} is each independently selected from the group consisting of deuterium, halogen, OH, C₁₋₃ alkyl, and C₁₋₃ alkoxy, the C₁₋₃ alkyl or C₁₋₃ alkoxy being optionally independently substituted with 1 or more substituents selected from the group consisting of deuterium, halogen, OH, CN, and NH₂. In some embodiments of the present disclosure, R^{z} is each independently selected from the group consisting of deuterium, halogen, OH, and C₁₋₃ alkyl. In some other embodiments of the present disclosure, R^{z} is each independently selected from C₁₋₃ alkoxy.

In some embodiments of the present disclosure, R^{z} is each independently selected from the group consisting of halogen, OH, and methyl. In some other embodiments of the present disclosure, R^{z} is each independently selected from methoxy.

In some embodiments of the present disclosure, R^{z} is each independently selected from the group consisting of F, OH, methyl, and methoxy.

In some embodiments of the present disclosure, R^{z} is each independently selected from the group consisting of F, OH, and methyl.

In some embodiments of the present disclosure, R^{z} is each independently selected from the group consisting of F and OH.

In some embodiments of the present disclosure, R^{z} is each independently selected from methyl.

In some embodiments of the present disclosure, R², R³, and R⁴ are each independently selected from the group consisting of H, deuterium, methyl, -CD₃, difluoromethyl, trifluoromethyl, ethyl, 2-hydroxyethyl, and cyclopropyl. In some other embodiments of the present disclosure, R², R³, and R⁴ are each independently selected from the group consisting of H, methyl, difluoromethyl, trifluoromethyl, 2-hydroxyethyl, and cyclopropyl.

In some embodiments of the present disclosure, at least one of R², R³, and R⁴ is selected from H.

In some other embodiments of the present disclosure, R² is selected from the group consisting of H and methyl, and R³ and R⁴ are each independently selected from the group consisting of H, methyl, difluoromethyl, trifluoromethyl, 2-hydroxyethyl, and cyclopropyl.

In some other embodiments of the present disclosure, R² is selected from the group consisting of H and methyl, R³ is selected from the group consisting of H and methyl, and R⁴ is selected from the group consisting of H, methyl, difluoromethyl, trifluoromethyl, 2-hydroxyethyl, and cyclopropyl.

In some embodiments of the present disclosure, R², R³, and R⁴ are H.

In some other embodiments of the present disclosure, R² is selected from H, R³ is selected from H, R⁴ is selected from the group consisting of H, methyl, difluoromethyl, trifluoromethyl, 2-hydroxyethyl, and cyclopropyl.

In some other embodiments of the present disclosure, R² is selected from H, R³ is selected from methyl, and R⁴ is selected from the group consisting of H, methyl, difluoromethyl, trifluoromethyl, 2-hydroxyethyl, and cyclopropyl. In some other embodiments of the present disclosure, R² is selected from methyl, R³ is selected from methyl, and R⁴ is selected from the group consisting of H, methyl, difluoromethyl, trifluoromethyl, 2-hydroxyethyl, and cyclopropyl.

In some embodiments of the present disclosure, R² and R³, together with the carbon atom linked thereto, form C₃₋₄ cycloalkyl or 3- to 4-membered heterocycloalkyl, and R⁴ is selected from the group consisting of H and C₁₋₃ alkyl, the C₁₋₃ alkyl, C₃₋₄ cycloalkyl or 3- to 4-membered heterocycloalkyl being optionally independently substituted with 1,2, or 3 R^{z}.

In some embodiments of the present disclosure, R² and R³, together with the carbon atom linked thereto, form cyclopropyl, oxiranyl, or aziridinyl, and R⁴ is selected from the group consisting of H, methyl, and ethyl, the methyl, ethyl, cyclopropyl, oxiranyl, or aziridinyl being optionally independently substituted with 1, 2, or 3 R^{z}. In some other embodiments of the present disclosure, R² and R³, together with the carbon atom linked thereto, form cyclobutyl, the cyclobutyl being optionally independently substituted with 1, 2, or 3 R^{z}.

In some embodiments of the present disclosure, R² and R³, together with the carbon atom linked thereto, form cyclopropyl, and R⁴ is selected from the group consisting of H and methyl, the cyclopropyl being optionally independently substituted with 1, 2, or 3 R^{z}. In some other embodiments of the present disclosure, R² and R³, together with the carbon atom linked thereto, form cyclobutyl, and R⁴ is selected from the group consisting of H and methyl, the cyclobutyl being optionally independently substituted with 1, 2, or 3 R^{z}.

In some embodiments of the present disclosure, R² and R³, together with the carbon atom linked thereto, form cyclopropyl, and R⁴ is selected from the group consisting of H and methyl, the cyclopropyl being optionally independently substituted with 1, 2, or 3 F. In some other embodiments of the present disclosure, R² and R³, together with the carbon atom linked thereto, form cyclobutyl, and R⁴ is selected from the group consisting of H and methyl, the cyclobutyl being optionally independently substituted with 1, 2, or 3 methoxy.

In some embodiments of the present disclosure, R² and R³, together with the carbon atom linked thereto, form cyclopropyl or 2,2-difluorocyclopropyl, and R⁴ is selected from the group consisting of H and methyl. In some other embodiments of the present disclosure, R² and R³, together with the carbon atom linked thereto, form 2-fluorocyclopropyl or 3-methoxycyclobutyl, and R⁴ is selected from the group consisting of H and methyl.

In some embodiments of the present disclosure, R² and R³, together with the carbon atom linked thereto, form cyclopropyl, and R⁴ is selected from H. In some other embodiments of the present disclosure, R² and R³, together with the carbon atom linked thereto, form 2-fluorocyclopropyl or 3-methoxycyclobutyl, and R⁴ is selected from H. In some embodiments of the present disclosure, R², R³, and R⁴, together with the carbon atom linked thereto, form C₅₋₈ bicycloalkyl or 5- to 8-membered heterobicycloalkyl, the C₅₋₈ bicycloalkyl or 5- to 8-membered heterobicycloalkyl being a bridged cyclyl, and the C₅₋₈ bicycloalkyl or 5- to 8-membered heterobicycloalkyl being optionally independently substituted with 1 or more R^{z}.

In some embodiments of the present disclosure, R², R³, and R⁴, together with the carbon atom linked thereto, form C₅₋₈ bicycloalkyl, the C₅₋₈ bicycloalkyl being a bridged cyclyl, and the C₅₋₈ bicycloalkyl being optionally independently substituted with 1 or more R^{z}.

In some embodiments of the present disclosure, R², R³, and R⁴, together with the carbon atom linked thereto, form 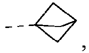 the 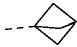 being optionally independently substituted with 1 or more R^{z}.

In some embodiments of the present disclosure, R², R³, and R⁴, together with the carbon atom linked thereto, form

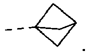

In some embodiments of the present disclosure, A is selected from the group consisting of 3-membered, 4-membered, 5-membered, and 6-membered heterocyclyl containing 1 or 2 N atoms, the 3-membered, 4-membered, 5-membered, or 6-membered heterocyclyl being optionally independently substituted with 1 or more R^{z}; optionally, the heterocyclyl forms a covalent bond with the parent structure via a N atom.

In some embodiments of the present disclosure, A is selected from 4- to 6-membered heterocyclyl containing 1 or 2 N atoms, the 4- to 6-membered heterocyclyl being optionally independently substituted with 1 or more R^{z}; optionally, the heterocyclyl forms a covalent bond with the parent structure via a N atom.

In some embodiments of the present disclosure, A is selected from the group consisting of azetidinyl, pyrrolidinyl, pyrazolidinyl, imidazolidinyl, piperidinyl, and piperazinyl, the azetidinyl, pyrrolidinyl, pyrazolidinyl, imidazolidinyl, piperidinyl, or piperazinyl being optionally independently substituted with 1 or more R^{z}.

In some embodiments of the present disclosure, A is selected from the group consisting of azetidinyl and piperazinyl, the azetidinyl or piperazinyl being optionally independently substituted with 1 or more R^{z}.

In some embodiments of the present disclosure, A is selected from the group consisting of 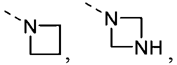 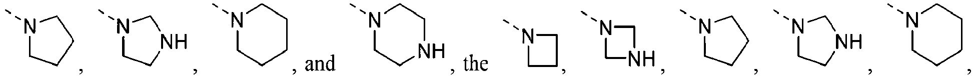 or 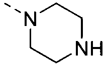 being optionally independently substituted with 1 or more R^{z}.

In some embodiments of the present disclosure, A is selected from the group consisting of 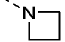 and 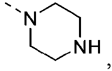 the 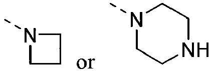 being optionally independently substituted with 1 or more R^{z}.

In some embodiments of the present disclosure, A is selected from the group consisting of 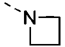 and 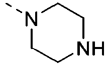 , the 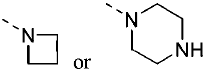 being optionally independently substituted with 1 or more methyl.

In some embodiments of the present disclosure, A is selected from the group consisting of 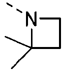 and 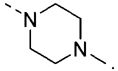 In some other embodiments of the present disclosure, A is selected from

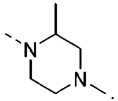

In some other embodiments of the present disclosure, A is selected from the group consisting of

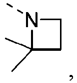

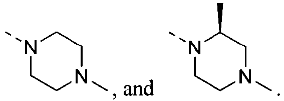

In some embodiments of the present disclosure, the compound of formula (I), the isomer thereof, or the pharmaceutically acceptable salt thereof is selected from the group consisting of compounds of formula (Ia) and formula (Ib), 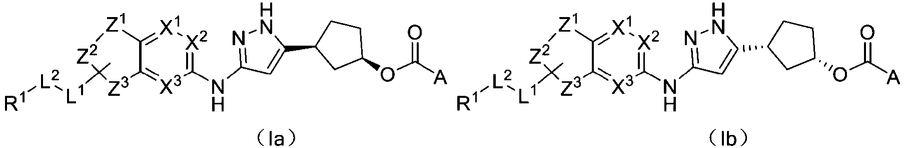 wherein, X¹, X², X³, Z¹, Z², Z³, L¹, L², R¹, and A are defined as in the compound of formula (I).

In some embodiments of the present disclosure, the compound of formula (I), the isomer thereof, or the pharmaceutically acceptable salt thereof is selected from a compound of formula (I-1), 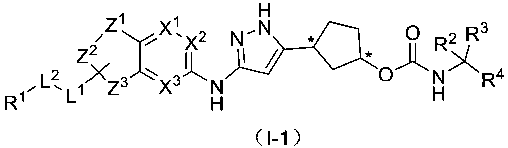 wherein, X¹, X², X³, Z¹, Z², Z³, L¹, L ², R¹, R², R³, and R⁴ are defined as in the compound of formula (I).

In some embodiments of the present disclosure, the compound of formula (I), the isomer thereof, or the pharmaceutically acceptable salt thereof is selected from the group consisting of compounds of formula (I-1a) and formula (I-1b), 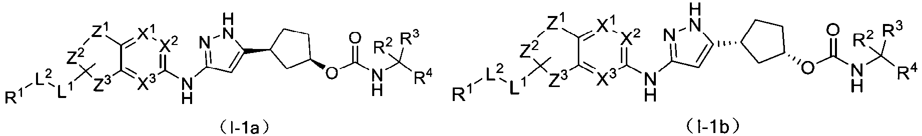 wherein, X¹, X², X³, Z¹, Z², Z³, L¹, L², R¹, R², R³, and R⁴ are defined as in the compound of formula (I).

In some embodiments of the present disclosure, the compound of formula (I), the isomer thereof, or the pharmaceutically acceptable salt thereof is selected from the group consisting of compounds of formula (I-2) and formula (I-3), 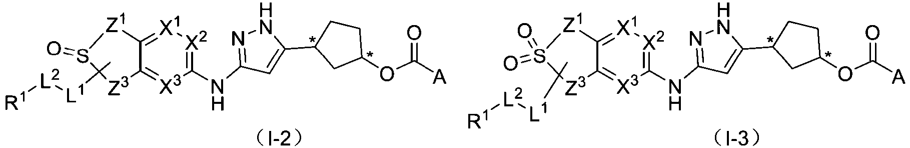 wherein, X¹, X², X³, Z¹, Z³, L¹, L², R¹, and A are defined as in the compound of formula (I).

In some embodiments of the present disclosure, the compound of formula (I), the isomer thereof, or the pharmaceutically acceptable salt thereof is selected from the group consisting of compounds of formula (I-2a), formula (I-3a), formula (I-2b), and formula (I-3b), 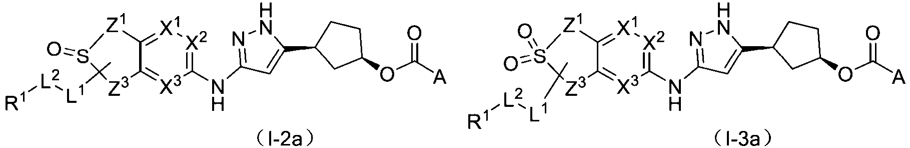 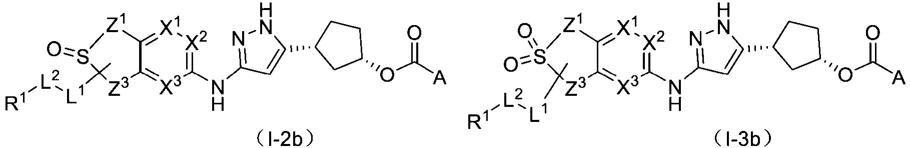 wherein, X¹, X², X³, Z¹, Z³, L¹, L², R¹, and A are defined as in the compound of formula (I).

In some embodiments of the present disclosure, the compound of formula (I), the isomer thereof, or the pharmaceutically acceptable salt thereof is selected from the group consisting of compounds of formula (I-2a') and formula (I-2b'), 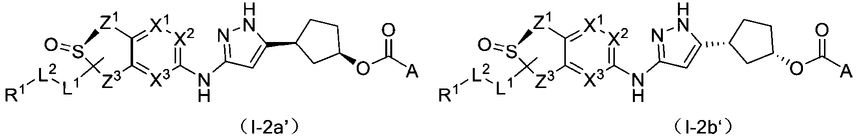 wherein, X¹, X², X³, Z¹, Z³, L¹, L², R¹, and A are defined as in the compound of formula (I).

In some embodiments of the present disclosure, the compound of formula (I), the isomer thereof, or the pharmaceutically acceptable salt thereof is selected from the group consisting of compounds of formula (I-4) and formula (I-5), 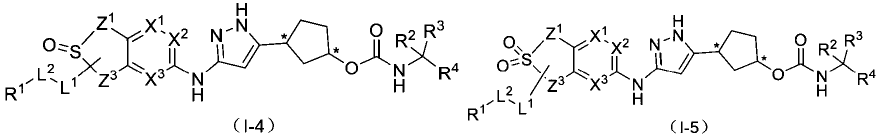 wherein, X¹, X², X³, Z¹, Z³, L¹, L², R¹, R², R³, and R⁴ are defined as in the compound of formula (I).

In some embodiments of the present disclosure, the compound of formula (I), the isomer thereof, or the pharmaceutically acceptable salt thereof is selected from the group consisting of compounds of formula (I-4a), formula (I-5a), formula (I-4b), and formula (I-5b), 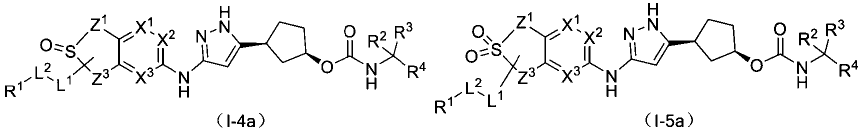 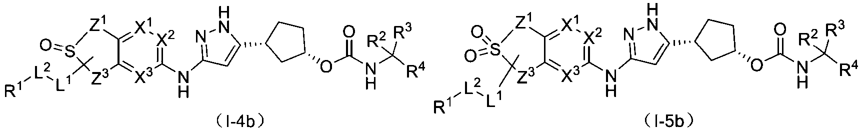 wherein, X¹, X², X³, Z¹, Z³, L¹, L², R¹, R², R³, and R⁴ are defined as in the compound of formula (I).

In some embodiments of the present disclosure, the compound of formula (I), the isomer thereof, or the pharmaceutically acceptable salt thereof is selected from the group consisting of compounds of formula (I-4a') and formula (I-4b'), 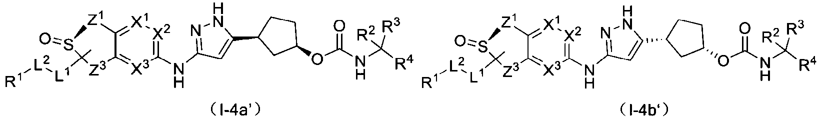 wherein, X¹, X², X³, Z¹, Z³, L¹, L², R¹, R², R³, and R⁴ are defined as in the compound of formula (I).

In some embodiments of the present disclosure, the compound of formula (I), the isomer thereof, or the pharmaceutically acceptable salt thereof is selected from the group consisting of compounds of formula (I-6a), formula (I-7a), formula (I-6b), and formula (I-7b), 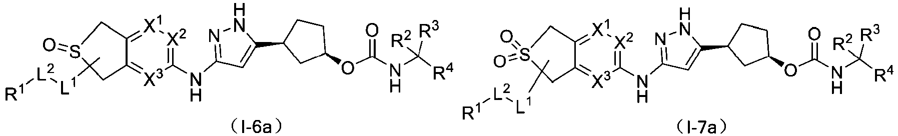 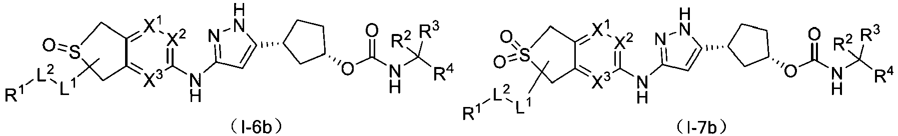 wherein, X¹, X², X³, L¹, L², R¹, R², R³, and R⁴ are defined as in the compound of formula (I).

In some embodiments of the present disclosure, the compound of formula (I), the isomer thereof, or the pharmaceutically acceptable salt thereof is selected from the group consisting of compounds of formula (I-6a) and formula (I-6b), 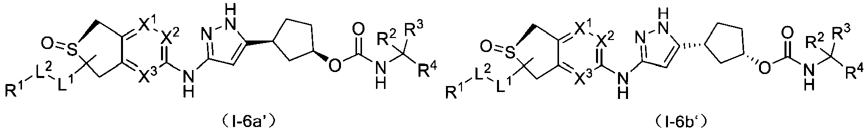 wherein, X¹, X², X³, L¹, L², R¹, R², R³, and R⁴ are defined as in the compound of formula (I).

In some embodiments, the present disclosure excludes compounds of the formulas below, isomers thereof, or pharmaceutically acceptable salts thereof,

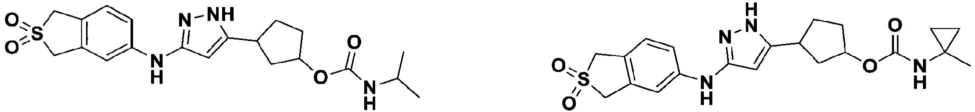

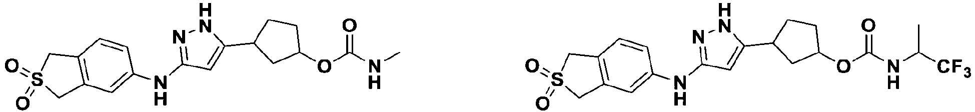

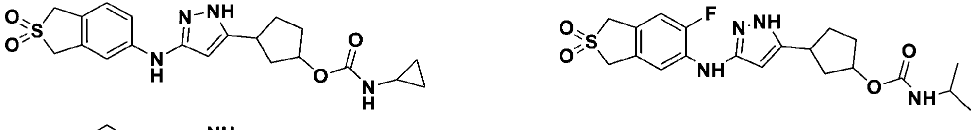

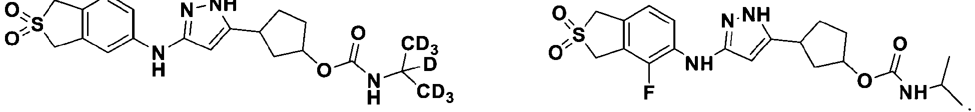

In some embodiments, the present disclosure encompasses the variables defined above and embodiments thereof, as well as any combination thereof.

In another aspect, the present disclosure further provides compounds of the formulas below, isomers thereof, or pharmaceutically acceptable salts thereof,

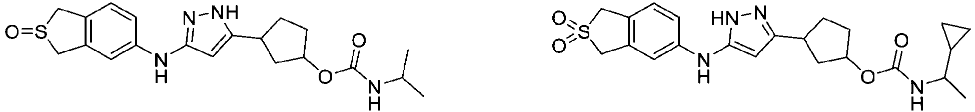

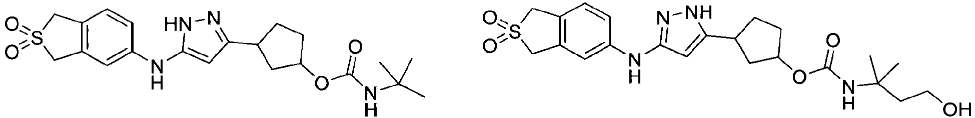

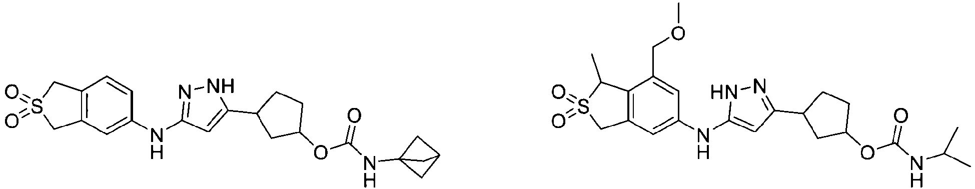

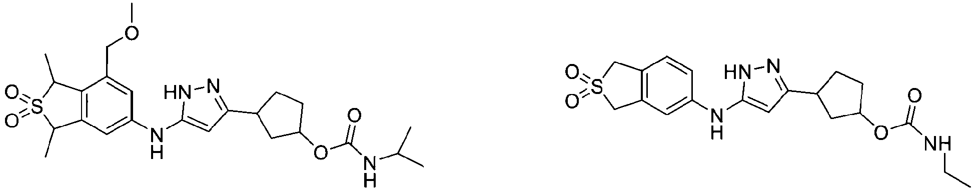

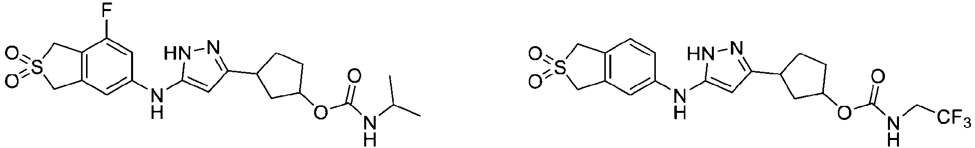

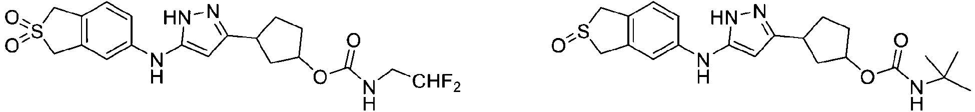

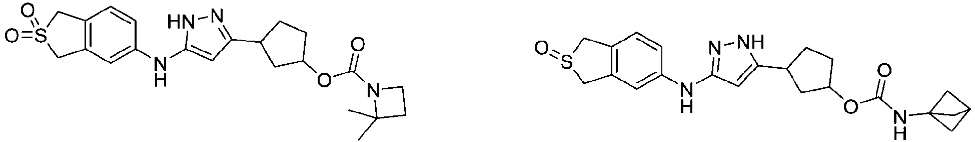

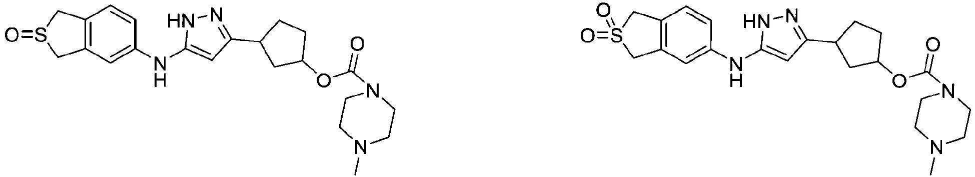

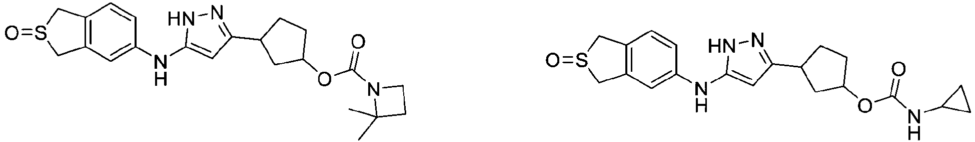

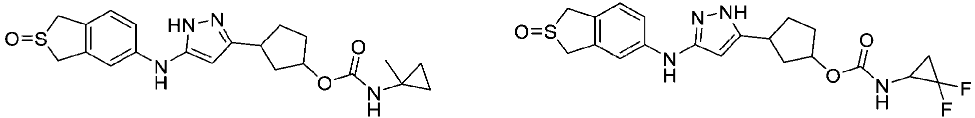

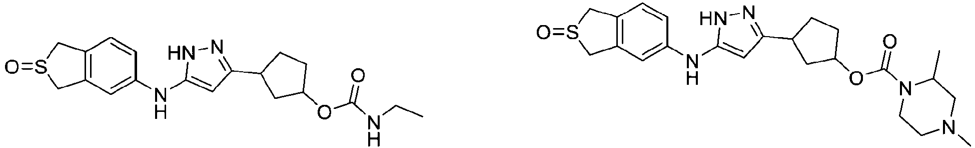

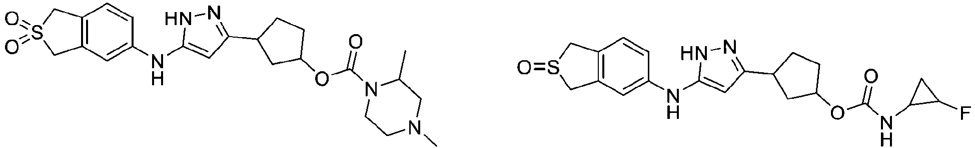

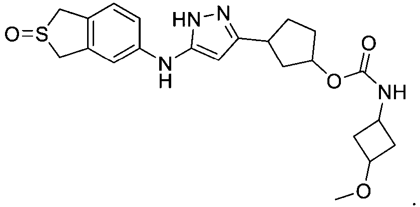

In another aspect, the present disclosure further provides compounds of the formulas below, isomers thereof, or pharmaceutically acceptable salts thereof,

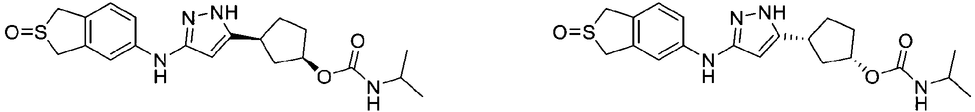

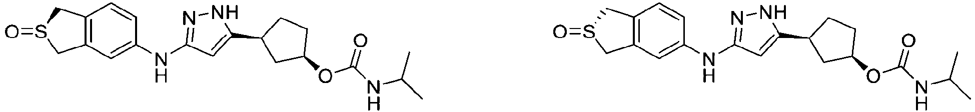

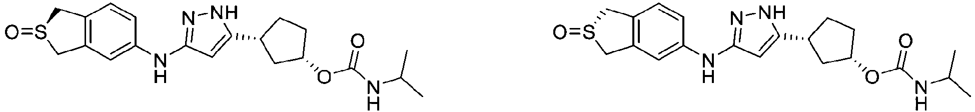

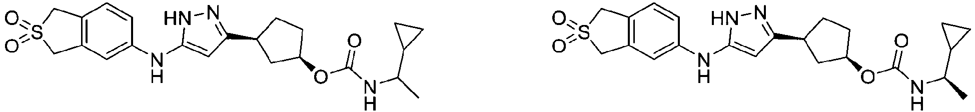

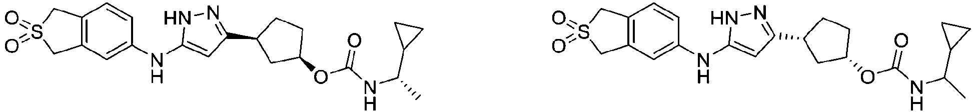

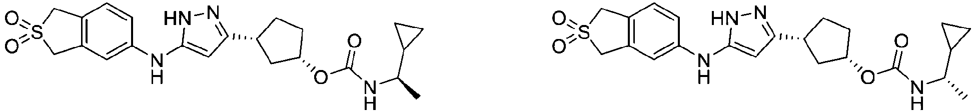

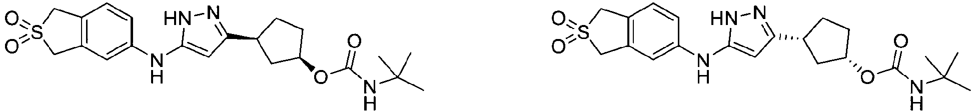

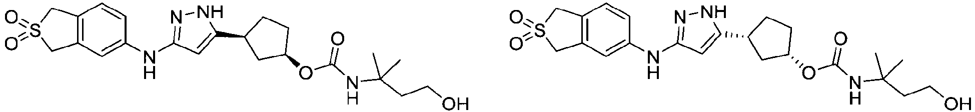

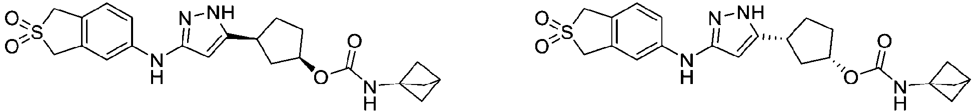

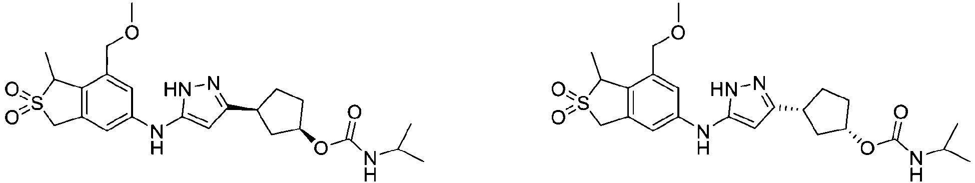

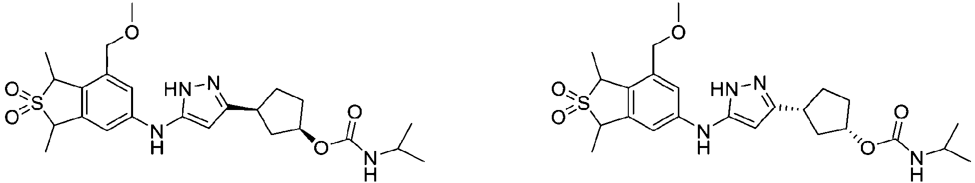

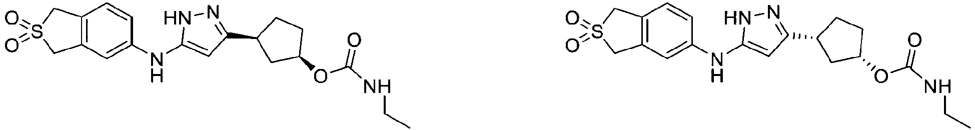

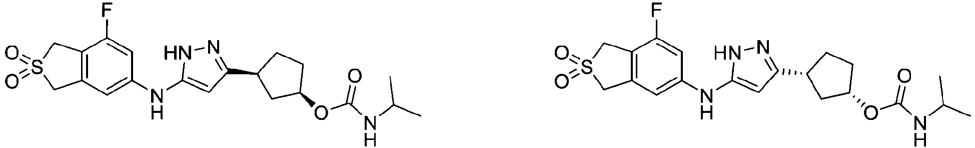

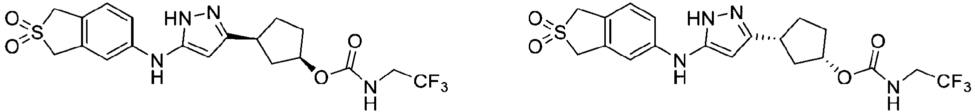

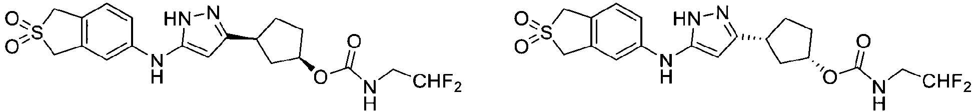

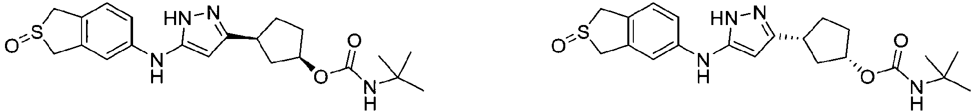

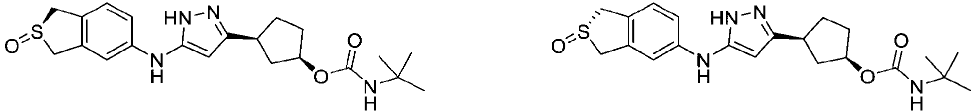

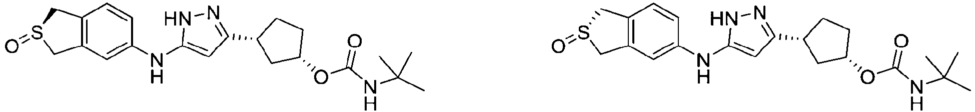

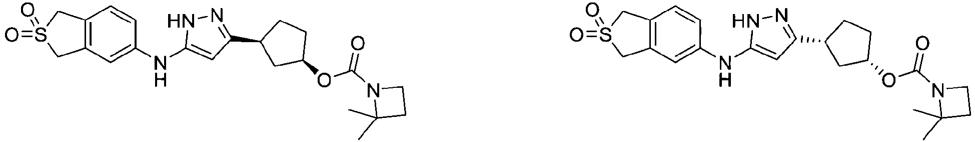

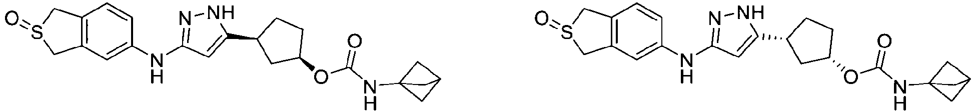

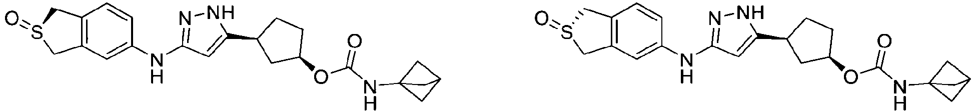

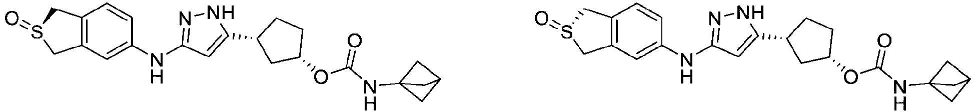

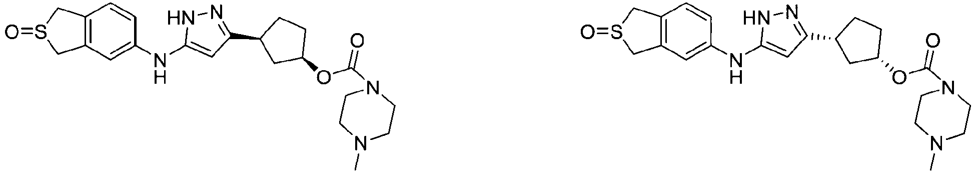

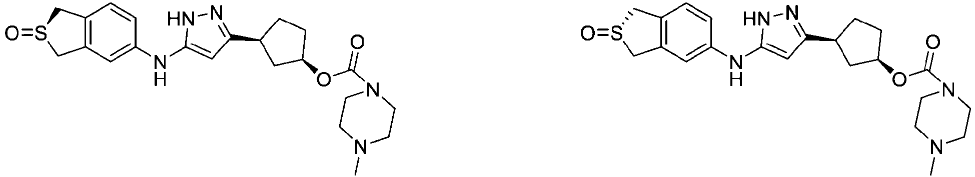

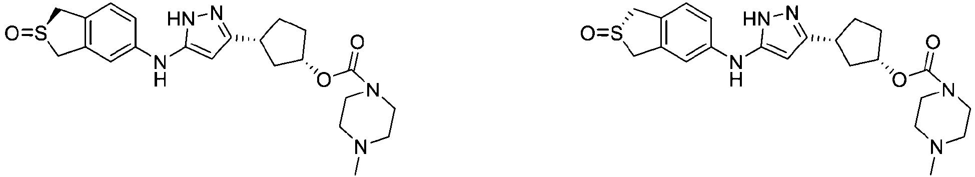

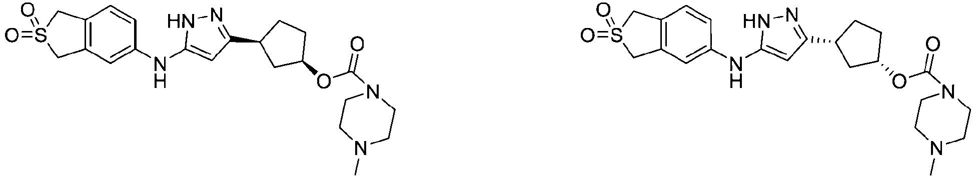

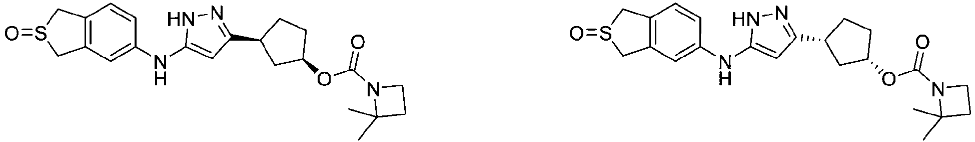

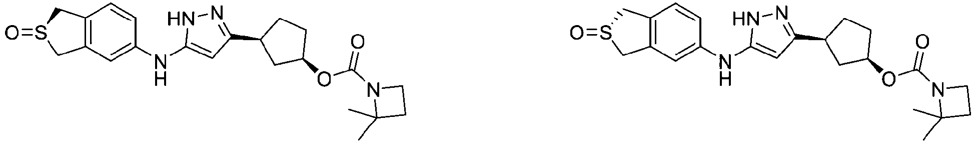

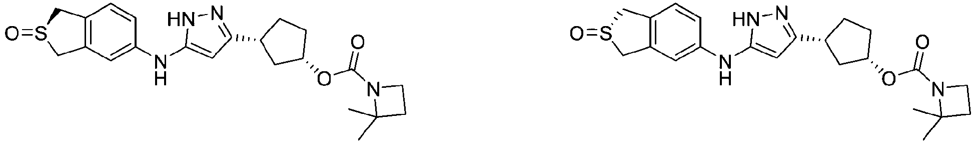

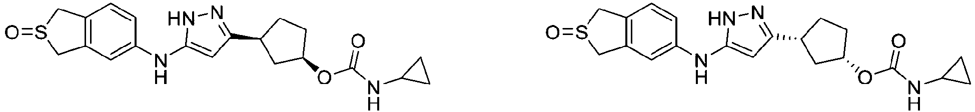

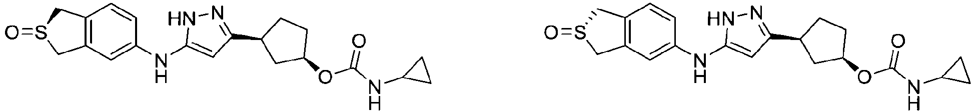

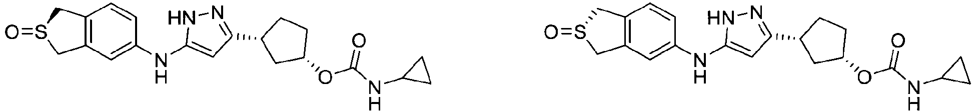

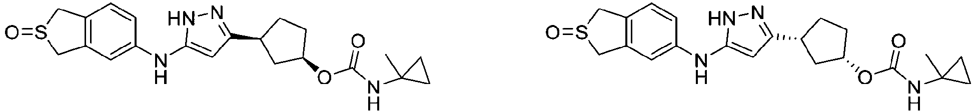

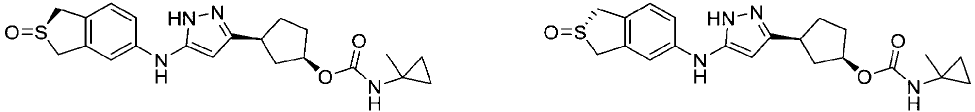

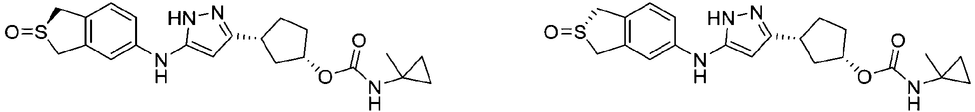

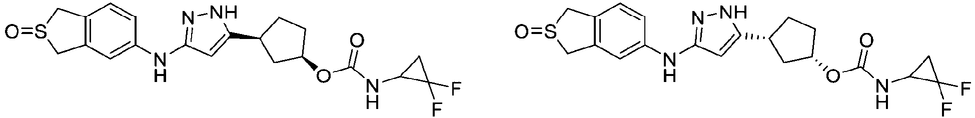

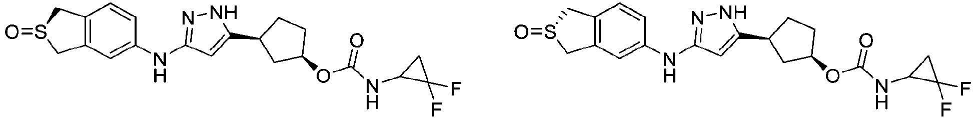

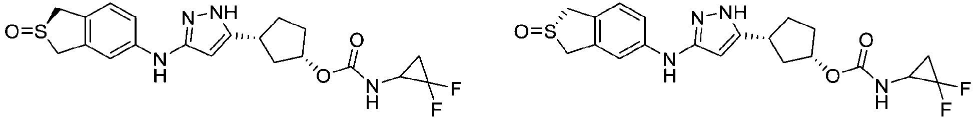

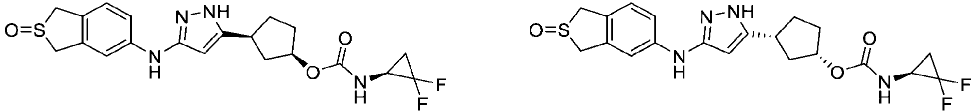

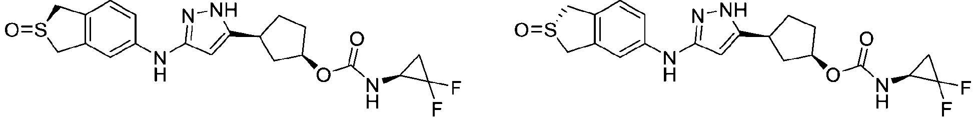

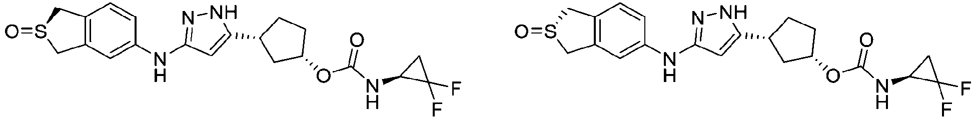

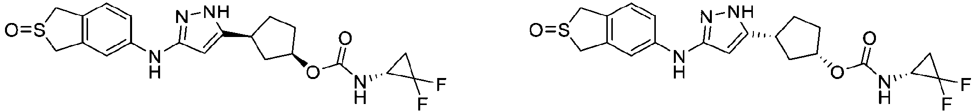

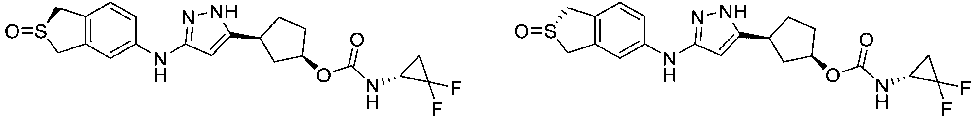

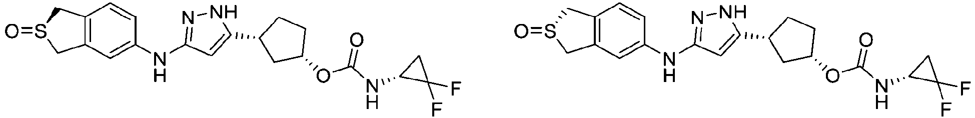

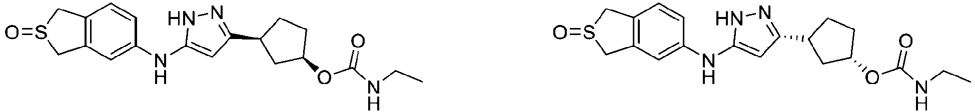

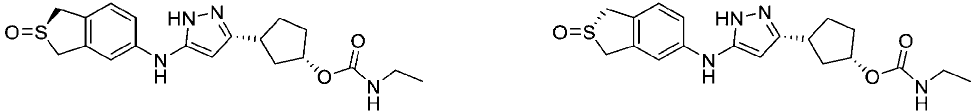

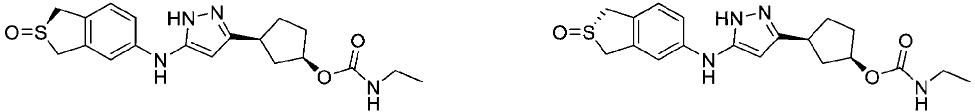

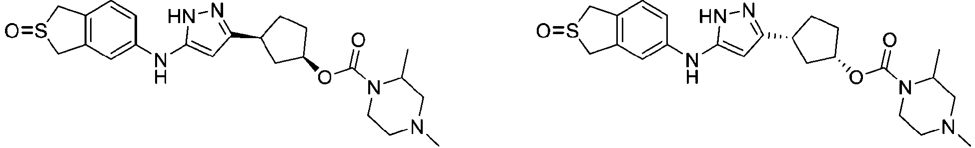

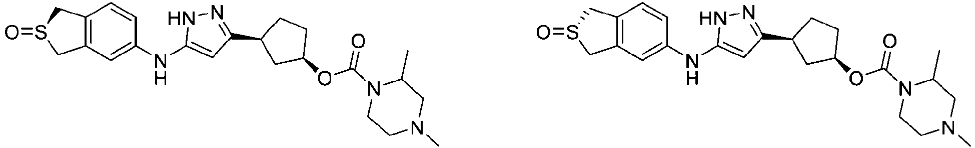

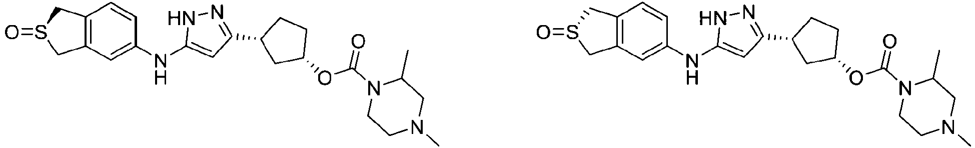

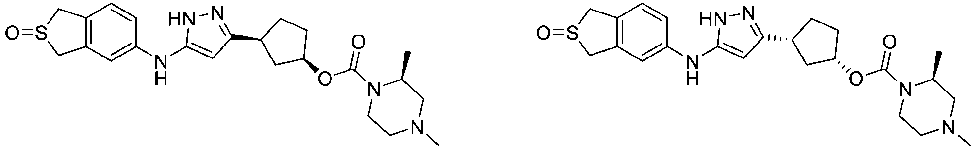

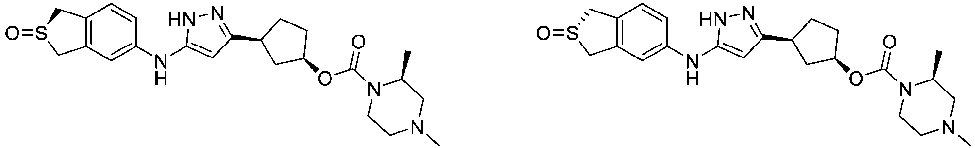

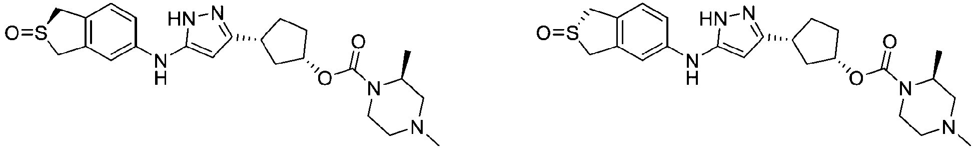

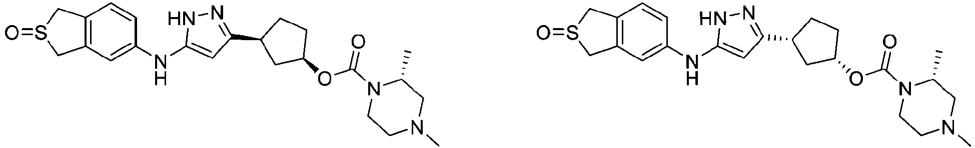

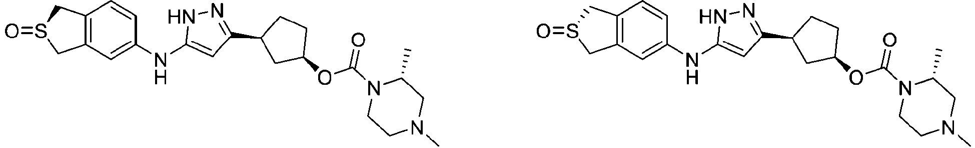

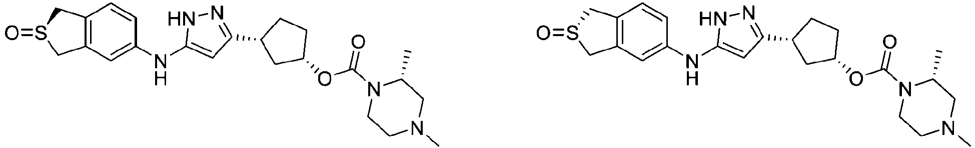

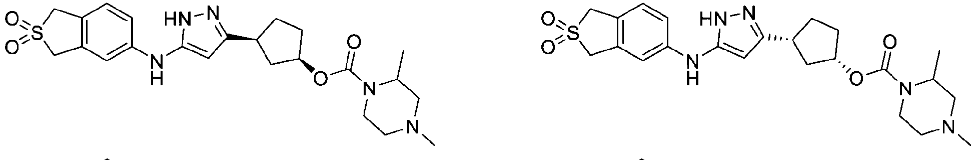

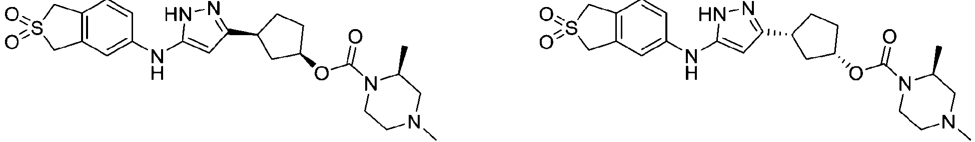

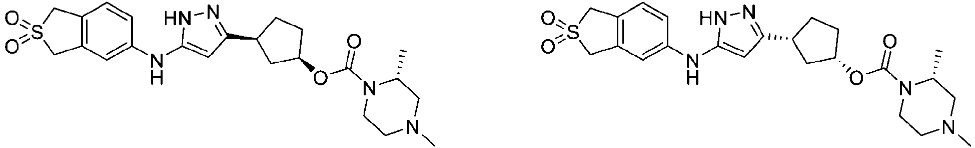

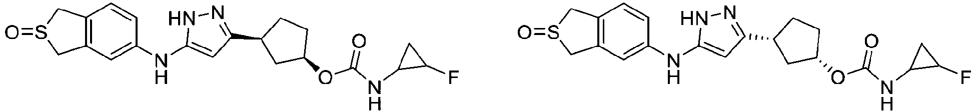

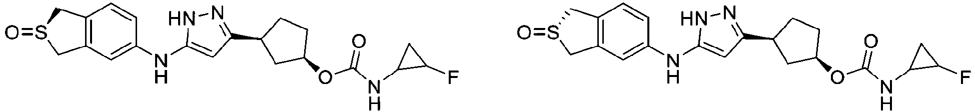

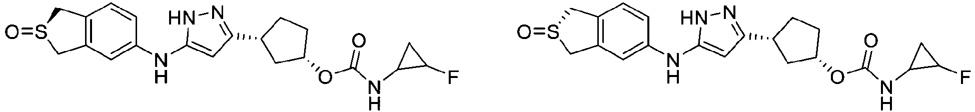

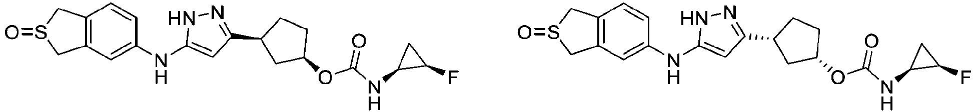

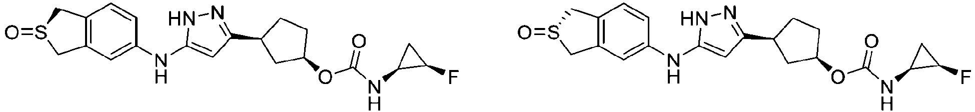

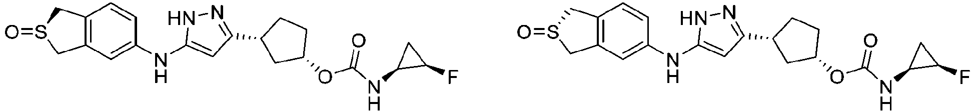

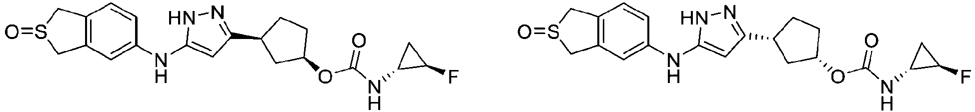

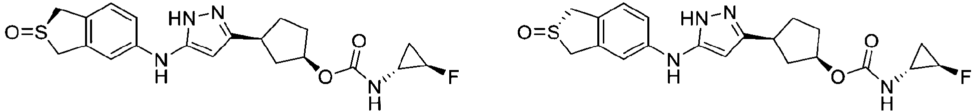

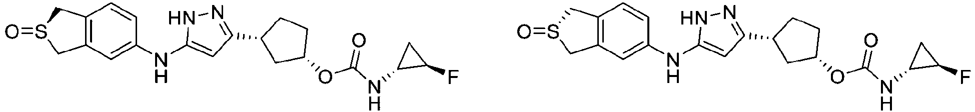

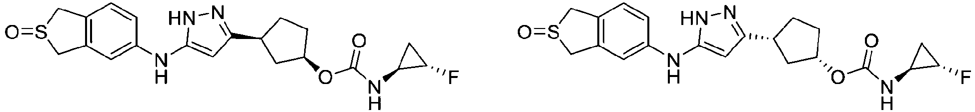

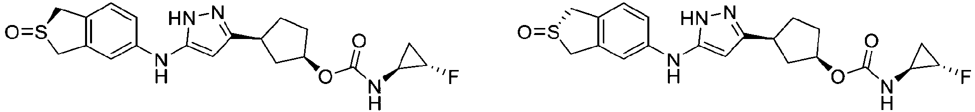

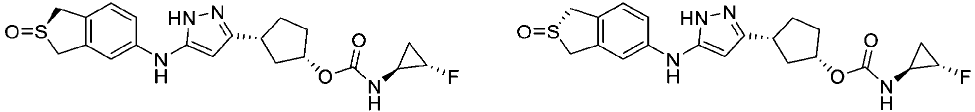

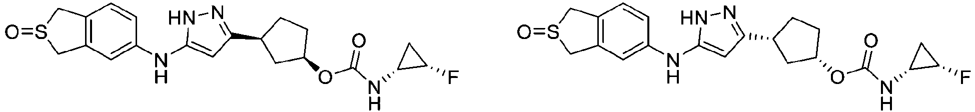

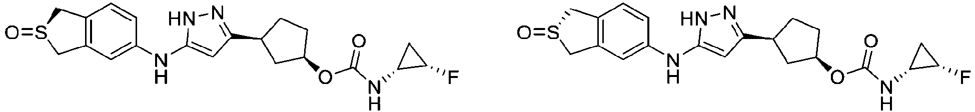

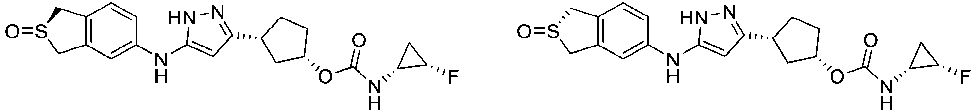

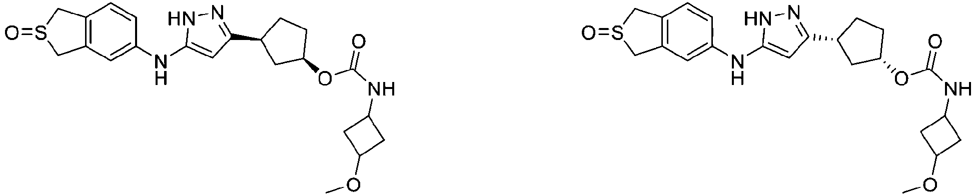

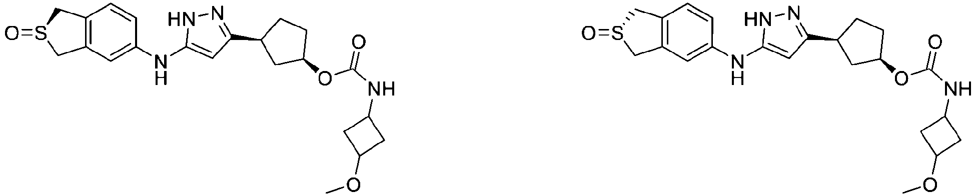

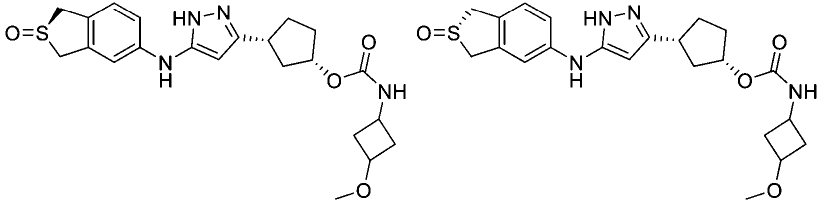

In another aspect, the present disclosure further provides a pharmaceutical composition comprising a therapeutically or prophylactically effective amount of the compound, the isomer thereof, or the pharmaceutically acceptable salt thereof described herein. In some embodiments, the pharmaceutical composition of the present disclosure further comprises a pharmaceutically acceptable excipient.

In another aspect, the present disclosure further provides use of the compound, the isomer thereof, or the pharmaceutically acceptable salt thereof, or the pharmaceutical composition thereof described herein for preparing a medicament for treating or preventing a disease.

In another aspect, the present disclosure further provides a method for treating or preventing a disease, comprising administering to a mammal (preferably a human) in need of the treatment or the prevention a therapeutically or prophylactically effective amount of the compound, the isomer thereof, or the pharmaceutically acceptable salt thereof, or the pharmaceutical composition thereof described herein.

In another aspect, the present disclosure further provides use of the compound, the isomer thereof, or the pharmaceutically acceptable salt thereof, or the pharmaceutical composition thereof described herein for treating or preventing a disease.

In another aspect, the present disclosure further provides the compound, the isomer thereof, or the pharmaceutically acceptable salt thereof, or the pharmaceutical composition thereof described herein for use in treating or preventing a disease.

In some embodiments, the disease described herein is selected from a CDK2-mediated disease.

In another aspect, in some embodiments of the present disclosure, the CDK2-mediated disease is selected from the group consisting of a tumor and a cancer.

In the present disclosure, the "isomer" includes, but is not limited to, a stereoisomer or a tautomer.

In some embodiments, the "isomer" is selected from a stereoisomer.

### Technical Effects

The compound in the present disclosure, as a CDK2 inhibitor with a brand-new structure, has a good inhibition effect on CDK2-induced signal transduction and a good inhibitory activity against CDK2 CycA2 and CDK2 CycE1 kinases, and possesses favorable properties in at least one or more aspects such as *in vivo* and *in vitro* inhibitory activity, safety (e.g., low toxicity), and CDK2 selectivity. The compound of the present disclosure has good pharmacokinetics, and can be developed into a novel CDK2 inhibitor drug.

### Definitions

Unless otherwise stated, the following terms and phrases used herein are intended to have the following meanings. A particular term or phrase, unless otherwise specifically defined, should not be considered indefinite or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms that are, within the scope of sound medical judgment, suitable for use in contact with the tissues of humans and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having particular substituents discovered by the present disclosure and a relatively nontoxic acid or base. When the compound of the present disclosure contains a relatively acidic functional group, a base addition salt can be obtained by allowing such a compound to be in contact with a sufficient amount of a base in a pure solution or a suitable inert solvent. When the compound of the present disclosure contains a relatively basic functional group, an acid addition salt can be obtained by allowing such a compound to be in contact with a sufficient amount of an acid in a pure solution or a suitable inert solvent. Certain particular compounds of the present disclosure contain both basic and acidic functional groups that allow the compounds to be converted into either base or acid addition salts.

The pharmaceutically acceptable salt of the present disclosure can be synthesized from the parent compound containing an acid radical or a basic group by conventional chemical methods. In general, such salts are prepared by subjecting the free acid or base form of the compound to a reaction with a stoichiometric amount of the appropriate base or acid in water or an organic solvent or a mixture thereof.

The compound of the present disclosure may exist in the form of a particular stereoisomer. All such compounds are contemplated herein, including *cis* and *trans* isomers, (-)- and (+)-enantiomers, (*R*)- and (*S*)-enantiomers, diastereoisomers, (*D*)-isomers, (*L*)-isomers, and racemic mixtures and other mixtures thereof, such as a mixture enriched in enantiomer or diastereoisomer, all of which are encompassed within the scope of the present disclosure. The substituents such as alkyl may have an additional asymmetric carbon atom. All these isomers and mixtures thereof are encompassed within the scope of the present disclosure. The substituents such as alkyl may have an additional asymmetric carbon atom. All these isomers and mixtures thereof are encompassed within the scope of the present disclosure.

Unless otherwise specified, the absolute configuration of a stereogenic center is represented by a wedged solid bond ( 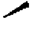 ) and a wedged dashed bond ( 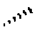 ), and the relative configuration of a stereogenic center is represented by a straight solid bond ( 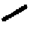 ) and a straight dashed bond ( 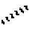 ).

The compounds and intermediates of the present disclosure may also be present in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of proton tautomers is the imidazole moiety where a proton can transfer between two ring nitrogen atoms. The valence tautomer includes the interconversion via recombination of some bonding electrons.

The compound of the present disclosure may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted by deuterium to form a deuterated drug, and the bond formed by deuterium and carbon is firmer than that formed by common hydrogen and carbon. Compared with an un-deuterated drug, a deuterated drug has the advantages of reduced adverse effects, increased stability, enhanced efficacy, prolonged biological half-life, and the like. All isotopic variations of the compound of the present disclosure, whether radioactive or not, are encompassed within the scope of the present disclosure. For example, it should be understood that compounds formed by replacing one or more hydrogen atoms in the compound of formula (I) of the present disclosure with a deuterium atom still fall within the scope of the compound of formula (I) disclosed herein.

The term "optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted by substituents, wherein the substituents may include deuterium and hydrogen variants, as long as the valence of the specific atom is normal and the compound after substitution is stable. When the substituent is oxygen (i.e., =O), it means that two hydrogen atoms are substituted. The substitution with oxygen does not occur on aromatic groups. The term "optionally substituted" means that an atom may or may not be substituted with a substituent. Unless otherwise specified, the type and number of the substituent may be arbitrary as long as being chemically achievable.

The term "1 or more substitutions" means that any one or more hydrogen atoms on a specific atom are substituted by substituents, wherein the substituents may include deuterium and hydrogen variants, and the number of the substituents includes 1, 2, 3, 4, 5, 6, 7, 8, 9, or 10 as long as being chemically achievable.

The "substituent" described herein includes, but is not limited to, the terms "alkyl", "alkoxy", "cycloalkyl", "cycloalkenyl", "heterocyclyl", "heterocycloalkyl", "heterocycloalkenyl", "heteroaryl", "alkyl ring", "heteroalkyl ring", "heteroaromatic ring", and the like, and the corresponding non-limiting or exemplary groups mentioned in the context, wherein some non-limiting examples of the "substituent" include deuterium, tritium, -OH, -SH, halogen, -NH₂, nitro, nitroso, -CN, an azide group, a sulfoxide group, a sulfone group, a sulfonamide group, carboxyl, a carboxaldehyde group, an imine group, alkyl, haloalkyl, cycloalkyl, halocycloalkyl, alkenyl, haloalkenyl, cycloalkenyl, halocycloalkenyl, alkynyl, haloalkynyl, cycloalkynyl, halocycloalkynyl, heteroalkyl, halogenated heteroalkyl, alkoxy, alkylthio, aryl, aryloxy, arylthio, arylalkyl, arylalkoxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkyl, heterocyclylalkoxy, heterocyclylalkylthio, acyl, acyloxy, a carbamate group, an amido group, ureido, an epoxy group, an ester group, and the like, wherein the groups are optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkyl, cycloalkyloxy, heterocyclyl, heterocyclylalkyl, heterocyclyloxy, heterocycloalkyl, heterocycloalkylalkyl, heterocycloalkyloxy, heteroaryl, heteroarylalkyl, heteroaryloxy, aryl, arylalkyl, and aryloxy. In some embodiments herein, the "substituent" is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, C₁₋₁₂ alkyl, halogenated C₁₋₁₂ alkyl, 3- to 12-membered cycloalkyl, halogenated 3- to 12-membered cycloalkyl, C₂₋₁₂ alkenyl, halogenated C₂₋₁₂ alkenyl, 3- to 12-membered cycloalkenyl, halogenated 3- to 12-membered cycloalkenyl, C₂₋₁₂ alkynyl, halogenated C₂₋₁₂ alkynyl, 8- to 12-membered cycloalkynyl, halogenated 8- to 12-membered cycloalkynyl, C₁₋₁₂ heteroalkyl, halogenated C₁₋₁₂ heteroalkyl, C₁₋₁₂ alkoxy, C₁₋₁₂ alkylthio, 6- to 10-membered aryl, 6- to 10-membered aryloxy, 6- to 10-membered arylthio, 6- to 10-membered aryl C₁₋₁₂ alkylene, 6- to 10-membered aryl C₁₋₁₂ alkoxy, 6- to 10-membered aryl C₁₋₁₂ alkylthio, 5- to 10-membered heteroaryl, 5- to 10-membered heteroaryloxy, 5- to 10-membered heteroarylthio, 5-to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl C₁₋₁₂ alkylene, 3-to 12-membered heterocyclyl C₁₋₁₂ alkoxy, 3- to 12-membered heterocyclyl C₁₋₁₂ alkylthio, C₁₋₁₂ acyl, C₁₋₁₂ acyloxy, a carbamate group, C₁₋₁₂ amido, ureido, an epoxy group, a C₂₋₁₂ ester group, and oxo, wherein these substituents are optionally substituted with one or more substituents selected from the group consisting of the following substituents: oxo, hydroxy, amino, nitro, halogen, cyano, C₁₋₁₂ alkyl, C₂₋₁₂ alkenyl, C₂₋₁₂ alkynyl, C₁₋₁₂ alkoxy, halogenated C₁₋₁₂ alkoxy, C₁₋₁₂ alkylamino, di-C₁₋₁₂ alkylamino, halogenated C₁₋₁₂ alkylamino, halogenated di-C₁₋₁₂ alkylamino, carboxyl, -C(O)O-C₁₋₁₂ alkyl, -OC(O)-C₁₋₁₂ alkyl, -C(O)NH₂, - C(O)NH-C₁₋₁₂ alkyl, -C(O)N(C₁₋₁₂ alkyl)₂, -NHC(O)-C₁₋₁₂ alkyl, -C(O)-C₁₋₁₂ alkyl, -S(O)-C₁₋₁₂ alkyl, -S(O)₂-C₁₋₁₂ alkyl, -S(O)₂NH₂, -S(O)₂NH-C₁₋₁₂ alkyl, -S(O)₂N(C₁₋₁₂ alkyl)₂, 3- to 12-membered cycloalkyl, 3- to 12-membered cycloalkyl C₁₋₁₂ alkylene, 3- to 12-membered cycloalkyloxy, 3- to 12-membered heterocyclyl, 3- to 12-membered heterocyclyl C₁₋₁₂ alkylene, 3- to 12-membered heterocyclyloxy, 3- to 12-membered heterocycloalkyl, 3- to 12-membered heterocycloalkyl C₁₋₁₂ alkylene, 3- to 12-membered heterocycloalkyloxy, 5- to 10-membered heteroaryl, 5- to 10-membered heteroaryl C₁₋₁₂ alkylene, 5- to 10-membered heteroaryloxy, 6- to 10-membered aryl, 6- to 10-membered aryl C₁₋₁₂ alkylene, and 6- to 10-membered aryloxy.

The Cₘ₋ₙ herein means that the moiety has an integer number of carbon atoms in the given range. For example, the "C₁₋₆" means that the group may have 1 carbon atom, 2 carbon atoms, 3 carbon atoms, 4 carbon atoms, 5 carbon atoms, or 6 carbon atoms. For example, the C₁₋₃ means that the group may have 1 carbon atom, 2 carbon atoms, or 3 carbon atoms.

When any variable (e.g., R) occurs more than once in the constitution or structure of a compound, the definition of the variable in each case is independent. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

When one of the variables is selected from a single bond, it means that the two groups to which it connects are connected directly. For example, in A-L-Z, when L represents a single bond, it means that the structure is actually A-Z.

When the direction for connection of the listed connecting group is not specified, the direction for connection is arbitrary. For example, when the connecting group L contained in 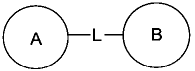 is -M-W-, -M-W- can either connect ring A and ring B in a direction same as left-to-right reading order to form 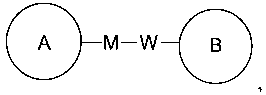 or connect ring A and ring B in a direction opposite to the left-to-right reading order to form 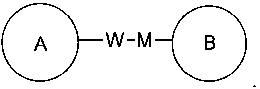 A combination of the connecting group, the substituent, and/or the variant thereof is permissible only if the combination can result in a stable compound.

Unless otherwise specified, when a group has one or more connectable sites, any one or more of the sites of the group may be connected to other groups by chemical bonds. If there is no designated connecting mode for chemical bonds and H atoms are present at a connectable site, when the connectable site is connected to chemical bonds, the number of the H atoms at the connectable site is correspondingly reduced based on the number of the connected chemical bonds, resulting in a group with the corresponding valence. The chemical bond that connects the site and another group may be represented by a straight solid bond ( 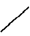 ), a straight dashed bond ( 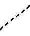 ), or a wavy line ( 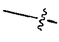 ). For example, the straight solid bond in -OCH₃ refers to being connected to another group via the oxygen atom in the group; the straight dashed bonds in 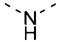 refer to being connected to other groups via two ends of the nitrogen atom in the group; the wavy lines in 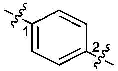 refer to being connected to other groups via the carbon atoms at positions 1 and 2 in the phenyl group; 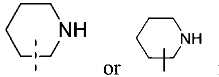 means that any connectable site on the piperidinyl can be connected to another group via 1 chemical bond in at least 4 connecting modes: 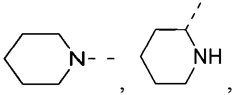 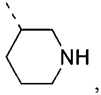 and 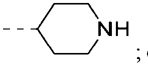 even if -N- is connected to a H atom, 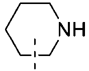 includes the connection mode of 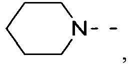 but when 1 chemical bond is connected to a site, the number of H at that site is correspondingly reduced by 1, resulting in a corresponding monovalent piperidinyl group.

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, and the like, and may be monovalent (e.g., methyl), divalent (e.g., methylene), or polyvalent (e.g., methenyl). Examples of the C₁₋₃ alkyl include, but are not limited to, methyl (Me), ethyl (Et), propyl (including n-propyl and isopropyl), and the like.

Unless otherwise specified, the term "C₁₋₃ alkoxy" refers to those alkyl groups that each contain 1 to 3 carbon atoms and are linked to the rest part of the molecule via an oxygen atom. The C₁₋₃ alkoxy includes C₁₋₂, C₂₋₃, C₃ and C₂ alkoxy, and the like. Examples of the C₁₋₃ alkoxy include, but are not limited to, methoxy, ethoxy, propoxy (including n-propoxy and isopropoxy), and the like.

Unless otherwise specified, the term "C₁₋₃ alkylene", by itself or as part of another substituent, refers to a linear or branched divalent hydrocarbyl group consisting of 1 to 3 carbon atoms. Non-limiting examples of C₁₋₃ alkylene include, but are not limited to, methylene (-CH₂-), ethylene (-CH₂CH₂-), and propylene (-CH₂CH₂CH₂- or - CH₂CH(CH₃)-).

Unless otherwise specified, the Cₙ₋ₙ₊ₘ or Cₙ-Cₙ₊ₘ includes any one of the specific cases of n to n+m carbon atoms. For example, the C₁₋₁₂ includes C₁, C₂, C₃, C₄, C₅, C₆, C₇, C₈, C₉, C₁₀, C₁₁, and C₁₂. Also, any range within n to n+m may be included. For example, the C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, and the like. Similarly, the n- to n+m-membered represents that the number of atoms on the ring is n to n+m. For example, the 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. n- to n+m-membered also represents any range within n to n+m. For example, 3- to 12-membered ring includes 3-to 6-membered ring, 3- to 9-membered ring, 5- to 6-membered ring, 5- to 7-membered ring, 6- to 7-membered ring, 6- to 8-membered ring, 6- to 10-membered ring, and the like.

Unless otherwise specified, the term "C₃₋₆ cycloalkyl" refers to a saturated cyclic hydrocarbon group consisting of 3 to 6 carbon atoms, including monocyclic and bicyclic systems. The C₃₋₆ cycloalkyl includes C₃₋₅, C₄₋₅ and C₅₋₆ cycloalkyl, and the like, and may be monovalent, divalent, or polyvalent. Examples of the C₃₋₆ cycloalkyl include, but are not limited to, cyclopropyl, cyclobutyl, cyclopentyl, cyclohexyl, and the like.

Unless otherwise specified, the term "C₃₋₆ cycloalkenyl" refers to a partially unsaturated (but not fully unsaturated, aromatic) cyclic hydrocarbon group consisting of 3 to 6 carbon atoms, including monocyclic and bicyclic systems. The C₃₋₆ cycloalkenyl includes C₃₋₅, C₄₋₅ and C₅₋₆ cycloalkenyl, and the like, and may be monovalent, divalent, or polyvalent. Examples of the C₃₋₆ cycloalkenyl include, but are not limited to, cyclopentenyl, cyclopentadienyl, cyclohexenyl, cyclohexadienyl, and the like.

Unless otherwise specified, the term "3- to 6-membered heterocyclyl" refers to a fully saturated or partially unsaturated (but not fully unsaturated, heteroaromatic) non-aromatic ring that may be present as a monocyclic ring, a bridged ring, a fused ring, or a spiro ring. Unless otherwise specified, the heterocyclyl is usually a 3- to 6-membered, 4- to 6-membered, or 5- to 6-membered ring (e.g., a 3-, 4-, 5-, or 6-membered ring) containing 1 to 3 heteroatoms (preferably 1 or 2 heteroatoms) independently selected from the group consisting of sulfur, oxygen, nitrogen, phosphorus, silicon, and/or boron. Non-limiting examples of the 3- to 6-membered heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like.

Unless otherwise specified, the term "3- to 6-membered heterocycloalkyl", by itself or in combination with other terms, refers to a saturated cyclic group consisting of 3 to 6 ring atoms, of which 1, 2, 3, or 4 ring atoms are heteroatoms independently selected from the group consisting of O, S, and N, with the remaining being carbon atoms. The nitrogen atom is optionally quaternized, and the nitrogen and sulfur heteroatoms can be optionally oxidized (i.e., NO and S(O)ₚ, wherein p is 1 or 2). The group is a monocyclic system. Furthermore, with respect to the "3- to 6-membered heterocycloalkyl", a heteroatom may occupy the position where the heterocycloalkyl is linked to the rest part of the molecule. The 3- to 6-membered heterocycloalkyl includes 3-membered, 4-membered, 5-membered and 6-membered heterocycloalkyl, and the like. Examples of 3- to 6-membered heterocycloalkyl include, but are not limited to, oxiranyl, thiiranyl, aziridinyl, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, and the like), tetrahydrothien-1,1-dioxide-3-yl, tetrahydrothien-1,1-dioxide-2-yl, tetrahydrofuranyl (including tetrahydrofuran-2-yl, and the like), tetrahydropyranyl, piperidinyl (including 1-piperidinyl, 2-piperidinyl, 3-piperidinyl, and the like), piperazinyl (including 1-piperazinyl, 2-piperazinyl, and the like), morpholinyl (including 3-morpholinyl, 4-morpholinyl, and the like), dioxanyl, dithianyl, isoxazolidinyl, isothiazolidinyl, 1,2-oxazinyl, 1,2-thiazinyl, hexahydropyridazinyl, hyperpiperazinyl, hyperpiperidinyl, or the like.

Unless otherwise specified, the term "3- to 6-membered heterocycloalkenyl", by itself or in combination with other terms, refers to a partially unsaturated (but not fully unsaturated, heteroaromatic) cyclic group consisting of 3 to 6 ring atoms, of which 1, 2, 3, or 4 ring atoms are heteroatoms independently selected from the group consisting of O, S, and N, with the remaining being carbon atoms. The nitrogen atom is optionally quaternized, and the nitrogen and sulfur heteroatoms can be optionally oxidized (i.e., NO and S(O)ₚ, wherein p is 1 or 2). The group is a monocyclic system. Furthermore, with respect to the "3- to 6-membered heterocycloalkenyl", a heteroatom may occupy the position where the heterocycloalkyl is linked to the rest part of the molecule. The 3- to 6-membered heterocycloalkenyl includes 3-membered, 4-membered, 5-membered and 6-membered heterocycloalkenyl, and the like. Examples of the 3- to 6-membered heterocycloalkenyl include, but are not limited to, dihydropyridinyl, dihydropyrrolyl, or tetrahydropyridinyl.

Unless otherwise specified, the term "5- to 6-membered heteroaryl" refers to a cyclic group consisting of 5 to 6 ring atoms and having a conjugated π-electron system, of which 1, 2, or 3 ring atoms are heteroatoms independently selected from the group consisting of O, S, and N, with the remaining being carbon atoms. The nitrogen atom is optionally quaternized, and the carbon, nitrogen, and sulfur heteroatoms may optionally be oxidized (i.e., C=O, NO, and S(O)ₚ, wherein p is 1 or 2). The 5- to 6-membered heteroaryl can be connected to the rest of the molecule through a heteroatom or a carbon atom. The 5- to 6-membered heteroaryl includes 5-membered heteroaryl and 6-membered heteroaryl. Examples of the 5- to 6-membered heteroaryl include, but are not limited to, pyrrolyl (including *N*-pyrrolyl, 2-pyrrolyl, 3-pyrrolyl, and the like), pyrazolyl (including 2-pyrazolyl, 3-pyrazolyl, and the like), imidazolyl (including *N*-imidazolyl, 2-imidazolyl, 4-imidazolyl, 5-imidazolyl, and the like), oxazolyl (including 2-oxazolyl, 4-oxazolyl, 5-oxazolyl, and the like), triazolyl (including 1*H*-1,2,3-triazolyl, 2*H*-1,2,3-triazolyl, 1*H-*1,2,4-triazolyl, 4*H*-1,2,4-triazolyl, and the like), tetrazolyl, isoxazolyl (including 3-isoxazolyl, 4-isoxazolyl, 5-isoxazolyl, and the like), thiazolyl (including 2-thiazolyl, 4-thiazolyl, 5-thiazolyl, and the like), furanyl (including 2-furanyl, 3-furanyl, and the like), thienyl (including 2-thienyl, 3-thienyl, and the like), pyridinyl (including 2-pyridinyl, 3-pyridinyl, 4-pyridinyl, and the like), pyrazinyl, or pyrimidinyl (including 2-pyrimidinyl, 4-pyrimidinyl, and the like).

Unless otherwise specified, the term "C₅₋₈ bicycloalkyl" refers to a saturated hydrocarbon ring consisting of 5 to 8 carbon atoms, which is a bicyclic system, wherein the bicyclic system includes spiro, fused, and bridged rings. The C₅₋₈ bicycloalkyl includes C₅₋₈ or C₅₋₆ bicycloalkyl, and may be monovalent, divalent, or polyvalent. Examples of the C₅₋₈ bicycloalkyl include, but are not limited to, bicyclo[1.1.1]pentane and the like.

Unless otherwise specified, the term "5- to 8-membered heterobicycloalkyl" refers to a saturated cyclic group consisting of 5 to 8 ring atoms, of which 1, 2, 3, or 4 ring atoms are heteroatoms independently selected from the group consisting of O, S, and N, with the remaining being carbon atoms. The nitrogen atom is optionally quaternized, and the carbon, nitrogen, and sulfur heteroatoms may optionally be oxidized (i.e., C=O, NO, and S(O)ₚ, wherein p is 1 or 2). The group is a bicyclic system, wherein the bicyclic system includes spiro, fused, and bridged rings. Furthermore, with respect to the "5- to 8-membered heterobicycloalkyl", a heteroatom may occupy the position where the heteroalkyl ring is linked to the rest part of the molecule. The 5- to 8-membered heterobicycloalkyl includes 5- to 6-membered heteroalkyl rings and the like. Examples of the 5- to 8-membered heterobicycloalkyl include, but are not limited to, 2-azabicyclo[1.1.1]pentane and the like.

Unless otherwise specified, the term "fused" refers to a saturated or partially unsaturated non-aromatic cyclic system formed by two or more cyclic structures that share two adjacent atoms with each other, including fused carbocyclic rings and fused heterocyclic rings. The ring atoms of the fused heterocyclic rings include one or more heteroatoms independently selected from the group consisting of oxygen, nitrogen, and sulfur.

Unless otherwise specified, the term "hetero" includes heteroatoms of sulfur, oxygen, nitrogen, phosphorus, silicon, and/or boron.

The term "treat", "treating", or "treatment" means administering the compound or formulation described in the present disclosure to ameliorate or eliminate a disease or one or more symptoms related to the disease, and includes:
(i) inhibiting the disease or disease state, i.e., arresting its development; and
(ii) alleviating the disease or disease state, i.e., causing its regression.

The term "prevent", "preventing", or "prevention" means administering the compound or formulation described in the present disclosure to prevent a disease or one or more symptoms related to the disease, and includes preventing the occurrence of the disease or disease state in a mammal, particularly when such a mammal is predisposed to the disease state but has not yet been diagnosed with it.

The term "therapeutically or prophylactically effective amount" refers to an amount of the compound of the present disclosure for (i) treating a specific disease, condition, or disorder, or (ii) alleviating, ameliorating, or eliminating one or more symptoms of a specific disease, condition, or disorder, or (iii) preventing or delaying the specific disease, condition, or disorder described herein. The amount of the compound of the present disclosure composing the "therapeutically or prophylactically effective amount" varies depending on the compound, the disease state and its severity, the mode of administration, and the age of the mammal to be treated, but may be determined routinely by those skilled in the art in accordance with their knowledge and the present disclosure.

The therapeutic or prophylactic dose of the compound of the present disclosure may be determined by, for example, the specific use of a treatment or prevention, the mode of administration of the compound, the health and condition of a patient, and the judgment of a prescribing physician. The proportion or concentration of the compound of the present disclosure in a pharmaceutical composition may not be constant and depends on a variety of factors including doses, chemical properties (e.g., hydrophobicity), and routes of administration. For example, the compound of the present disclosure may be provided for parenteral administration by a physiological buffered aqueous solution containing about 0.1%-10% w/v of the compound. Some typical dose ranges are about 0.001 mg/kg body weight/day to about 1000 mg/kg body weight/day. The dose is likely to depend on variables such as the type and degree of progression of the disease or disorder, the general health condition of a specific patient, the relative biological potency of the compound selected, the excipient formulation, and its route of administration. Effective doses can be extrapolated from dose-response curves derived from *in-vitro* or animal model test systems. The word "comprise" and variations thereof such as "comprises" or "comprising" will be understood in an open, non-exclusive sense, i.e., "including but not limited to".

The "pharmaceutical composition" refers to a composition comprising one or more of the compound, the isomer thereof, or the pharmaceutically acceptable salt thereof described in the present disclosure, as well as other components such as a physiologically/pharmaceutically acceptable carrier and excipient. The pharmaceutical composition is intended to promote the administration to an organism and facilitate the absorption of the active ingredient so that it can exert its biological activity.

The pharmaceutical composition of the present disclosure may be prepared by combining the compound of the present disclosure with a suitable pharmaceutically acceptable excipient.

In some embodiments, the pharmaceutical composition is in an oral form. For oral administration, the pharmaceutical composition can be formulated by mixing the active compounds with pharmaceutically acceptable excipients well known in the art.

The compounds of the present disclosure can be prepared using a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions in the specific embodiments of the present disclosure are conducted in a proper solvent that must be suitable for the chemical changes in the present disclosure and the reagents and materials required. In order to obtain the compounds of the present disclosure, it is sometimes necessary for those skilled in the art to modify or select a synthetic procedure or a reaction process based on existing embodiments.

The starting materials or intermediates used in the embodiments of the present disclosure may be obtained commercially or prepared using methods in the prior art.

An important consideration in synthetic route planning in the art is the selection of proper protecting groups for reactive functional groups (e.g., amino in the present disclosure). For example, reference may be made to Greene's Protective Groups in Organic Synthesis (4th Ed.). Hoboken, New Jersey: John Wiley & Sons, Inc.

In some embodiments, some of the compounds of the present disclosure can be prepared by those skilled in the art of organic synthesis by reference to the following routes: 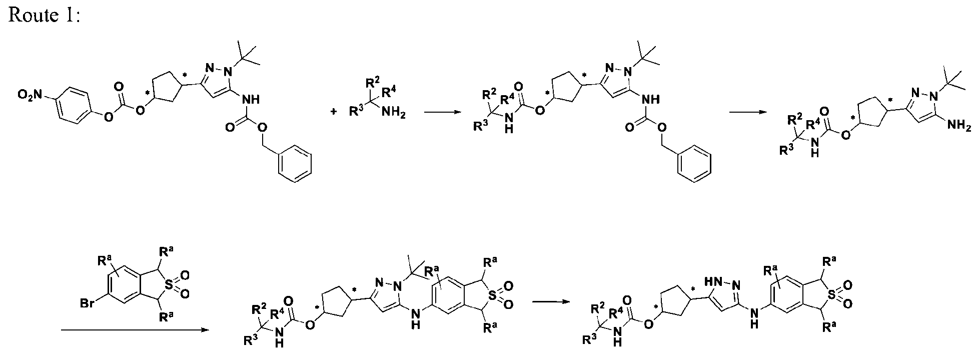 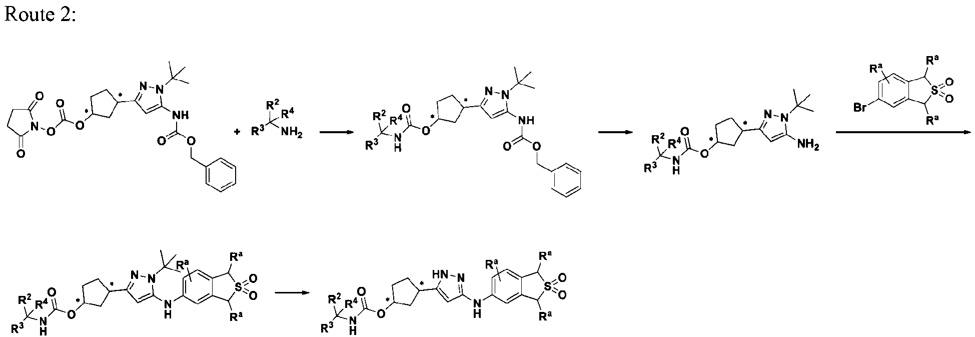 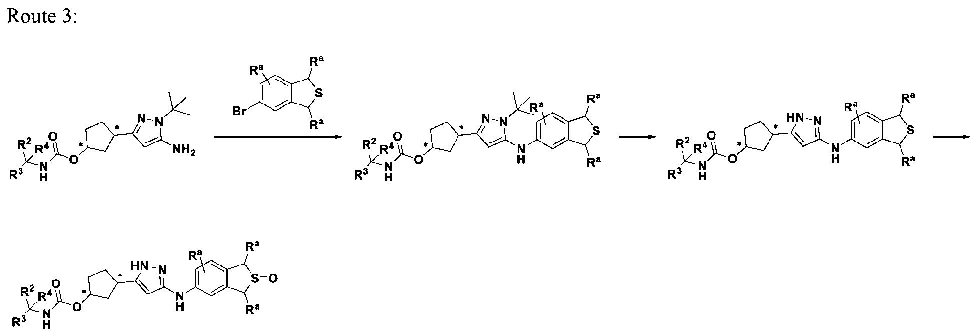 wherein, R², R³, R⁴, and R^{a} are as defined in the compound of formula (I).

The compound disclosed herein may be structurally confirmed by conventional methods well known to those skilled in the art; if the present application relates to the absolute configuration of the compound, this absolute configuration may be confirmed by means of conventional techniques in the art, for example, single crystal X-ray diffraction (SXRD) and microcrystalline electron diffraction.

For clarity, the present disclosure is further described with the following examples, which are, however, not intended to limit the scope of the present disclosure. All the reagents used in the present disclosure are commercially available and can be used without further purification.

### DETAILED DESCRIPTION

The present disclosure is described in detail below by way of examples. However, this is by no means disadvantageously limiting the scope of the present disclosure. Although the present disclosure has been described in detail herein and specific embodiments thereof have also been disclosed, it will be apparent to those skilled in the art that various changes and modifications can be made to the specific embodiments disclosed herein without departing from the spirit and scope of the present disclosure.

### Example 1: Preparation of compound 1

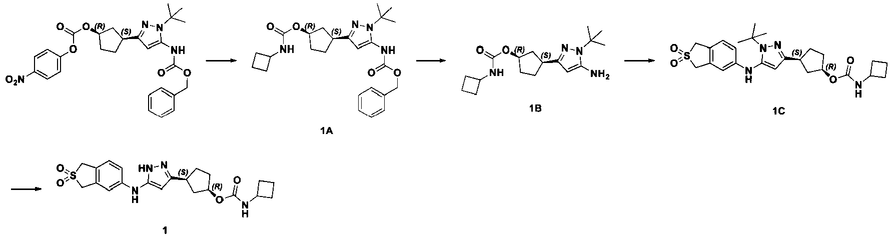

Benzyl (1-(tert-butyl)-3-((1S,3R)-3-(4-nitrophenoxy)carbonyl)oxy)cyclopentyl)-1H-pyrazol-5-yl)carbamate (0.95 g), N,N-diisopropylethylamine (0.70 g), and cyclobutanamine (0.20 g) were added to tetrahydrofuran (30 mL), and the mixture was stirred at room temperature for reaction. After the reaction was completed, the reaction solution was concentrated by evaporation at reduced pressure to remove the solvent, and ethyl acetate was added to the residue. The mixture was washed with an aqueous sodium hydroxide solution (1 N) and saturated brine, dried over anhydrous sodium sulfate, filtered, and concentrated to give intermediate **1A** (0.90 g). MS (ESI):m/z 455.3 [M+H]⁺. Intermediate **1A** (0.826 g) and palladium on carbon (palladium content 5%, water content 50% (w/w), 0.15 g) were added to tetrahydrofuran (10 mL) and ethyl acetate (10 mL), and the mixture was stirred at room temperature in a hydrogen atmosphere. After the reaction was completed, the reaction solution was filtered and concentrated to give intermediate **1B** (0.435 g). MS (ESI): m/z 321.18 [M+H]⁺.

Intermediate **1B** (0.3 g), 5-bromo-1,3-dihydrobenzo[c]thiophene-2,2-dioxide (0.347 g), chloro(2-dicyclohexylphosphine-2',4',6'-triisopropyl-1,1'-biphenyl)[2-(2'-amino-1,1'-biphenyl)]palladium (II) (0.11 g), 2-dicyclohexylphosphine-2',4',6'-triisopropylbiphenyl (0.067 g), and potassium phosphate (0.596 g) were added to N,N-dimethylformamide (30 mL). After the addition was completed, the mixture was stirred at 110 °C in a nitrogen atmosphere. After the reaction was completed, the reaction solution was filtered, concentrated, and separated by column chromatography (developing solvents: dichloromethane/methanol) to give intermediate 1C (0.35 g). MS (ESI): m/z 487.30 [M+H]⁺.

Intermediate **1C** (0.35 g) was added to ethanol (2 mL) and diluted hydrochloric acid (1 N, 20 mL), and the mixture was stirred at 100 °C in microwave. After the reaction was completed, the reaction solution was concentrated by evaporation at reduced pressure to remove the solvent, and the pH of the residue was adjusted to alkalinity with a saturated aqueous sodium bicarbonate solution. The mixture was extracted with dichloromethane, dried over anhydrous sodium sulfate, filtered, concentrated, and purified by medium and low pressure preparative liquid phase chromatography (C18 column, acetonitrile/water (20/80), the eluent contained one thousandth of ammonium hydroxide) to give compound **1** (65 mg). MS (ESI): m/z 431.1753 [M+H]⁺.

¹H NMR (500 MHz, DMSO) δ 11.73 (s, 1H), 8.47 (s, 1H), 7.54 - 7.28 (m, 2H), 7.22 (d, 1H), 7.14 (d, 1H), 5.63 (s, 1H), 4.98 (d, 1H), 4.40 (s, 2H), 4.32 (s, 2H), 3.97-3.91 (m, 1H), 3.10 - 2.97 (m, 1H), 2.44 (td, 1H), 2.09 (ddd, 2H), 2.01 (q, 1H), 1.88 (p, 3H), 1.71 (q, 2H), 1.55 (dp, 3H).

### Example 2: Preparation of compound 2

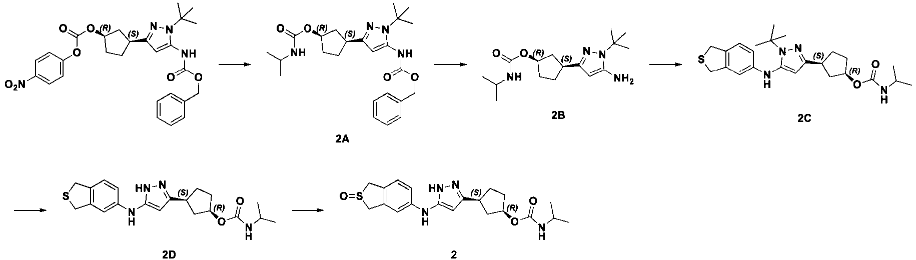

The key intermediate **2D** (75 mg) of Example 2 was obtained with reference to the preparation of compound 1 in Example 1, with cyclobutanamine being replaced by isopropylamine and 5-bromo-1,3-dihydrobenzo[c]thiophene-2,2-dioxide being replaced by 5-bromo-1,3-dihydrobenzo[c]thiophene. MS (ESI): m/z 387.29[M+H]⁺.

**2D** (75 mg) and m-chloroperoxybenzoic acid (30 mg) were added to a solution of dichloromethane (30 mL) at - 15 °C, and the mixture was stirred for reaction. After the reaction was completed, water was added to quench the reaction, and the resultant mixture was extracted with ethyl acetate, dried over anhydrous sodium sulfate, and separated by column chromatography (developing solvents: dichloromethane/methanol) to give compound **2** (30 mg) of Example 2. MS (ESI): m/z 403.1803 [M+H]⁺

¹H NMR (500 MHz, DMSO-d6) δ 11.68 (s, 1H), 8.37 (s, 1H), 7.42 (s, 1H), 7.18 (d, 2H), 6.94 (s, 1H), 5.62 (s, 1H), 4.99 (s, 1H), 4.31 - 4.23 (m, 2H), 3.96 - 3.87 (m, 2H), 3.56 - 3.59(m, 1H), 3.03 (q, 1H), 2.43 - 2.50(m, 1H), 2.02 - 2.00 (m, 1H), 1.99 (ddd, 1H), 1.92 - 1.86 (m, 3H), 1.73 (s, 6H).

### Example 3: Preparation of compound 3

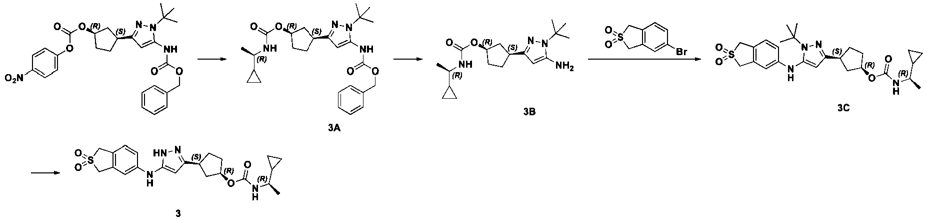

Compound 3 (21 mg) of Example 3 was obtained with reference to the preparation of compound 1 in Example 1, with cyclobutanamine being replaced by (S)-1-cyclopropylethylamine hydrochloride. MS (ESI): m/z 445.1906 [M+H]⁺.

¹H NMR (500 MHz, DMSO-d6) δ 11.73 (s, 1H), 8.47 (s, 1H), 7.40 (s, 1H), 7.21 (d, 1H), 7.14 (d, 1H), 7.00 (d, 1H), 5.63 (s, 1H), 4.99 (d, 1H), 4.40 (s, 2H), 4.32 (s, 2H), 3.02 (dt, 2H), 2.47 - 2.39 (m, 1H), 2.01 (q, 1H), 1.90 (q, 1H), 1.76 - 1.68 (m, 2H), 1.60 (s, 1H), 1.08 (d, 3H), 0.81 (s, 1H), 0.42 - 0.29 (m, 2H), 0.24 (dd, 1H), 0.10 (dd, 1H).

### Example 4: Preparation of compound 4

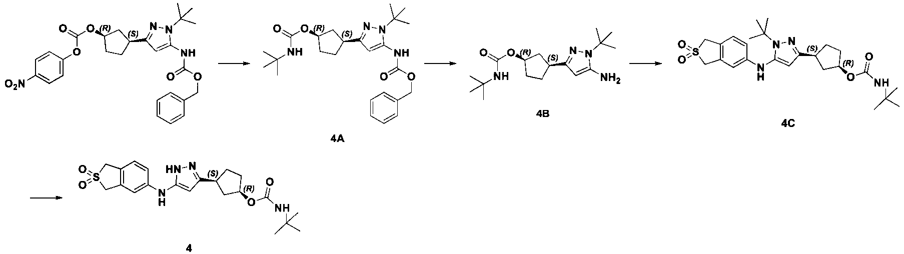

Compound **4** (15 mg) of Example 4 was obtained with reference to the preparation of compound **1** in Example 1, with cyclobutanamine being replaced by tert-butylamine. MS (ESI): m/z 433.1903 [M+H]⁺.

¹H NMR (500 MHz, DMSO-d6) δ 11.72 (s, 1H), 8.46 (s, 1H), 7.40 (s, 1H), 7.21 (d, 1H), 7.14 (d, 1H), 6.76 (s, 1H), 5.63 (s, 1H), 4.97 (s, 1H), 4.40 (s, 2H), 4.32 (s, 2H), 3.02 (q, 1H), 2.45 (dt, 1H), 2.01 (q, 1H), 1.89 (ddd, 1H), 1.71 (dt, 2H), 1.58 (s, 1H), 1.21 (s, 9H).

### Example 5: Preparation of compound 5

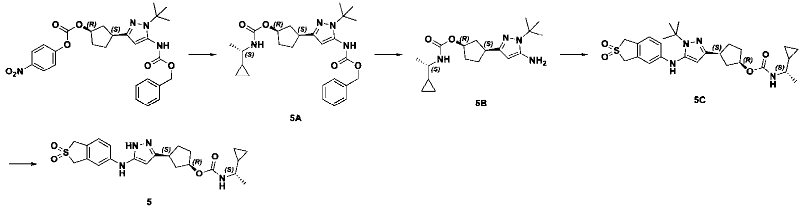

Compound **5** (15 mg) of Example 5 was obtained with reference to the preparation of compound **1** in Example 1, with cyclobutanamine being replaced by (R)-1-cyclopropylethylamine hydrochloride. MS (ESI): m/z 445.1906 [M+H]⁺.

¹H NMR (500 MHz, DMSO-d6) δ 11.73 (s, 1H), 8.47 (s, 1H), 7.40 (s, 1H), 7.21 (d, 1H), 7.14 (d, 1H), 7.00 (d, 1H), 5.63 (s, 1H), 4.99 (d, 1H), 4.40 (s, 2H), 4.32 (s, 2H), 3.02 (dt, 2H), 2.47 - 2.39 (m, 1H), 2.01 (q, 1H), 1.90 (q, 1H), 1.76 - 1.68 (m, 2H), 1.60 (s, 1H), 1.08 (d, 3H), 0.81 (s, 1H), 0.42 - 0.29 (m, 2H), 0.24 (dd, 1H), 0.10 (dd, 1H).

### Example 6: Preparation of compound 6

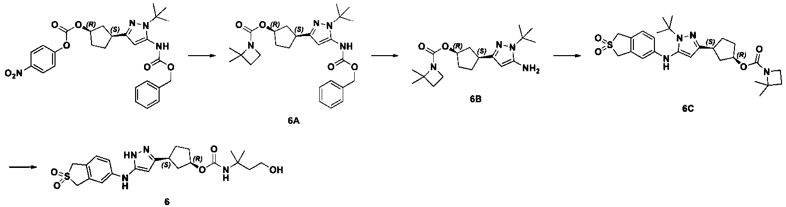

Compound 6 (5 mg) of Example 6 was obtained with reference to the preparation of compound 1 in Example 1, with cyclobutanamine being replaced by 2,2-dimethylazetidine hydrochloride. MS (ESI): m/z 463.2017 [M+H]⁺.

### Example 7: Preparation of compound 7

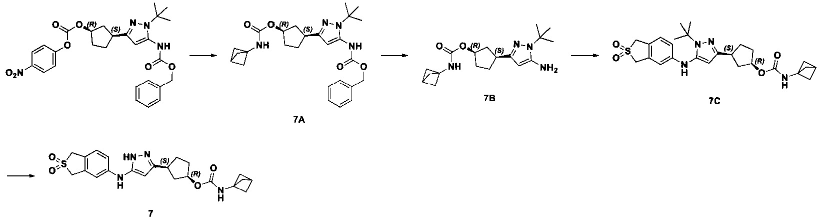

Compound **7** (30 mg) of Example 7 was obtained with reference to the preparation of compound **1** in Example 1, with cyclobutanamine being replaced by bicyclo[1.1.1]pentan-1-amine hydrochloride. MS (ESI): m/z 443.1758 [M+H]⁺.

¹H NMR (500 MHz, DMSO-d6) δ 11.73 (s, 1H), 8.47 (s, 1H), 7.75 (s, 1H), 7.40 (s, 1H), 7.21 (d, J = 8.5 Hz, 1H), 7.13 (d, J = 8.4 Hz, 1H), 5.63 (s, 1H), 4.99 (s, 1H), 4.40 (s, 2H), 4.32 (s, 2H), 3.03 (m, 1H), 2.49 - 2.41 (m, 1H), 2.35 (s, 1H), 2.01 (s, 1H), 1.90 (m, 7H), 1.71 (m, 2H), 1.59 (s, 1H).

### Example 8: Preparation of compound 8

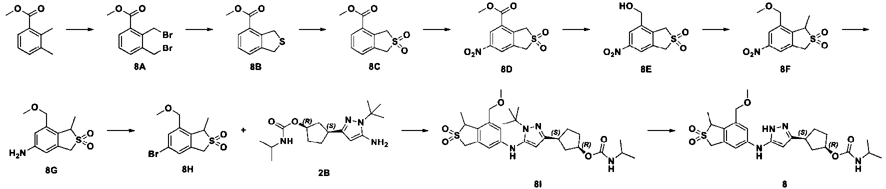

Methyl 2,3-dimethylbenzoate (14 g), N-bromosuccinimide (30 g), and benzoyl peroxide (1.02 g) were added to 1,2-dichloroethane (40 mL), and the mixture was stirred at 80 °C. After the reaction was completed, the reaction solution was concentrated by evaporation at reduced pressure to remove the solvent, and separated by column chromatography (developing solvents: petroleum ether/ethyl acetate) to give intermediate **8A** (28 g).

**8A** (26 g) and sodium sulfide nonahydrate (24 g) were added to ethanol (500 mL), and the mixture was stirred at room temperature for reaction. After the reaction was completed, the reaction solution was concentrated by evaporation at reduced pressure to remove the solvent, washed with water, extracted with dichloromethane, dried over anhydrous sodium sulfate, and separated by column chromatography (developing solvents: petroleum ether/ethyl acetate) to give intermediate **8B** (6.71 g).

¹H NMR (500 MHz, DMSO-d6) δ 7.83 (d, 1H), 7.58 (d, 1H), 7.39 (t, 1H), 4.51 (s, 2H), 4.31 (s, 2H), 3.86 (s, 3H). **8B** (6.71 g) and m-chloroperoxybenzoic acid (21 g) were added to dichloromethane (250 mL) at -20 °C, and the mixture was stirred for reaction. After the reaction was completed, the reaction solution was washed with a saturated aqueous sodium bicarbonate solution, extracted with dichloromethane, dried over anhydrous sodium sulfate, and separated by column chromatography (developing solvents: petroleum ether/ethyl acetate) to give intermediate **8C** (5.4 g).

¹H NMR (500 MHz, DMSO-d6) δ 7.96 (d, 1H), 7.66 (d, 1H), 7.54 (t, 1H), 4.72 (s, 2H), 4.57 (s, 2H), 3.88 (s, 3H) Concentrated nitric acid (2.5 g) was slowly and dropwise added to a solution **of 8C** (5.4 g) in concentrated sulfuric acid (30 mL) with stirring in an ice bath. After the reaction was completed, the reaction solution was extracted with ethyl acetate, washed with saturated brine, dried over anhydrous sodium sulfate, and separated by column chromatography (developing solvents: petroleum ether/ethyl acetate) to give intermediate **8D** (4.56 g).

¹H NMR (500 MHz, DMSO) δ 8.62 (d, J = 2.4 Hz, 1H), 8.57 - 8.55 (m, 1H), 4.89 (s, 2H), 4.72 (s, 2H), 3.93 (s, 3H).

Diisobutylaluminum hydride (28 mL, 1.5 M) was slowly and dropwise added to a solution of **8D** (4.56 g) in tetrahydrofuran (100 mL) at -5 °C, and the mixture was stirred for reaction. After the reaction was completed, the reaction solution was washed with a saturated aqueous potassium sodium tartrate solution, extracted with ethyl acetate, dried over anhydrous sodium sulfate, and separated by column chromatography (developing solvents: dichloromethane/methanol) to give intermediate **8E** (3.5 g).

1H NMR (500 MHz, DMSO) δ 8.24 (d, J = 2.4 Hz, 1H), 8.19 (d, J = 2.4 Hz, 1H), 5.65 (t, J = 5.6 Hz, 1H), 4.67 (s, 2H), 4.62 (s, 2H), 4.60 (d, J = 5.5 Hz, 2H).

Iodomethane (3.05 g) was slowly and dropwise added to a solution of **8E** (3.5 g) and sodium hydride (1.72 g) in DMF (50 mL) at -5 °C, and the mixture was stirred for reaction. After the reaction was completed, water was added to quench the reaction, and the resultant mixture was extracted with ethyl acetate, dried over anhydrous sodium sulfate, and separated by column chromatography (developing solvents: petroleum ether/ethyl acetate) to give intermediate **8F** (2.0 g). MS (ESI):m/z:270.06[M-H]⁻.

Pd-C (0.8 g) was slowly added to a solution of **8F** (2.0 g) in tetrahydrofuran (50 mL). The mixture was purged with hydrogen 2-3 times and then stirred at room temperature for reaction. After the reaction was completed, the reaction solution was filtered and concentrated to give intermediate **8G** (1.8 g).

**8G** (1 g), copper bromide (1.24 g), and tert-butyl nitrite (0.67 g) were added to acetonitrile (15 mL), and the mixture was reacted at 40 °C in an oil bath in a nitrogen atmosphere. After the reaction was completed, the reaction solution was concentrated by evaporation at reduced pressure to remove the solvent, and separated by column chromatography (developing solvents: dichloromethane/methanol) to give intermediate **8H** (800 mg).

Compound **8** (18 mg) of Example 8 was obtained with reference to the preparation of compound 1 in Example 1, with intermediate 5-bromo-1,3-dihydrobenzo[c]thiophene-2,2-dioxide being replaced by intermediate 8H. MS (ESI): m/z 477.2172[M+H]⁺

¹H NMR (500 MHz, DMSO-d6) δ 11.72 (s, 1H), 8.46 (s, 1H), 7.33 (s, 1H), 7.25 (s, 1H), 6.94 (d, 1H), 5.62 (s, 1H), 5.27 (t, 1H), 5.02 - 4.95 (m, 1H), 4.54 (d, 2H), 4.41 (s, 2H), 3.58 (dd, 1H), 3.07 - 2.99 (m, 1H), 2.44 (dd, 1H), 2.01 (q, 1H), 1.90 (ddd, 1H), 1.70 (dd, 2H), 1.61 (d, 1H), 1.56 (s, 6H), 1.03 (dd, 6H).

### Example 9: Preparation of compound 9

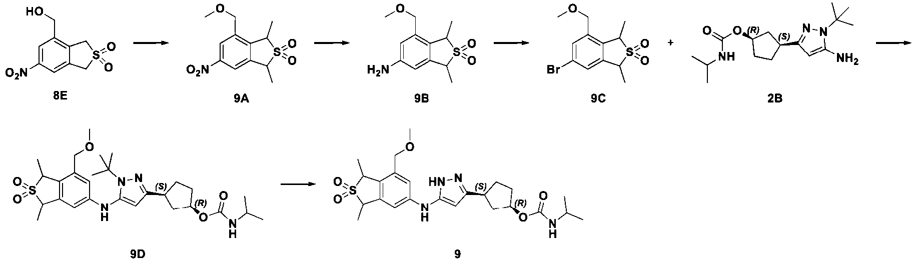

Iodomethane (3.0 g) was slowly and dropwise added to a solution of **8E** (1.75 g) and sodium hydride (0.85 g) in DMF (30 mL) at -5 °C, and the mixture was stirred for reaction. After the reaction was completed, water was added to quench the reaction, and the resultant mixture was extracted with ethyl acetate, dried over anhydrous sodium sulfate, and separated by column chromatography (developing solvents: petroleum ether/ethyl acetate) to give intermediate **9A** (1.0 g).

Compound **9** (18 mg) of Example 9 was obtained with reference to the preparation of compound **8** in Example 8, with intermediate **8F** being replaced by intermediate **9A.** MS (ESI): m/z m/z: 491.2330[M+H]⁺

¹H NMR (500 MHz, DMSO-d6) δ 11.77 (s, 1H), 8.49 (s, 1H), 7.32 (d, 2H), 6.94 (d, 1H), 5.62 (s, 1H), 5.28 (s, 1H), 5.00 (s, 1H), 4.61 (d, 1H), 4.53 - 4.44 (m, 2H), 3.63 - 3.52 (m, 1H), 3.09 - 3.00 (m, 1H), 2.46 (dt, 1H), 2.03 - 1.98 (m, 1H), 1.94 - 1.87 (m, 1H), 1.72 (d, 2H), 1.61 (s, 3H), 1.56 (s, 3H), 1.50 (d, 3H), 1.04 (dd, 6H).

### Example 10: Preparation of compound 10

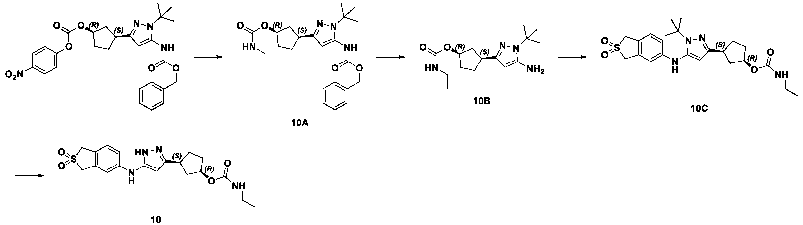

Compound **10** (150 mg) of Example 10 was obtained with reference to the preparation of compound 1 in Example 1, with cyclobutanamine being replaced by ethylamine. MS (ESI): m/z 405.1598[M+H]⁺.

¹H NMR (500 MHz, DMSO-d6) δ 11.73 (s, 1H), 8.47 (s, 1H), 7.41 (s, 1H), 7.22 (d, 1H), 7.13 (d, 1H), 7.02 (t, 1H), 5.63 (d, 1H), 4.99 (q, 1H), 4.40 (s, 2H), 4.32 (s, 2H), 3.04 (t, 1H), 2.99 (p, 2H), 2.45 (dt, 1H), 2.01 (q, 1H), 1.91 (q, 1H), 1.79 - 1.68 (m, 2H), 1.61 (d, 1H), 0.99 (t, 3H).

### Example 11: Preparation of compound 11

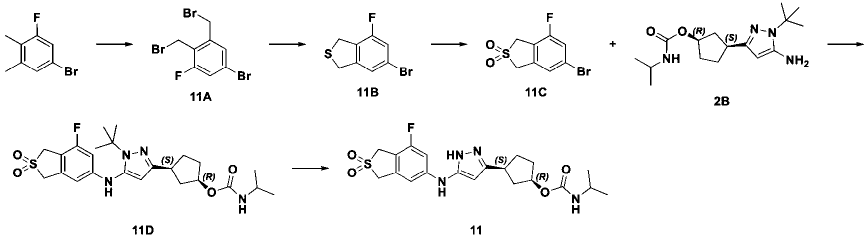

The key intermediate **11C** (410 mg) of Example 11 was obtained with reference to the preparation of compound 8 in Example 8, with methyl 2,3-dimethylbenzoate being replaced by 5-bromo-1-fluoro-2,3-dimethylbenzene.

Compound **11** (80 mg) of Example 11 was obtained with reference to the preparation of compound **1** in Example 1, with intermediate 5-bromo-1,3-dihydrobenzo[c]thiophene-2,2-dioxide being replaced by intermediate **11C.** MS (ESI): m/z 437.1656[M+H]⁺

1H NMR (500 MHz, DMSO-d6) δ 11.83 (s, 1H), 8.79 (s, 1H), 7.36 - 7.24 (m, 1H), 7.09 (s, 1H), 6.93 (d, 1H), 5.64 (d, 1H), 4.99 (s, 1H), 4.48 (s, 2H), 4.35 (s, 2H), 3.13 - 2.99 (m, 1H), 2.46 (p, 1H), 2.01 (q, 1H), 1.89 (ddt, 1H), 1.72 (d, 2H), 1.59 (s, 1H), 1.03 (dd, 6H).

### Example 12: Preparation of compound 12

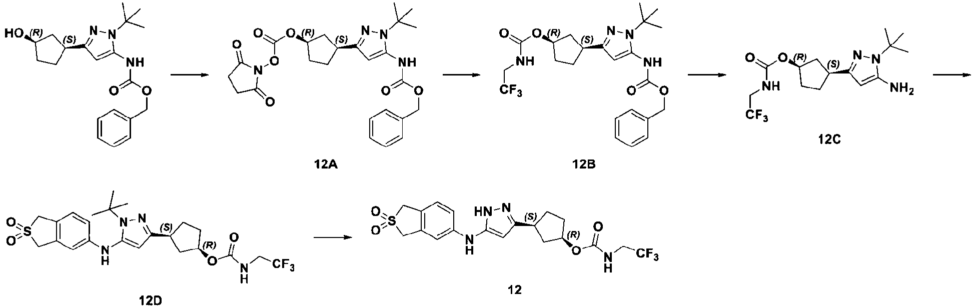

Benzyl (1-(tert-butyl)-3-((1S,3R)-3-hydroxycyclopentyl)-1H-pyrazol-5-yl)carbamate (2.00 g), triethylamine (1.70 g), and bis(2,5-dioxopyrrolidin-1-yl)carbonate (4.30 g) were added to acetonitrile (20 mL), and the mixture was stirred at 40 °C. After the reaction was completed, the reaction solution was concentrated by evaporation at reduced pressure to remove the solvent, and separated by column chromatography (developing solvents: petroleum ether/ethyl acetate) to give intermediate **12A** (1.73 g). MS (ESI): m/z 499.32 [M+H]⁺.

Compound **12** of Example 12 (44 mg) was obtained with reference to the preparation of compound **1** in Example 1, with cyclobutanamine being replaced by trifluoroethylamine and benzyl (1-(tert-butyl)-3-((1S,3R)-3-(4-nitrophenoxy)carbonyl)oxy)cyclopentyl)-1H-pyrazol-5-yl)carbamate being replaced by intermediate **12A.** MS (ESI): m/z 459.1316[M+H]⁺

¹H NMR (500 MHz, DMSO-d6) δ 11.74 (s, 1H), 8.47 (s, 1H), 7.87 (t, 1H), 7.41 (s, 1H), 7.22 (d, 1H), 7.14 (d, 1H), 5.64 (s, 1H), 5.06 (qd, 1H), 4.40 (s, 2H), 4.32 (s, 2H), 3.76 (td, 2H), 3.06 (p, 1H), 2.49 - 2.45 (m, 1H), 2.01 (dd, 1H), 1.98 - 1.89 (m, 1H), 1.75 (qd, 2H), 1.67 - 1.59 (m, 1H).

### Example 13: Preparation of compound 13

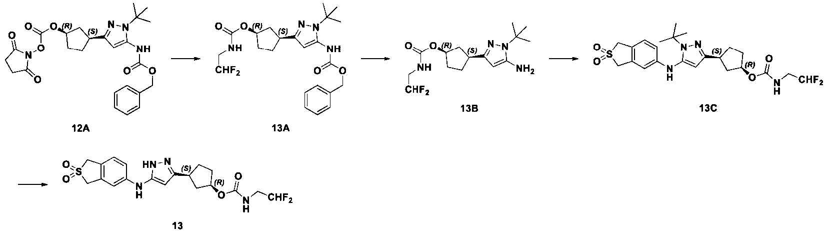

Compound **13** (60 mg) of Example 13 was obtained with reference to the preparation of compound **12** in Example 12, with trifluoroethylamine being replaced by difluoroethylamine. MS (ESI): m/z 441.1408 [M+H]⁺

¹H NMR (500 MHz, DMSO-d6) δ 11.73 (s, 1H), 8.46 (s, 1H), 7.51 (t, 1H), 7.41 (s, 1H), 7.22 (d, 1H), 7.13 (d, 1H), 6.14 - 5.81 (m, 1H), 5.64 (s, 1H), 5.03 (tdd, 1H), 4.40 (s, 2H), 4.32 (s, 2H), 3.43 - 3.33 (m, 2H), 3.05 (p, 1H), 2.49 - 2.36 (m, 1H), 2.02 (q, 1H), 1.97 - 1.86 (m, 1H), 1.73 (td, 2H), 1.67 - 1.58 (m, 1H).

### Example 14: Preparation of compound 14

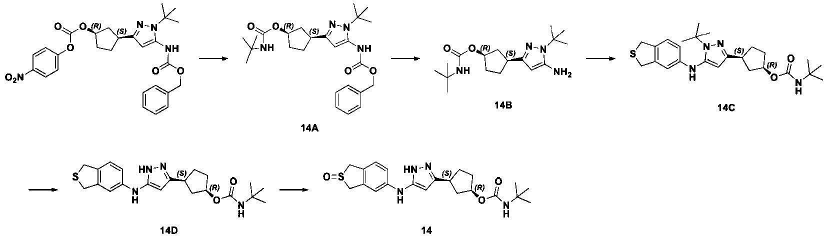

Compound **14** (60 mg) of Example 14 was obtained with reference to the preparation of compound **2** in Example 2, with isopropylamine being replaced by tert-butylamine. MS (ESI): m/z 417.1954 [M+H]⁺

¹H NMR (500 MHz, DMSO-d6) δ 11.69 (s, 1H), 8.37 (s, 1H), 7.41 (s, 1H), 7.18 (d, 2H), 6.76 (s, 1H), 5.62 (s, 1H), 4.97 (s, 1H), 4.36 - 4.18 (m, 2H), 4.03 - 3.84 (m, 2H), 3.02 (q, 1H), 2.46 (dt, 1H), 2.08 - 1.96 (m, 1H), 1.89 (ddd, 1H), 1.77 - 1.52 (m, 3H), 1.21 (s, 9H).

### Example 15: Preparation of compound 15

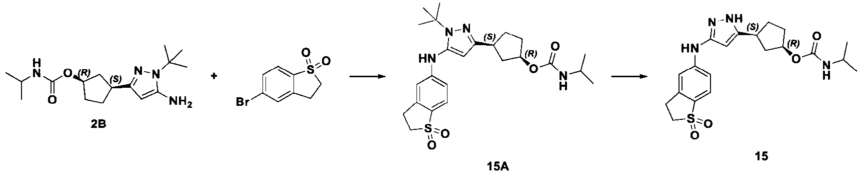

Compound **15** (30 mg) of Example 15 was obtained with reference to the preparation of compound **1** in Example 1, with 5-bromo-1,3-dihydrobenzo[c]thiophene-2,2-dioxide being replaced by intermediate 5-bromo-2,3-dihydrobenzothiophene 1,1-dioxide and intermediate 1B being replaced by 2B. MS (ESI): m/z 419.1752 [M+H]^{+ 1}H NMR (500 MHz, DMSO-d6) δ 11.90 (s, 1H), 8.99 (s, 1H), 7.52 - 7.41 (m, 2H), 7.29 (d, J = 8.0 Hz, 1H), 6.94 (d, J = 7.8 Hz, 1H), 5.70 (s, 1H), 5.00 (s, 1H), 3.58 (m, 1H), 3.47 (m, 2H), 3.24 (t, J = 6.9 Hz, 2H), 3.10 - 3.01 (m, 1H), 2.49 - 2.41 (m, 1H), 2.02 (m, 1H), 1.90 (m, 1H), 1.73 (m, 2H), 1.60 (s, 1H), 1.03 (m, 6H).

### Example 16: Preparation of compound 16

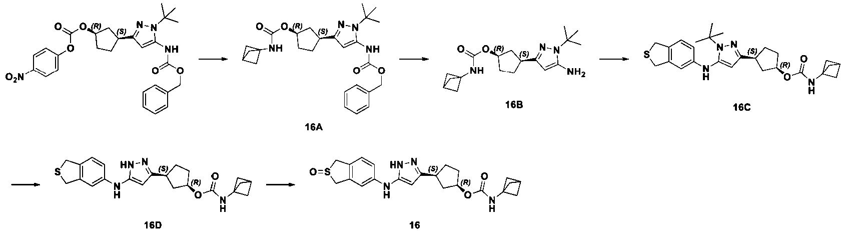

Compound **16** (35 mg) of Example 16 was obtained with reference to the preparation of compound **2** in Example 2, with isopropylamine being replaced by bicyclo[1.1.1]pentan-1-amine hydrochloride. MS (ESI): m/z 427.1804[M+H]⁺

¹H NMR (500 MHz, DMSO-d6) δ 11.69 (s, 1H), 8.37 (s, 1H), 7.59 (d, 2H), 7.18 (d, 2H), 5.62 (s, 1H), 4.99 (s, 1H), 4.27 (dd, 2H), 3.91 (dd, 2H), 3.13 - 2.95 (m, 1H), 2.49 - 2.40 (m, 1H), 2.35 (s, 1H), 1.98 (d, 8H), 1.66 (d, 3H).

### Example 17: Preparation of compound 17

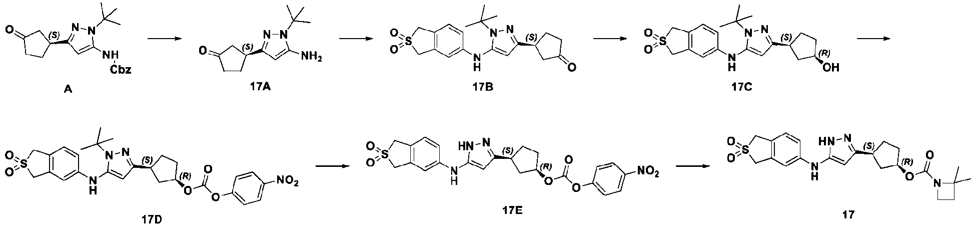

Intermediate A (5 g) and palladium on carbon (palladium content 5%, water content 50% (w/w), 0.5 g) were added to tetrahydrofuran (50 mL) and ethyl acetate (50 mL), and the mixture was stirred at room temperature in a hydrogen atmosphere. After the reaction was completed, the reaction solution was filtered and concentrated to give intermediate **17A** (2.9 g). MS (ESI): m/z 222.31 [M+H]⁺.

**17A** (2.9 g), 5-bromo-1,3-dihydrobenzo[c]thiophene-2,2-dioxide (5 g), chloro(2-dicyclohexylphosphine-2',4',6'-triisopropyl-1,1'-biphenyl)[2-(2'-amino-1,1'-biphenyl)]palladium (II) (2.1 g), 2-dicyclohexylphosphine-2',4',6'-triisopropylbiphenyl (1.3 g), and potassium phosphate (8.6 g) were added to 1,4-dioxane (100 mL). After the addition was completed, the mixture was stirred at 110 °C in a nitrogen atmosphere. After the reaction was completed, the reaction solution was filtered, concentrated, and separated by column chromatography (developing solvents: dichloromethane/methanol) to give intermediate **17B** (4 g). MS (ESI): m/z 388.25 [M+H]⁺.

Lithium triethylborohydride (25.8 mL, 1 mol/L) was slowly and dropwise added to a solution of **17B** (4 g) in tetrahydrofuran (50 mL) with stirring at -78 °C. After the reaction was completed, the reaction was quenched with a saturated aqueous sodium bicarbonate solution, and the resultant mixture was extracted with ethyl acetate, dried over anhydrous sodium sulfate, and separated by column chromatography (developing solvents: petroleum ether/ethyl acetate) to give intermediate **17C** (1.65 g). MS (ESI): m/z 390.26 [M+H]⁺.

**17C** (1.65 g), p-nitrochloroformate (0.9 g), 4-dimethylaminopyridine (0.1 g), and pyridine (1 g) were added to dichloromethane (30 mL), and the mixture was stirred at room temperature for reaction. After the reaction was completed, the reaction solution was concentrated by evaporation at reduced pressure to remove the solvent, and separated by column chromatography (developing solvents: petroleum ether/ethyl acetate) to give intermediate **17D** (1.02 g). MS (ESI): m/z 555.25 [M+H]⁺.

**17D** (1.02 g) was added to formic acid (50 mL), and the mixture was stirred at 100 °C. After the reaction was completed, the reaction solution was concentrated by evaporation at reduced pressure to remove the solvent, and separated by column chromatography (developing solvents: petroleum ether/ethyl acetate) to give intermediate **17E** (0.25 g). MS (ESI): m/z 499.20 [M+H]⁺.

**17E** (0.25 g), N,N-diisopropylethylamine (0.32 g), and 2,2-dimethylazetidine hydrochloride (0.18 g) were added to tetrahydrofuran (10 mL), and the mixture was stirred at room temperature for reaction. After the reaction was completed, the reaction solution was concentrated by evaporation at reduced pressure to remove the solvent, and separated by column chromatography (developing solvents: dichloromethane/methanol) to give compound **17** (30 mg) of Example 17. MS (ESI): m/z 445.1904 [M+H]⁺

¹H NMR (500 MHz, DMSO-d6) δ 11.76 (s, 1H), 8.46 (s, 1H), 7.46 - 7.34 (m, 1H), 7.21 (s, 1H), 7.13 (d, J = 8.4 Hz, 1H), 5.62 (d, J = 5.2 Hz, 1H), 5.06 - 4.94 (m, 1H), 4.40 (s, 2H), 4.32 (s, 2H), 3.69 (m, 2H), 3.06 (m, 1H), 2.42 (m, 1H), 2.08 - 1.98 (m, 1H), 1.98 - 1.91 (m, 2H), 1.87 (s, 1H), 1.73 (m, 2H), 1.65 (m, 1H), 1.39 - 1.31 (m, 6H).

### Example 18: Preparation of compound 18

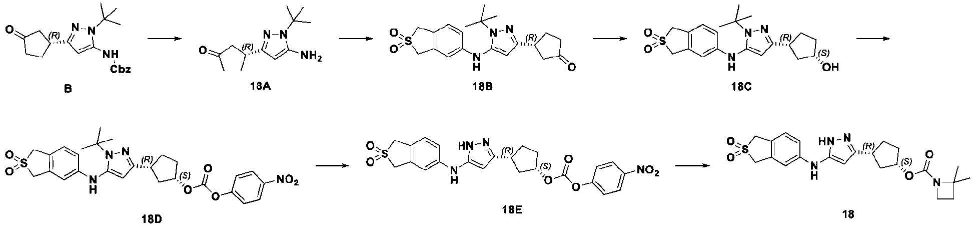

Compound **18** (35 mg) of Example 18 was obtained with reference to the preparation of compound 17 in Example 17, with intermediate A being replaced by intermediate B. MS (ESI): m/z 445.1905 [M+H]⁺.

¹H NMR (500 MHz, DMSO-d6) δ 11.77 (s, 1H), 8.47 (s, 1H), 7.40 (s, 1H), 7.21 (s, 1H), 7.13 (d, J = 8.4 Hz, 1H), 5.65 (s, 1H), 5.12 - 4.87 (m, 1H), 4.40 (s, 2H), 4.32 (s, 2H), 3.77 - 3.61 (m, 2H), 3.05 (p, J = 8.5 Hz, 1H), 2.48 - 2.35 (m, 1H), 2.07 - 1.99 (m, 1H), 1.95 (t, J = 7.6 Hz, 2H), 1.92 - 1.82 (m, 1H), 1.73 (q, J = 9.9 Hz, 2H), 1.65 (td, J = 11.0, 4.0 Hz, 1H), 1.41 - 1.29 (m, 6H).

### Example 19: Preparation of compound 19

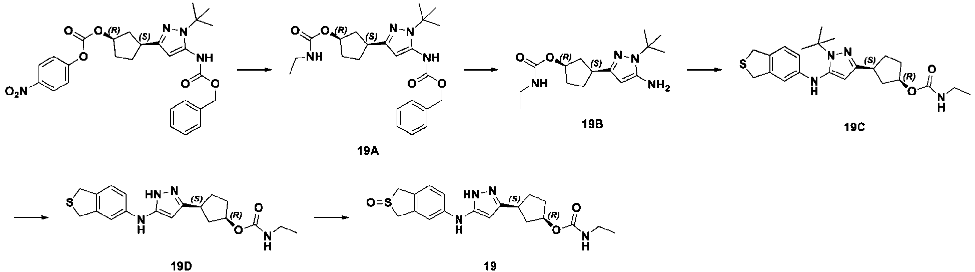

Compound **19** (85 mg) of Example 19 was obtained with reference to the preparation of compound 2 in Example 2, with isopropylamine being replaced by ethylamine. MS (ESI): m/z 389.1654 [M+H]⁺

¹H NMR (500 MHz, DMSO-d6) δ 11.69 (s, 1H), 8.37 (s, 1H), 7.42 (s, 1H), 7.18 (d, J = 4.5 Hz, 2H), 7.02 (m, 1H), 5.62 (s, 1H), 4.99 (m, 1H), 4.27 (m, 2H), 3.92 (m, 2H), 3.09 - 3.01 (m, 1H), 3.01 - 2.91 (m, 2H), 2.45 (m, 1H), 2.00 (m, 1H), 1.96 - 1.86 (m, 1H), 1.72 (m, 2H), 1.60 (m, 1H), 0.99 (m, 3H).

### Example 20: Preparation of compound 20

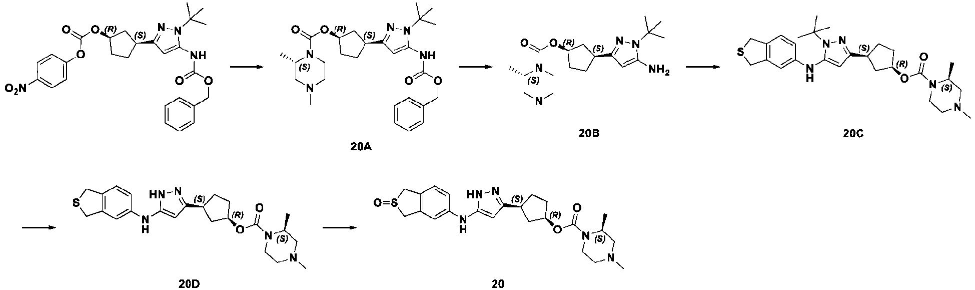

Compound **20** (25 mg) of Example 20 was obtained with reference to the preparation of compound **2** in Example 2, with isopropylamine being replaced by 3-S-1,3-dimethylpiperazine. MS (ESI): m/z 458.2236 [M+H]⁺

¹H NMR (500 MHz, DMSO-d6) δ 11.71 (s, 1H), 8.36 (s, 1H), 7.42 (s, 1H), 7.18 (q, J = 8.4 Hz, 2H), 5.61 (s, 1H), 5.03 (tdd, J = 6.6, 5.4, 2.4 Hz, 1H), 4.27 (dd, J = 24.4, 15.8 Hz, 2H), 4.06 (s, 1H), 3.91 (dd, J = 22.8, 15.8 Hz, 2H), 3.64 (d, J = 13.1 Hz, 1H), 3.08 (p, J = 8.1 Hz, 1H), 2.97 (td, J = 12.7, 3.4 Hz, 1H), 2.64 (d, J = 11.0 Hz, 1H), 2.54 (s, 1H), 2.42 (dt, J = 14.5, 8.0 Hz, 1H), 2.10 (s, 3H), 2.02 (q, J = 9.2 Hz, 1H), 1.91 (td, J = 13.0, 5.3 Hz, 2H), 1.80 - 1.67 (m, 4H), 1.12 (d, J = 6.8 Hz, 3H).

### Example 21: Preparation of compound 21

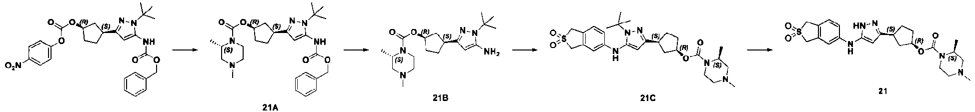

Compound **21** (19 mg) of Example 21 was obtained with reference to the preparation of compound **1** in Example 1, with cyclobutanamine being replaced by 3-S-1,3-dimethylpiperazine. MS (ESI): m/z 474.2183 [M+H]⁺.

¹H NMR (500 MHz, DMSO-d6) δ 11.75 (s, 1H), 8.46 (s, 1H), 7.40 (s, 1H), 7.27 - 7.10 (m, 2H), 5.68 (d, J = 56.2 Hz, 1H), 5.06 - 5.01 (m, 1H), 4.40 (s, 2H), 4.32 (s, 2H), 4.06 (s, 1H), 3.64 (d, J = 13.6 Hz, 1H), 3.09 (q, J = 8.3 Hz, 1H), 3.01 - 2.95 (m, 1H), 2.63 (d, J = 10.7 Hz, 1H), 2.55 (d, J = 16.5 Hz, 1H), 2.42 (dt, J = 14.2, 7.2 Hz, 1H), 2.10 (s, 3H), 2.04 - 1.98 (m, 1H), 1.92 (dd, J = 11.3, 4.1 Hz, 2H), 1.79 - 1.69 (m, 4H), 1.11 (d, J = 6.9 Hz, 3H).

### Example 22: Preparation of compounds 22 and 23

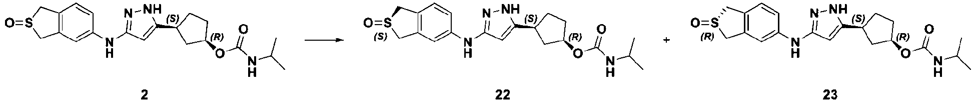

Compound **2** (100 mg) was prepared by chiral resolution with SFC. The chromatographic conditions were as follows:
Instrument: supercritical fluid chromatograph
Column: CHIRALART Cellulose-SB (Innovation 012, 30 × 250 mm, 5 µm)
Mobile phase A: carbon dioxide;
Mobile phase B: methanol;
Detection wavelength: 254 nm;
Compound 2 (0.1 g) was dissolved in 1.5 mL of dichloromethane, and the solution was filtered through a 0.45 µm organic membrane to give a filtrate.
Preparative gradient:
Injection volume = 1.5 mL
Isocratic elution (see table below):

| T(min) | Flow rate (mL/min) | A% | B% |
|---|---|---|---|
| 0 | 80 | 50 | 50 |
| 8 | 80 | 50 | 50 |

Compound **22** (45 mg) was obtained. MS (ESI): m/z 403.1804 [M+H]⁺; UPCC: Purity: 100%, RT = 2.025 min, 254 nm.

¹H NMR (500 MHz, DMSO-d6) δ 11.69 (s, 1H), 8.37 (s, 1H), 7.42 (s, 1H), 7.18 (q, J = 8.3 Hz, 2H), 6.94 (d, J = 7.8 Hz, 1H), 5.62 (s, 1H), 4.99 (s, 1H), 4.36 - 4.19 (m, 2H), 3.92 (dd, J = 25.3, 15.8 Hz, 2H), 3.58 (dd, J = 13.3, 6.7 Hz, 1H), 3.09 - 2.98 (m, 1H), 2.45 (dt, J = 14.4, 7.3 Hz, 1H), 2.01 (q, J = 8.2 Hz, 1H), 1.96 - 1.85 (m, 1H), 1.70 (dd, J = 15.6, 9.2 Hz, 2H), 1.60 (s, 1H), 1.03 (dd, J = 6.7, 1.9 Hz, 6H).

Compound **23** (45 mg). MS (ESI): m/z 403.1801 [M+H]⁺; UPCC: Purity: 100%, RT = 3.441 min, 254 nm

¹H NMR (500 MHz, DMSO-d6) δ 11.69 (s, 1H), 8.37 (s, 1H), 7.42 (s, 1H), 7.17 (t, J = 7.3 Hz, 2H), 6.94 (d, J = 7.8 Hz, 1H), 5.62 (s, 1H), 4.99 (s, 1H), 4.34 - 4.21 (m, 2H), 3.98 - 3.86 (m, 2H), 3.58 (dd, J = 13.7, 7.1 Hz, 1H), 3.11 - 2.98 (m, 1H), 2.45 (dt, J = 14.2, 7.4 Hz, 1H), 2.01 (q, J = 8.3 Hz, 1H), 1.96 - 1.86 (m, 1H), 1.78 - 1.68 (m, 2H), 1.60 (s, 1H), 1.03 (dd, J = 6.8, 1.9 Hz, 6H).

### UPCC detection method:

Column: CHIRALPAK IB-3 (4.6 × 100 mm, 3 µm)
Column temperature: 40 °C
Flow rate: 2.0 mL/min
ABPR: 1600 psi
Mobile phase: CO₂-methanol (60:40)

The mixture was subjected to isocratic elution for 10 min.

### Example 23: Preparation of compound 24

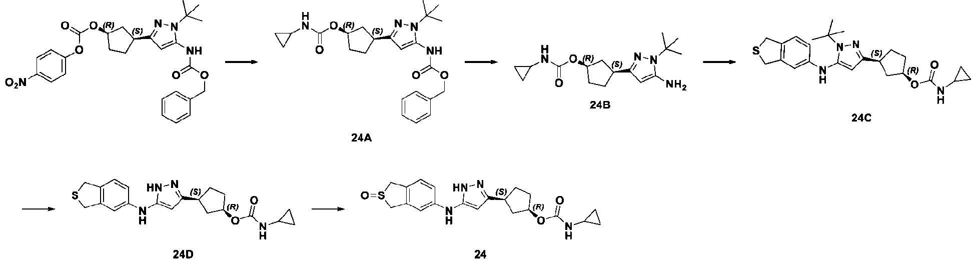

Compound **24** (19 mg) of Example 23 was obtained with reference to the preparation of compound **2** in Example 2, with isopropylamine being replaced by cyclopropylamine. MS (ESI): m/z 401.1642 [M+H]⁺

¹H NMR (500 MHz, DMSO-d6) δ 11.69 (s, 1H), 8.36 (s, 1H), 7.41 (s, 1H), 7.23 (s, 1H), 7.17 (s, 2H), 5.62 (s, 1H), 5.00 (s, 1H), 4.27 (dd, J = 24.7, 15.8 Hz, 2H), 3.92 (dd, J = 24.2, 15.8 Hz, 2H), 3.04 (s, 1H), 2.44 (q, J = 7.2 Hz, 2H), 2.01 (d, J = 9.0 Hz, 1H), 1.90 (dd, J = 10.6, 6.9 Hz, 1H), 1.71 (s, 2H), 1.60 (s, 1H), 0.55 (dt, J = 7.2, 3.7 Hz, 2H), 0.37 (p, J = 4.5 Hz, 2H).

### Example 24: Preparation of compound 25

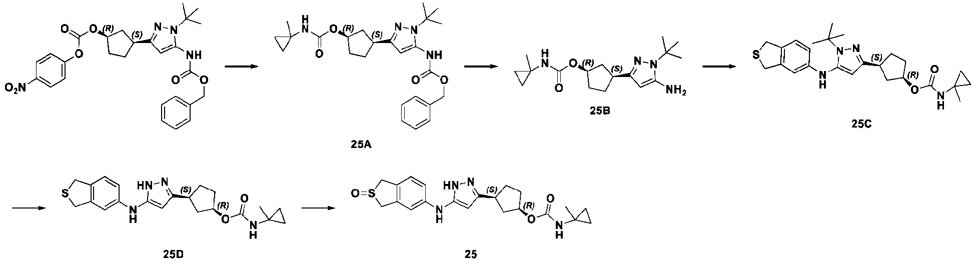

Compound **25** (10 mg) of Example 24 was obtained with reference to the preparation of compound **2** in Example 2, with isopropylamine being replaced by 1-methylcyclopropylamine. MS (ESI): m/z 415.1807 [M+H]⁺

¹H NMR (500 MHz, DMSO-d6) δ 11.68 (s, 1H), 8.36 (s, 1H), 7.42 (s, 1H), 7.34 (s, 1H), 7.17 (d, J = 8.1 Hz, 2H), 5.62 (s, 1H), 4.98 (s, 1H), 4.27 (dd, J = 24.7, 15.8 Hz, 2H), 3.91 (dd, J = 24.3, 15.8 Hz, 2H), 3.03 (s, 1H), 2.46 (d, J = 7.9 Hz, 1H), 2.00 (s, 1H), 1.89 (s, 1H), 1.69 (s, 2H), 1.57 (s, 1H), 1.23 (s, 3H), 0.59 (t, J = 3.2 Hz, 2H), 0.47 (q, J = 4.5 Hz, 2H).

### Example 25: Preparation of compound 26

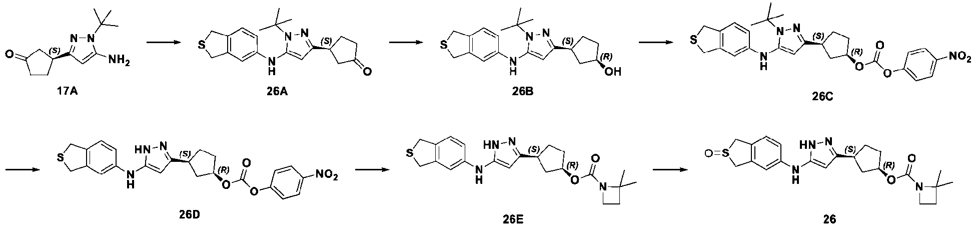

The key intermediate 26**E** (130 mg) of Example **25** was obtained with reference to the preparation of compound **17** in Example 17, with 5-bromo-1,3-dihydrobenzo[c]thiophene-2,2-dioxide being replaced by 5-bromo-1,3-dihydrobenzo[c]thiophene. MS (ESI): m/z 413.57 [M+H]⁺

26E (130 mg) and m-chloroperoxybenzoic acid (67 mg) were added to a solution of dichloromethane (30 mL) at - 20 °C, and the mixture was stirred for reaction. After the reaction was completed, water was added to quench the reaction, and the resultant mixture was extracted with ethyl acetate, dried over anhydrous sodium sulfate, and separated by column chromatography (developing solvents: dichloromethane/methanol) to give compound **26** (80 mg) of Example 25. MS (ESI): m/z 429.1962 [M+H]⁺

¹H NMR (500 MHz, DMSO-d6) δ 11.72 (s, 1H), 8.37 (s, 1H), 7.40 (s, 1H), 7.16 (d, J = 7.9 Hz, 2H), 5.62 (d, J = 4.2 Hz, 1H), 5.09 - 4.93 (m, 1H), 4.27 (m, 2H), 3.91 (m, 2H), 3.73 (t, J = 7.6 Hz, 1H), 3.65 (t, J = 7.6 Hz, 1H), 3.05 (m, 1H), 2.42 (m, 1H), 2.08 - 1.99 (m, 1H), 1.95 (t, J = 7.6 Hz, 2H), 1.86 (m, 1H), 1.74 (m, 2H), 1.65 (m, 1H), 1.43 - 1.28 (m, 6H).

### Example 26: Preparation of compound 27

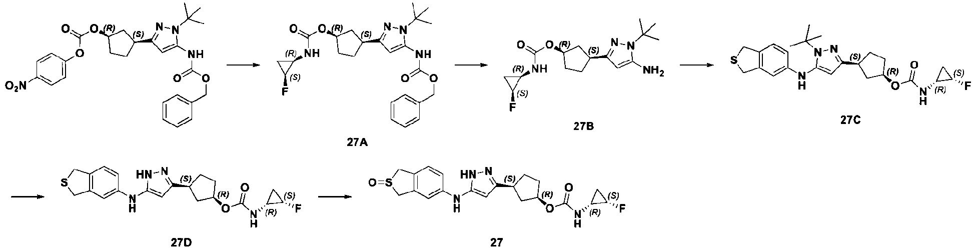

Compound **27** (20 mg) of Example 26 was obtained with reference to the preparation of compound 2 in Example 2, with isopropylamine being replaced by (1R,2S)-2-fluorocyclopropan-1-amine. MS (ESI): m/z 419.1555 [M+H]^{+ 1}H NMR (500 MHz, DMSO-d6) δ 11.70 (s, 1H), 8.38 (s, 1H), 7.40 (d, J = 22.4 Hz, 2H), 7.22 - 7.13 (m, 2H), 5.63 (s, 1H), 5.03 (s, 1H), 4.62 (d, J = 65.0 Hz, 1H), 4.27 (dd, J = 24.8, 15.8 Hz, 2H), 3.91 (dd, J = 24.8, 15.8 Hz, 2H), 3.36 (d, J = 7.3 Hz, 1H), 3.09 - 3.00 (m, 1H), 2.47 (s, 1H), 2.02 (q, J = 7.9 Hz, 1H), 1.91 (ddd, J = 13.6, 11.4, 7.4 Hz, 1H), 1.73 (d, J = 8.9 Hz, 2H), 1.63 (s, 1H), 1.24 (d, J = 7.2 Hz, 2H).

### Example 27: Preparation of compound 28

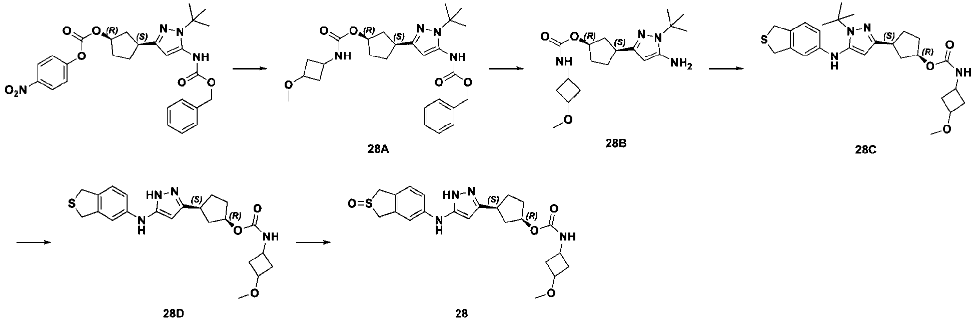

Compound **28** (105 mg) of Example 27 was obtained with reference to the preparation of compound 2 in Example 2, with isopropylamine being replaced by 3-methoxycyclobutylamine. MS (ESI): m/z 445.1906 [M+H]⁺

¹H NMR (500 MHz, DMSO-d6) δ 11.69 (s, 1H), 8.37 (s, 1H), 7.49 - 7.30 (m, 2H), 7.18 (d, J = 5.7 Hz, 2H), 5.62 (s, 1H), 4.98 (d, J = 8.0 Hz, 1H), 4.33 - 4.21 (m, 2H), 3.92 (m, 2H), 3.59 - 3.45 (m, 1H), 3.14 - 2.97 (m, 4H), 2.49 - 2.35 (m, 3H), 2.18 - 2.05 (m, 1H), 2.01 (m, 1H), 1.89 (m, 1H), 1.80 - 1.64 (m, 4H), 1.61 (d, J = 13.4 Hz, 1H).

### Example 28: Preparation of compounds 29 and 30

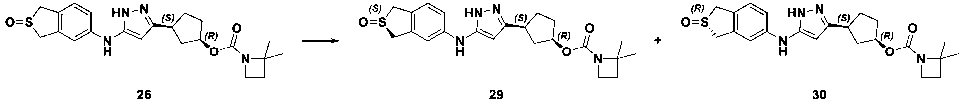

Compound **29** (35 mg) was obtained by subjecting compound 26 (80 mg) to chiral resolution by SFC with reference to the preparation of compounds 22 and 23 in Example 22. MS (ESI): m/z 429.1960 [M+H]⁺; UPCC: Purity: 100%, RT = 1.232 min, 254 nm.

¹H NMR (500 MHz, DMSO-d6) δ 11.72 (s, 1H), 8.37 (s, 1H), 7.40 (t, J = 4.1 Hz, 1H), 7.17 (s, 2H), 5.62 (d, J = 4.3 Hz, 1H), 5.14 - 4.90 (m, 1H), 4.35 - 4.19 (m, 2H), 3.91 (dd, J = 22.0, 15.8 Hz, 2H), 3.69 (dt, J = 37.2, 7.7 Hz, 2H), 3.05 (p, J = 8.5 Hz, 1H), 2.42 (ddd, J = 26.2, 14.4, 7.3 Hz, 1H), 2.08 - 1.98 (m, 1H), 1.95 (t, J = 7.6 Hz, 2H), 1.91 - 1.82 (m, 1H), 1.73 (q, J = 10.0 Hz, 2H), 1.69 - 1.59 (m, 1H), 1.40 - 1.32 (m, 6H).

Compound **30** (35 mg) was obtained. MS (ESI): m/z 429.1964 [M+H]⁺; **UPCC:** Purity: 100%, RT = 2.262 min, 254 nm.

¹H NMR (500 MHz, DMSO-d6) δ 11.72 (s, 1H), 8.37 (s, 1H), 7.49 - 7.34 (m, 1H), 7.16 (d, J = 7.9 Hz, 2H), 5.62 (d, J = 4.1 Hz, 1H), 5.07 - 4.94 (m, 1H), 4.27 (dd, J = 24.6, 15.7 Hz, 2H), 3.91 (dd, J = 22.0, 15.8 Hz, 2H), 3.69 (dt, J = 36.9, 7.7 Hz, 2H), 3.05 (p, J = 8.5 Hz, 1H), 2.42 (ddd, J = 26.4, 14.5, 7.4 Hz, 1H), 2.07 - 1.98 (m, 1H), 1.95 (t, J = 7.6 Hz, 2H), 1.91 - 1.82 (m, 1H), 1.80 - 1.69 (m, 2H), 1.68 - 1.59 (m, 1H), 1.37 - 1.29 (m, 6H). The UPCC detection method was the same as that in Example 22.

### Example 29: Preparation of compounds 31 and 32

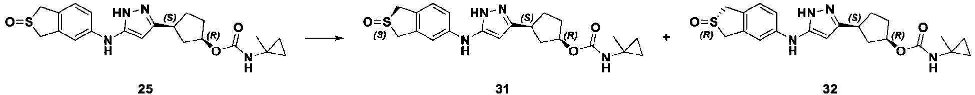

Compound **31** (20 mg) was obtained by subjecting compound **25** (65 mg) to chiral resolution by SFC with reference to the preparation of compounds 22 and 23 in Example 22. MS (ESI): m/z 415.1803 [M+H]⁺; **UPCC:** Purity: 100%, RT = 1.501 min, 254 nm.

¹H NMR (500 MHz, DMSO-d6) δ 11.68 (s, 1H), 8.36 (s, 1H), 7.38 (d, J = 36.5 Hz, 2H), 7.18 (s, 2H), 5.62 (s, 1H), 4.98 (s, 1H), 4.34 - 4.19 (m, 2H), 3.92 (dd, J = 24.6, 15.8 Hz, 2H), 3.03 (s, 1H), 2.00 (s, 1H), 1.89 (s, 1H), 1.70 (s, 2H), 1.57 (s, 1H), 1.23 (s, 3H), 0.59 (d, J = 2.7 Hz, 2H), 0.47 (q, J = 3.5 Hz, 2H).

Compound 32 (19 mg) was obtained. MS (ESI): m/z 415.1804 [M+H]⁺; **UPCC:** Purity: 99.15%, RT = 2.107 min, 254 nm.

¹H NMR (500 MHz, DMSO-d6) δ 11.68 (s, 1H), 8.36 (s, 1H), 7.38 (d, J = 39.0 Hz, 2H), 7.17 (d, J = 8.1 Hz, 2H), 5.62 (s, 1H), 4.98 (s, 1H), 4.35 - 4.21 (m, 2H), 3.91 (dd, J = 23.6, 15.8 Hz, 2H), 3.03 (s, 1H), 2.47 - 2.41 (m, 1H), 2.05 - 1.97 (m, 1H), 1.90 (d, J = 9.2 Hz, 1H), 1.70 (s, 2H), 1.57 (s, 1H), 1.23 (s, 3H), 0.59 (q, J = 4.5 Hz, 2H), 0.49 - 0.43 (m, 2H).

The UPCC detection method was the same as that in Example 22.

### Example 30: Preparation of compounds 33 and 34

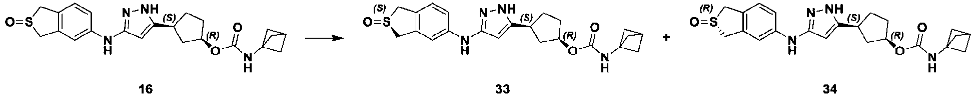

Compound 33 (8.7 mg; RT = 5.467 min (HPLC), MS (ESI): m/z 427.1803 [M+H]+) and compound 34 (8.4 mg; RT = 10.181 min (HPLC), MS (ESI): m/z 427.1796 [M+H]+) were obtained by subjecting compound 16 (20 mg) to chiral resolution by SFC with reference to the preparation of compounds 22 and 23 in Example 22.

### HPLC detection method:

Column: CHIRALART Cellulose-SB (4.6 × 250 mm, 5 µm)
Mobile phase: isocratic elution with n-hexane/ethanol (30%/70%)
Flow rate: 1.0 mL/min
Column temperature: 25 °C
Detection wavelength: 254 nm.

### Example 31: Preparation of compounds 35 and 36

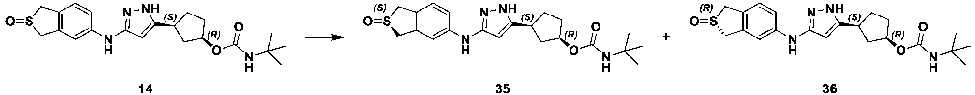

Compound 35 (10.3 mg; RT = 5.769 min (HPLC), MS (ESI): m/z 417.1953 [M+H]+) and compound 36 (11.8 mg; RT = 12.739 min (HPLC), MS (ESI): m/z 417.1956 [M+H]+) were obtained by subjecting compound 14 (25 mg) to chiral resolution by SFC with reference to the preparation of compounds 22 and 23 in Example 22.

The HPLC detection method was the same as that in Example 30.

### Example 32: Preparation of compounds 37 and 38

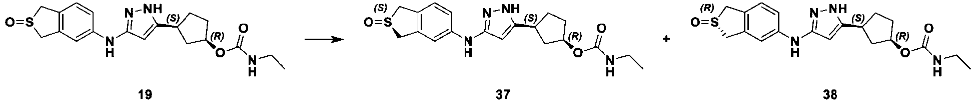

Compound 37 (28.2 mg; RT = 6.348 min (HPLC), MS (ESI): m/z 389.1648 [M+H]+) and compound 38 (27.5 mg, RT = 13.435 min (HPLC), MS (ESI): m/z 389.1650 [M+H]+) were obtained by subjecting compound 19 (85 mg) to chiral resolution by SFC with reference to the preparation of compounds 22 and 23 in Example 22.

The HPLC detection method was the same as that in Example 30.

### Example 33: Preparation of compounds 39 and 40

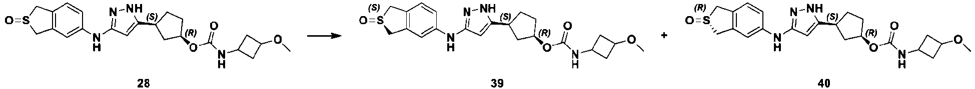

Compound **39** (41.2 mg; RT = 7.083 min (HPLC), MS (ESI): m/z 445.1911 [M+H]⁺) and compound **40** (42.9 mg, RT = 13.003 min (HPLC), MS (ESI): m/z 445.1905 [M+H]⁺) were obtained by subjecting compound 28 (105 mg) to chiral resolution by SFC with reference to the preparation of compounds **22** and **23** in Example **22.**

The HPLC detection method was the same as that in Example 30.

### Example 34: Preparation of compounds 41 and 42

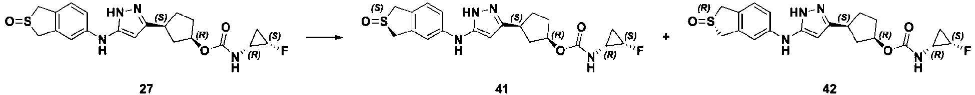

Compound **41** (7.3 mg; RT = 6.288 min (HPLC), MS (ESI): m/z 419.1553 [M+H]⁺) and compound **42** (7.4 mg, RT = 13.602 min (HPLC), MS (ESI): m/z 419.1553 [M+H]⁺) were obtained by subjecting compound 27 (25 mg) to chiral resolution by SFC with reference to the preparation of compounds **22** and **23** in Example **22.**

The HPLC detection method was the same as that in Example 30.

### Experimental Example 1: In vitro inhibitory activity against kinase

### 1.1 Determination of inhibitory activity against CDK2/CycA2 kinase

CDK2/CycA2 kinase solution (concentration: 0.078 ng/µL) was added to assay wells at 6 µL/well, and different compounds dissolved in DMSO were added to the assay wells separately using a nanoliter pipettor, such that the final concentrations of the compounds were 1000 nM to 0.244 nM. The wells were set in duplicate, and a control well was set. After a 30-min incubation of the above system, ATP (concentration: 50 µM or 5000 µM) was mixed with ULight-Myelin Basic Protein Peptide substrate (manufacturer: PerkinElmer, concentration: 0.25 µM) in a ratio of 1:1, and the mixture was added to the assay wells at 4 µL/well. The resultant mixture was left to react at room temperature for 2 h, and then 5 µL of EDTA was added to terminate the reaction, followed by the addition of 5 µL of a detection antibody (manufacturer: PerkinElmer, concentration: 8 nM). The mixture was incubated at room temperature for 1 h. The assay was performed using a PerkinElmer Envision multifunctional microplate reader (excitation: 320 nm, emission: 615 nm/665 nm), and IC₅₀ was calculated by four-parameter fitting.

### 1.2 Determination of inhibitory activity against CDK2 CycE1 kinase

CDK2 CycEl kinase solution (concentration: 0.015 ng/µL) was added to assay wells at 6 µL/well, and different compounds dissolved in DMSO were added to the assay wells separately using a nanoliter pipettor, such that the final concentrations of the compounds were 300 nM to 0.07 nM. The wells were set in duplicate, and a control well was set. After a 30-min incubation of the above system, ATP (concentration: 50 µM or 5000 µM) was mixed with ULight-Myelin Basic Protein Peptide substrate (manufacturer: PerkinElmer, concentration: 0.25 µM) in a ratio of 1:1, and the mixture was added to the assay wells at 4 µL/well. The resultant mixture was left to react at room temperature for 2 h, and then 5 µL of EDTA was added to terminate the reaction, followed by the addition of 5 µL of a detection antibody (manufacturer: PerkinElmer, concentration: 8 nM). The mixture was incubated at room temperature for 1 h. The assay was performed using a PerkinElmer Envision multifunctional microplate reader (excitation: 320 nm, emission: 615 nm/665 nm), and IC₅₀ was calculated by four-parameter fitting. The experimental results are shown in Table 1, wherein A represents: 0 nM < IC₅₀ ≤ 10 nM; B represents: 10 nM < IC₅₀ ≤ 50 nM; C represents: 50 nM < IC₅₀ ≤ 100 nM; D represents: 100 nM < IC₅₀ ≤ 1000 nM; E represents: 1000 nM < IC₅₀.

**Table 1: Inhibitory activity of compound against CDK2 CycA2 and CDK2 CycEl kinases**

| Example | CDK2/CycA2 | CDK2/CycE1 |
|---|---|---|
| | 10 µM ATP (kinase) | 10 µM ATP (kinase) |
| | IC₅₀ (nM) | IC₅₀ (nM) |
| Compound 1 | A | A |
| Compound 2 | B | A |
| Compound 3 | A | A |
| Compound 4 | A | A |
| Compound 5 | A | A |
| Compound 6 | B | A |
| Compound 7 | A | A |
| Compound 8 | - | B |
| Compound 10 | A | A |
| Compound 11 | A | A |
| Compound 12 | B | A |
| Compound 13 | A | A |
| Compound 14 | A | A |
| Compound 15 | - | A |
| Compound 16 | A | A |
| Compound 17 | A | A |
| Compound 18 | A | A |
| Compound 19 | - | B |
| Compound 20 | - | B |
| Compound 21 | A | A |
| Compound 22 | B | A |
| Compound 23 | - | A |
| Compound 24 | - | B |
| Compound 25 | B | A |
| Compound 26 | A | A |
| Compound 29 | A | A |
| Compound 30 | B | A |
| Compound 31 | A | A |
| Compound 32 | - | A |
| Compound 33 | - | A |
| Compound 34 | - | A |
| Compound 35 | - | A |
| Compound 36 | - | A |
| Compound 37 | - | B |
| Compound 41 | - | A |

| | | |
|---|---|---|
| "-" denotes not available. | | |

## Claims

1. A compound of formula (I), an isomer thereof, or a pharmaceutically acceptable salt thereof, 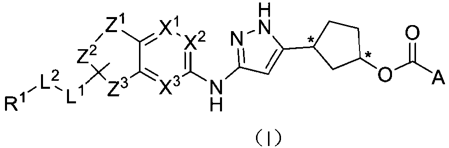 wherein
X¹, X², and X³ are each independently selected from the group consisting of N and C(R^{a});
Z¹, Z², and Z³ are each independently selected from the group consisting of O, S, N, N(R^{b}), S(O), S(O)₂, C(O), C(R^{a}), and C(R^{a}R^{a});
L¹ and L² are each independently selected from the group consisting of a single bond, -N(R^{b})-, -N=, -O-, -S-, - (CH₂)ₘ-, -(CD₂)ₘ-, -(CHD)ₘ-, -C(=O)-, -S(=O) -, -S(=O)₂ -, -S(=O)(=N(R^{b}))-, -S(=O)(R^{c})-, -P(=O)(R^{c})-, and - P(=O)(NR^{b}R^{b})-;
R¹ is selected from the group consisting of H, deuterium, C₁₋₃ alkyl, C₁₋₃ alkoxy, -NH₂, -NH(C₁₋₃ alkyl), -N(C₁₋₃ alkyl)₂, C₃₋₆ cycloalkyl, C₃₋₆ cycloalkenyl, 3- to 6-membered heterocyclyl, phenyl, and 5- to 6-membered heteroaryl, the C₁₋₃ alkyl or C₁₋₃ alkoxy being optionally independently substituted with 1, 2, or 3 R^{d}, and the C₃₋₆ cycloalkyl, C₃₋₆ cycloalkenyl, 3- to 6-membered heterocyclyl, phenyl, or 5- to 6-membered heteroaryl being optionally independently substituted with 1 or more R^{e};
A is selected from the group consisting of 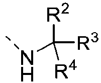 and 3- to 6-membered heterocyclyl containing 1 or 2 N atoms, the 3- to 6-membered heterocyclyl being optionally independently substituted with 1 or more R^{z};
R², R³, and R⁴ are each independently selected from the group consisting of H, deuterium, halogen, OH, CN, NH₂, C₁₋₃ alkyl, C₁₋₃ alkoxy, C₃₋₆ cycloalkyl, C₃₋₆ cycloalkenyl, and 3- to 6-membered heterocyclyl, the C₁₋₃ alkyl, C₁₋₃ alkoxy, C₃₋₆ cycloalkyl, C₃₋₆ cycloalkenyl, or 3- to 6-membered heterocyclyl being optionally independently substituted with 1 or more R^{z};
or, R² and R³, together with the carbon atom linked thereto, form C₃₋₆ cycloalkyl, C₃₋₆ cycloalkenyl, or 3- to 6-membered heterocyclyl, and R⁴ is selected from the group consisting of H, deuterium, halogen, OH, CN, NH₂, C₁₋₃ alkyl, and C₁₋₃ alkoxy, the C₁₋₃ alkyl, C₁₋₃ alkoxy, C₃₋₆ cycloalkyl, C₃₋₆ cycloalkenyl, or 3- to 6-membered heterocyclyl being optionally independently substituted with 1 or more R^{z};
or, R², R³, and R⁴, together with the carbon atom linked thereto, form C₅₋₈ bicycloalkyl or 5- to 8-membered heterobicycloalkyl, the C₃₋₈ bicycloalkyl or 5- to 8-membered heterobicycloalkyl being optionally independently substituted with 1 or more R^{z};
R^{a} is each independently selected from the group consisting of hydrogen, deuterium, halogen, OH, CN, NH₂, COOH, NO₂, C₁₋₃ alkyl, C₁₋₃ alkoxy, C₁₋₃ alkoxy C₁₋₃ alkylene, C₁₋₃ alkylamino, and (C₁₋₃ alkyl)₂ amino, the C₁₋₃ alkyl or C₁₋₃ alkoxy being optionally independently substituted with 1 or more R^{x};
or, the two R^{a} in C(R^{a}R^{a}), together with the carbon atom linked thereto, form C₃₋₆ cycloalkyl, C₃₋₆ cycloalkenyl, or 3- to 6-membered heterocyclyl, the C₃₋₆ cycloalkyl, C₃₋₆ cycloalkenyl, or 3- to 6-membered heterocyclyl being optionally independently substituted with 1 or more R^{y};
R^{b} is each independently selected from the group consisting of H, C₁₋₃ alkyl, C₁₋₃ alkoxy, -C(=O)H, -S(=O)₂C₁₋₃ alkyl, C₃₋₆ cycloalkyl, C₃₋₆ cycloalkenyl, and 3- to 6-membered heterocyclyl, the C₁₋₃ alkyl or C₁₋₃ alkoxy being optionally independently substituted with 1, 2, or 3 R^{x}, and the C₃₋₆ cycloalkyl, C₃₋₆ cycloalkenyl, or 3- to 6-membered heterocyclyl being optionally independently substituted with 1 or more R^{y};
R^{c} is selected from the group consisting of halogen, OH, CN, and C₁₋₃ alkyl, the C₁₋₃ alkyl being optionally independently substituted with 1 or more R^{x};
R^{d} is each independently selected from the group consisting of deuterium, halogen, OH, CN, and NH₂;
R^{e} is each independently selected from the group consisting of deuterium, halogen, OH, CN, NH₂, =O, C₁₋₃ alkyl, and C₁₋₃ alkoxy, the C₁₋₃ alkyl or C₁₋₃ alkoxy being optionally independently substituted with 1 or more R^{x};
R^{x} is each independently selected from the group consisting of deuterium, halogen, OH, CN, and NH₂;
R^{y} is each independently selected from the group consisting of deuterium, halogen, OH, CN, NH₂, =O, C₁₋₃ alkyl, and C₁₋₃ alkoxy, the C₁₋₃ alkyl or C₁₋₃ alkoxy being optionally independently substituted with 1 or more substituents selected from the group consisting of deuterium, halogen, OH, CN, and NH₂;
R^{z} is each independently selected from the group consisting of deuterium, halogen, OH, CN, NH₂, C₁₋₃ alkyl, and C₁₋₃ alkoxy, the C₁₋₃ alkyl or C₁₋₃ alkoxy being optionally independently substituted with 1 or more substituents selected from the group consisting of deuterium, halogen, OH, CN, and NH₂;
m is selected from the group consisting of 1, 2, and 3;
the carbon atom with "*" is a chiral carbon atom present in a form of a single (R) or (S) enantiomer or in a form enriched with one enantiomer;
or, L¹, L², R¹, R², R³, R⁴, R^{a}, R^{b}, R^{c}, R^{d}, R^{e}, R^{x}, R^{y}, or R^{z} is each independently optionally substituted with one or more substituents.

2. The compound of formula (I), the isomer thereof, or the pharmaceutically acceptable salt thereof according to claim 1, wherein X¹, X², and X³ are selected from C(R^{a}); or, one of X¹, X², and X³ is selected from N; or X¹ is selected from N, and X² and X³ are selected from C(R^{a}); or X² is selected from N, and X¹ and X³ are selected from C(R^{a}); or X³ is selected from N, and X¹ and X² are selected from C(R^{a}).

3. The compound of formula (I), the isomer thereof, or the pharmaceutically acceptable salt thereof according to claim 1, wherein R^{a} is each independently selected from the group consisting of hydrogen, F, Cl, Br, I, CN, C₁₋₃ alkyl, C₁₋₃ alkoxy, and C₁₋₃ alkoxy C₁₋₃ alkylene, the C₁₋₃ alkyl or C₁₋₃ alkoxy being optionally independently substituted with 1, 2, or 3 R^{x}; or R^{a} is each independently selected from the group consisting of hydrogen, F, C₁₋₃ alkyl, C₁₋₃ alkoxy, and C₁₋₃ alkoxy C₁₋₃ alkylene, the C₁₋₃ alkyl or C₁₋₃ alkoxy being optionally independently substituted with 1, 2, or 3 R^{x}; or R^{a} is each independently selected from the group consisting of hydrogen, F, methyl, and methoxymethyl, the methyl or methoxymethyl being optionally independently substituted with 1, 2, or 3 R^{x}; or R^{a} is each independently selected from the group consisting of hydrogen, F, methyl, and methoxymethyl.

4. The compound of formula (I), the isomer thereof, or the pharmaceutically acceptable salt thereof according to claim 1, wherein Z¹, Z², and Z³ are each independently selected from the group consisting of S(O), S(O)₂, and C(R^{a}R^{a}); or Z² is selected from the group consisting of S(O) and S(O)₂, and Z¹ and Z³ are each independently selected from C(R^{a}R^{a}).

5. The compound of formula (I), the isomer thereof, or the pharmaceutically acceptable salt thereof according to claim 1, wherein L¹ is selected from the group consisting of a single bond, -N(R_{b})-, -O-, -S(=O)₂-, -S-, - (CH₂)ₘ-, and -C(=O)-; or L¹ is selected from the group consisting of a single bond, -NH-, -N(CH₃)-, - S(=O)₂ -, -CH₂-, -CH₂-CH₂-, -CH₂-CH₂-CH₂-, and -C(=O)-; or L¹ is selected from the group consisting of a single bond and -NH-;
L² is selected from the group consisting of a single bond, -NH-, -N(C₁₋₃ alkyl)-, -CH₂-, -CH₂-CH₂-, -CH₂-CH₂-CH₂-, -C(=O)-, -S(=O)-, -S(=O)₂-, and -S(=O)(C₁₋₃ alkyl)-; or L² is selected from the group consisting of a single bond, -NH-, -N(CH₃)-, -CH₂-, -CH₂-CH₂-, -CH₂-CH₂-CH₂-, - C(=O)-, -S(=O)-, -S(=O)₂-, and - S(=O)(CH₃)-; or L² is selected from the group consisting of a single bond and -S(=O)₂-;
-L²-L¹- is selected from the group consisting of a single bond, -NH-, -N(CH₃)-, -C(=O)-, -CH₂-, -CH₂-CH₂-, -S(=O)₂-, -CH₂-NH-, -NH-CH₂-, -CH₂-N(CH₃)-, -N(CH₃)-CH₂-, -C(=O)-CH₂-, -CH₂-C(=O)-, -C(=O)-NH-, -NH-C(=O)-, -NH-S(=O)₂-, -S(=O)₂-NH-, -N(CH₃)-S(=O)₂-, -S(=O)₂-N(CH₃)-, -N(CH₃)-C(=O)-, -C(=O)-N(CH₃)-, -N(CH₃)-CH₂-CH₂-, -CH₂-CH₂-N(CH₃)-, -NH-CH₂-CH₂-, and -CH₂-CH₂-NH-; or -L²-L¹- is selected from the group consisting of a single bond, -NH-, -CH₂-, and -S(=O)₂-NH-; or -L²-L¹- is selected from a single bond.

6. The compound of formula (I), the isomer thereof, or the pharmaceutically acceptable salt thereof according to claim 1, wherein R¹ is selected from the group consisting of H, deuterium, C₁₋₃ alkyl, C₁₋₃ alkoxy, -NH₂, -NH(C₁₋₃ alkyl), -N(C₁₋₃ alkyl)₂, C₃₋₆ cycloalkyl, 3- to 6-membered heterocycloalkyl, phenyl, and 5- to 6-membered heteroaryl, the C₁₋₃ alkyl or C₁₋₃ alkoxy being optionally independently substituted with 1, 2, or 3 R^{d}, and the C₃₋₆ cycloalkyl, 3- to 6-membered heterocycloalkyl, phenyl, or 5- to 6-membered heteroaryl being optionally independently substituted with 1 or more R^{e}; or R¹ is selected from the group consisting of H, methyl, methoxy, -NH₂, -NH(CH₃), -NH(CH₂CH₃), cyclopropyl, oxetanyl, pyrazolidinyl, isothiazolidinyl, isoxazolidinyl, imidazolyl, oxazolyl, and pyrimidinyl, the methyl, ethyl, or methoxy being optionally independently substituted with 1, 2, or 3 R^{d}, and the cyclopropyl, oxetanyl, pyrazolidinyl, isothiazolidinyl, isoxazolidinyl, imidazolyl, oxazolyl, or pyrimidinyl being optionally independently substituted with 1, 2, or 3 R^{e}; or R ¹ is selected from H.

7. The compound of formula (I), the isomer thereof, or the pharmaceutically acceptable salt thereof according to claim 1, wherein R^{d} is each independently selected from the group consisting of F, Cl, Br, I, and OH; or R^{d} is each independently selected from the group consisting of F and OH;
R^{e} is each independently selected from the group consisting of F, Cl, Br, I, =O, and C₁₋₃ alkyl, the C₁₋₃ alkyl being optionally independently substituted with 1, 2, or 3 R^{x}; or R^{e} is each independently selected from the group consisting of F and methyl;
R^{x} is each independently selected from halogen; or R^{x} is each independently selected from F;
R^{y} is each independently selected from halogen; or R^{y} is each independently selected from F.

8. The compound of formula (I), the isomer thereof, or the pharmaceutically acceptable salt thereof according to claim 1, wherein R^{b} is each independently selected from the group consisting of H, C₁₋₃ alkyl, C₁₋₃ alkoxy, -C(=O)H, -S(=O)₂C₁₋₃ alkyl, C₃₋₆ cycloalkyl, and 3- to 6-membered heterocycloalkyl, the C₁₋₃ alkyl or C₁₋₃ alkoxy being optionally independently substituted with 1, 2, or 3 R^{x}, and the C₃₋₆ cycloalkyl or 3- to 6-membered heterocycloalkyl being optionally independently substituted with 1 or more R^{y}; or R^{b} is each independently selected from the group consisting of H and C₁₋₃ alkyl, the C₁₋₃ alkyl being optionally independently substituted with 1, 2, or 3 R^{x}; or R^{b} is each independently selected from the group consisting of H and methyl.

9. The compound of formula (I), the isomer thereof, or the pharmaceutically acceptable salt thereof according to claim 1, wherein R^{c} is selected from C₁₋₃ alkyl, the C₁₋₃ alkyl being optionally independently substituted with 1, 2, or 3 R^{x}; or R^{c} is selected from methyl.

10. The compound of formula (I), the isomer thereof, or the pharmaceutically acceptable salt thereof according to claim 1, wherein R², R³, and R⁴ are each independently selected from the group consisting of H, deuterium, C₁₋₃ alkyl, and C₃₋₆ cycloalkyl, the C₁₋₃ alkyl or C₃₋₆ cycloalkyl being optionally independently substituted with 1, 2, or 3 R^{z}; or R², R³, and R⁴ are each independently selected from the group consisting of H, deuterium, methyl, ethyl, and cyclopropyl, the methyl, ethyl, or cyclopropyl being optionally independently substituted with 1, 2, or 3 R^{z}; or R², R³, and R⁴ are each independently selected from the group consisting of H, deuterium, methyl, -CD₃, difluoromethyl, trifluoromethyl, ethyl, 2-hydroxyethyl, and cyclopropyl; or R², R³, and R⁴ are each independently selected from the group consisting of H, methyl, difluoromethyl, trifluoromethyl, 2-hydroxyethyl, and cyclopropyl;
or R² and R³, together with the carbon atom linked thereto, form C₃₋₄ cycloalkyl or 3- to 4-membered heterocycloalkyl, and R⁴ is selected from the group consisting of H and C₁₋₃ alkyl, the C₁₋₃ alkyl, C₃₋₄ cycloalkyl, or 3- to 4-membered heterocycloalkyl being optionally independently substituted with 1, 2, or 3 R^{z}; or R² and R³, together with the carbon atom linked thereto, form cyclopropyl or cyclobutyl, and R⁴ is selected from the group consisting of H and methyl, the cyclopropyl or cyclobutyl being optionally independently substituted with 1, 2, or 3 R^{z};
or R², R³, and R⁴, together with the carbon atom linked thereto, form C₅₋₈ bicycloalkyl, the C₅₋₈ bicycloalkyl being a bridged cyclyl, and the C₅₋₈ bicycloalkyl being optionally independently substituted with 1 or more R^{z}; or R², R³, and R⁴, together with the carbon atom linked thereto, form

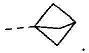

11. The compound of formula (I), the isomer thereof, or the pharmaceutically acceptable salt thereof according to claim 1, wherein A is selected from the group consisting of 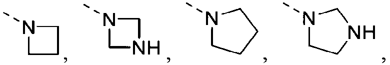 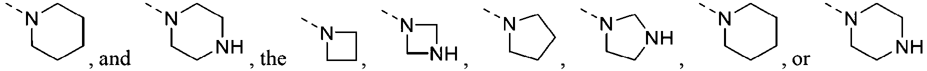 being optionally independently substituted with 1 or more R^{z}; or A is selected from the group consisting of 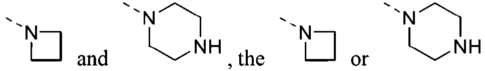 being optionally independently substituted with 1 or more methyl; or A is selected from the group consisting of

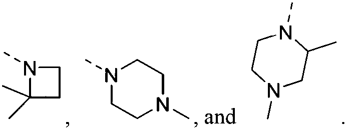

12. The compound of formula (I), the isomer thereof, or the pharmaceutically acceptable salt thereof according to claim 1, wherein R^{z} is each independently selected from the group consisting of deuterium, halogen, OH, C₁₋₃ alkyl, and C₁₋₃ alkoxy, the C₁₋₃ alkyl or C₁₋₃ alkoxy being optionally independently substituted with 1 or more substituents selected from the group consisting of deuterium, halogen, OH, CN, and NH₂; or R^{z} is each independently selected from the group consisting of deuterium, halogen, OH, C₁₋₃ alkyl, and C₁₋₃ alkoxy; or R^{z} is each independently selected from the group consisting of F, OH, methyl, and methoxy.

13. The compound of formula (I), the isomer thereof, or the pharmaceutically acceptable salt thereof according to claim 1, wherein the compound is selected from a compound of formula (I-1), 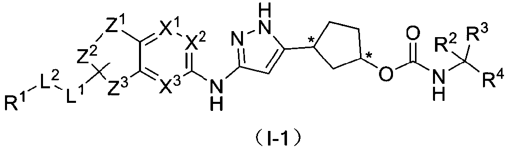
X¹, X², X³, Z¹, Z², Z³, L¹, L², R¹, R², R³, and R⁴ being as defined in claim 1;
or, the compound is selected from the group consisting of compounds of formula (I-2) and formula (I-3), 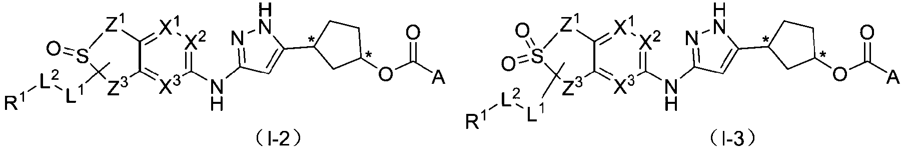
X¹, X², X³, Z¹, Z³, L¹, L², R¹, and A being as defined in claim 1;
or, the compound is selected from the group consisting of compounds of formula (I-4) and formula (I-5), 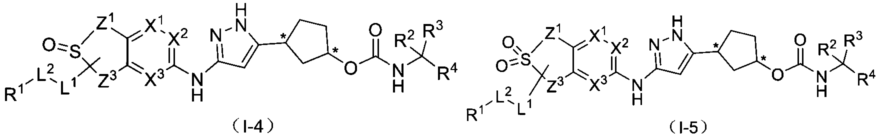
X¹, X², X³, Z¹, Z³, L¹, L², R¹, R², R³, and R⁴ being as defined in claim 1.

14. A compound of the formula below, an isomer thereof, or a pharmaceutically acceptable salt thereof,

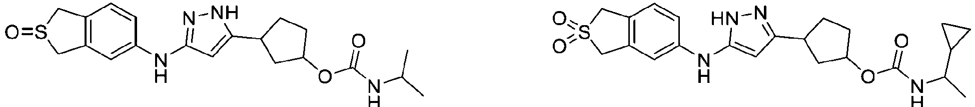

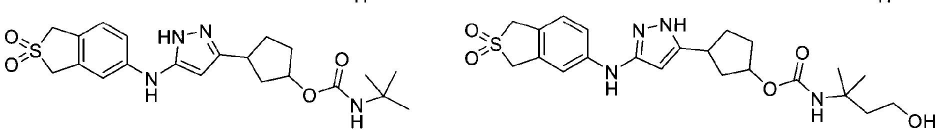

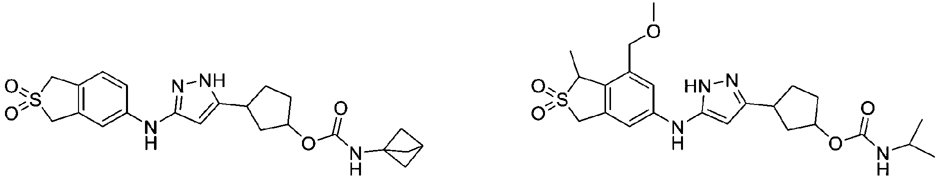

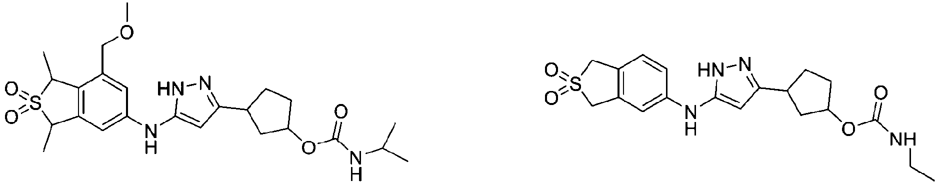

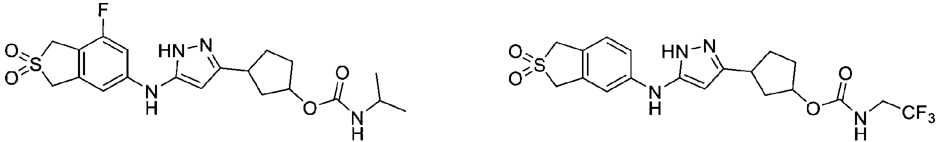

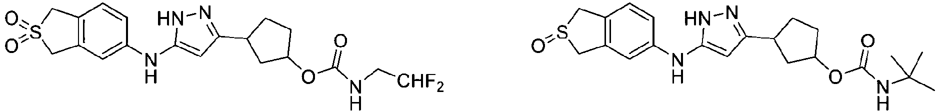

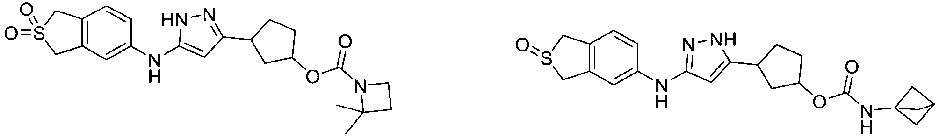

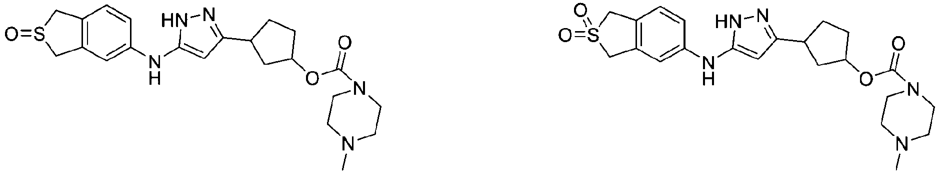

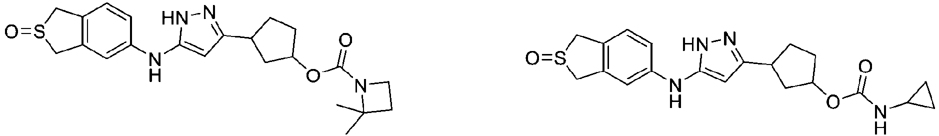

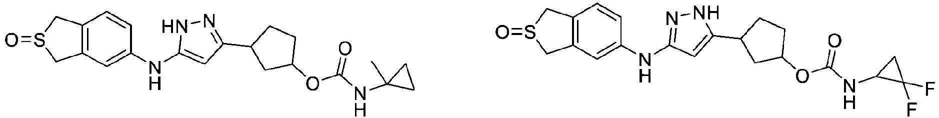

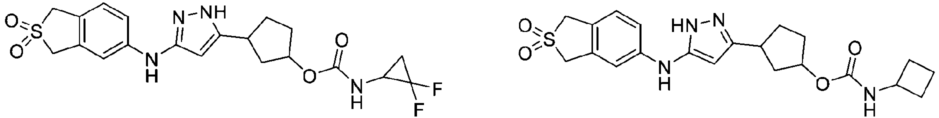

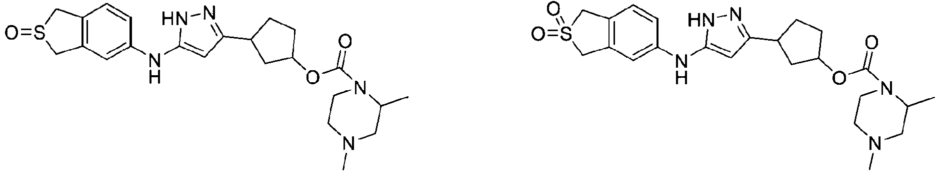

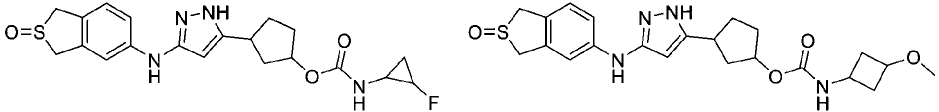

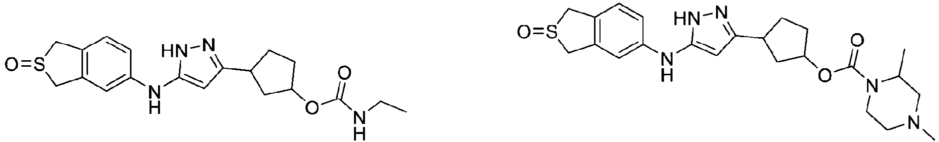

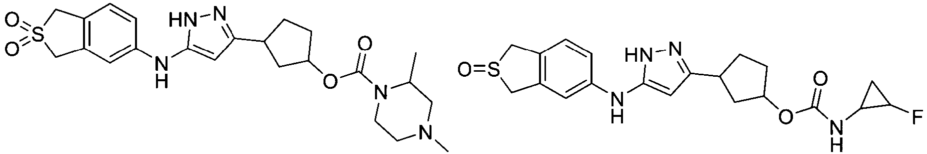

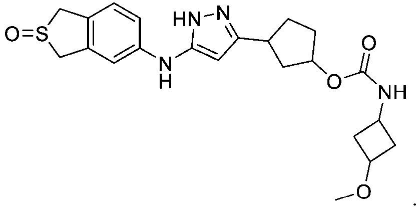

15. A compound of the formula below, an isomer thereof, or a pharmaceutically acceptable salt thereof,

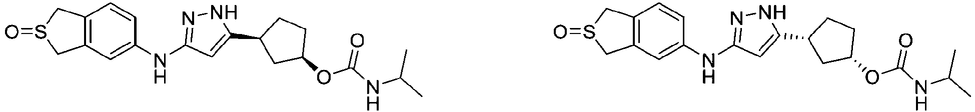

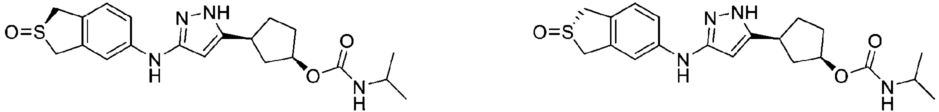

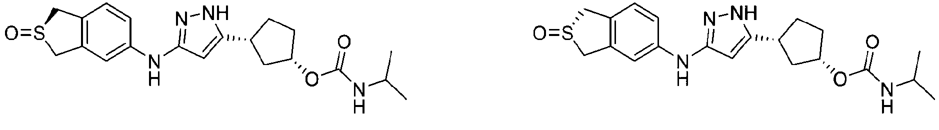

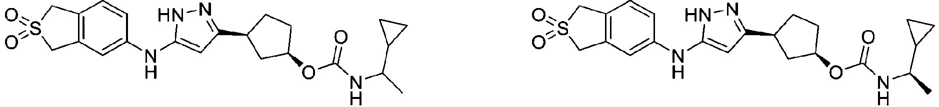

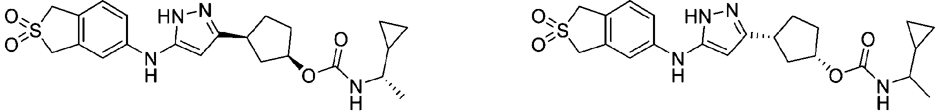

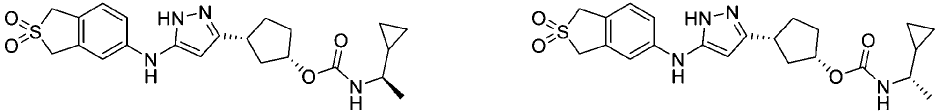

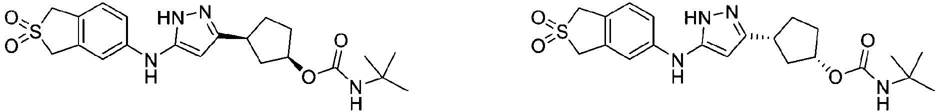

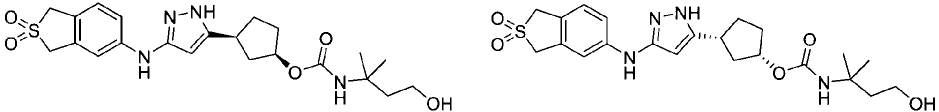

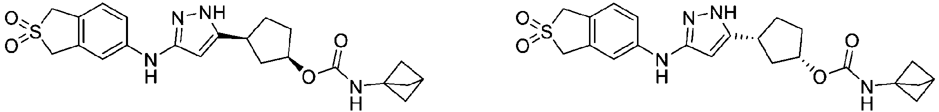

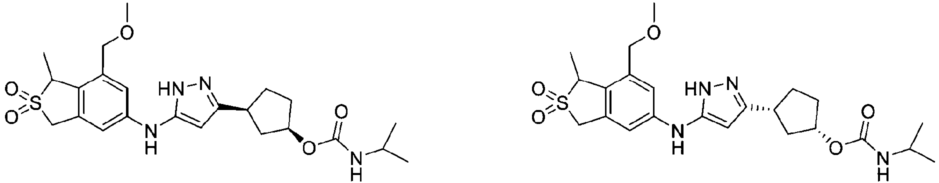

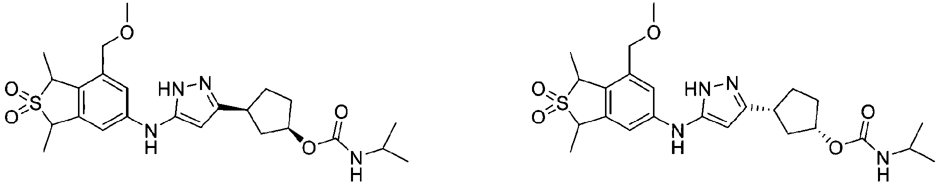

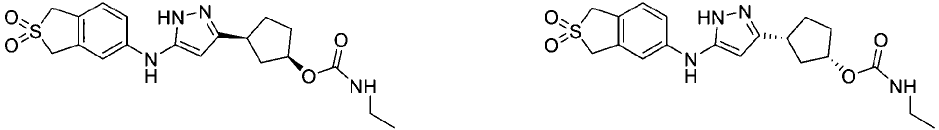

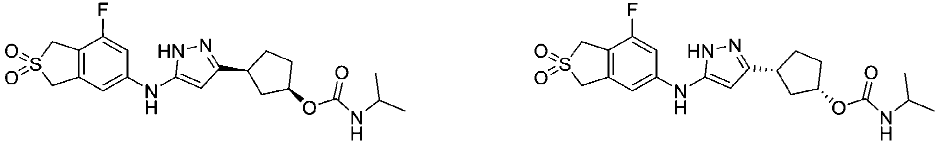

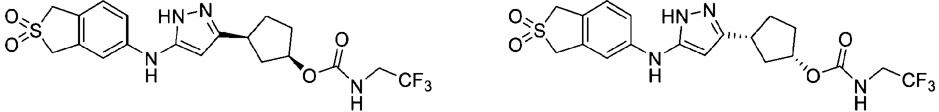

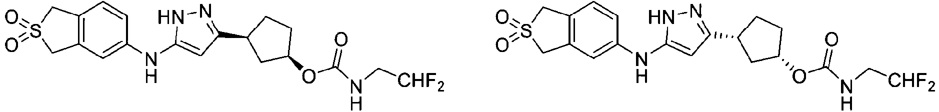

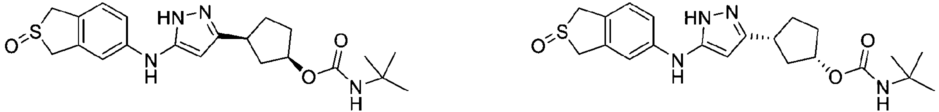

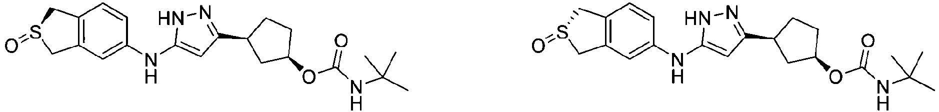

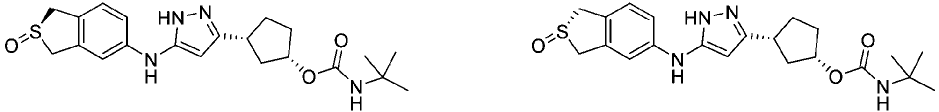

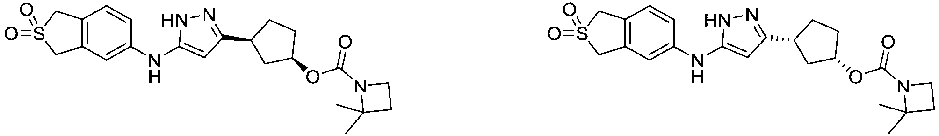

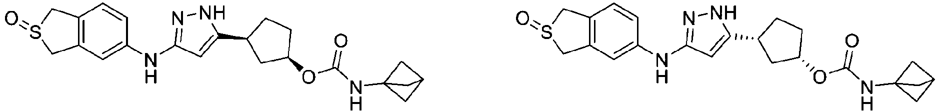

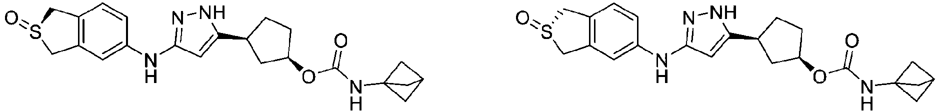

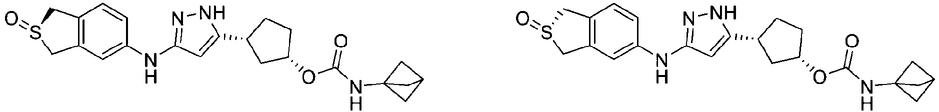

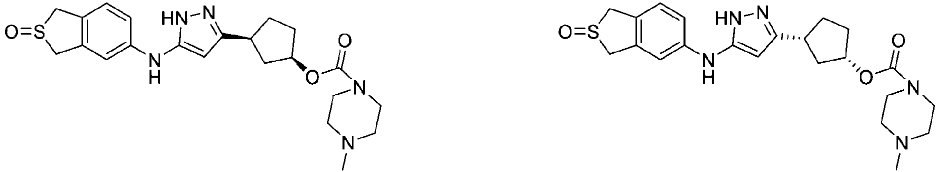

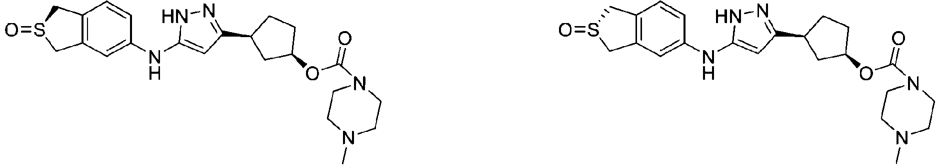

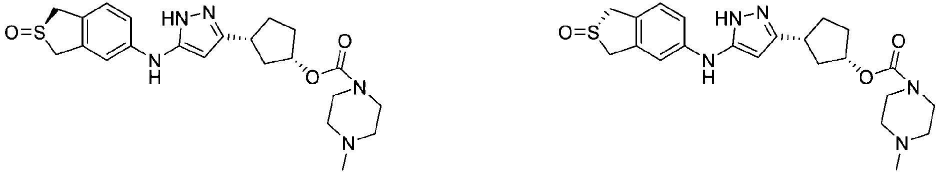

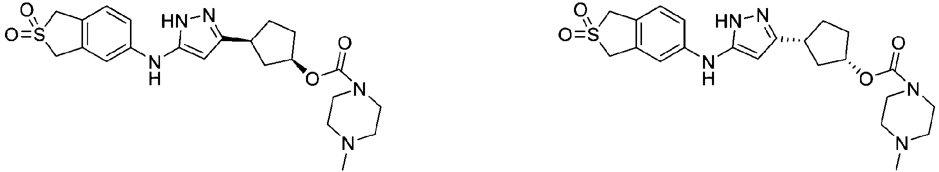

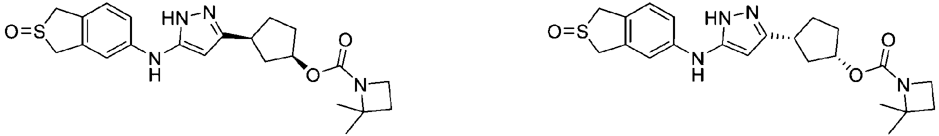

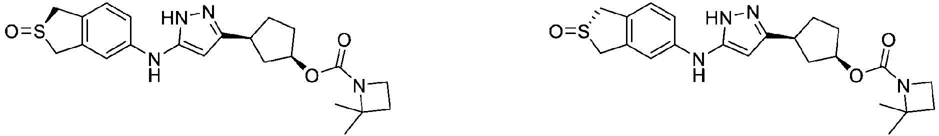

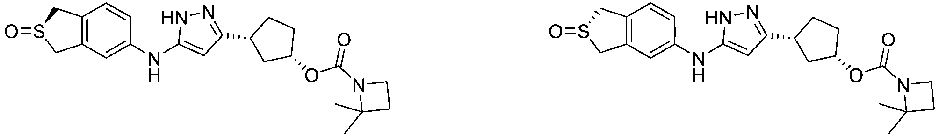

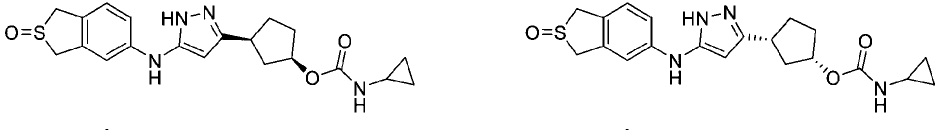

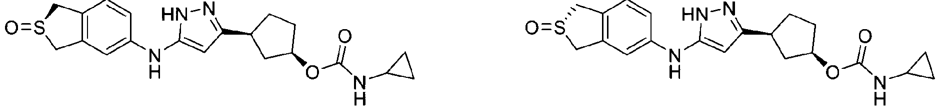

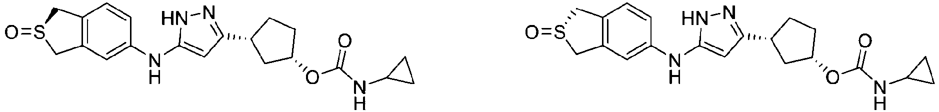

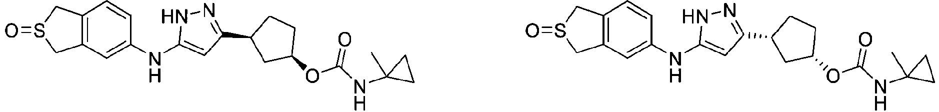

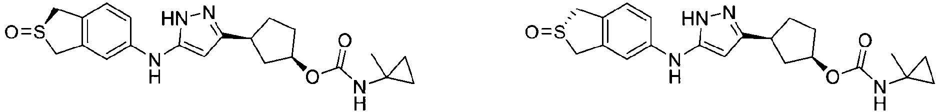

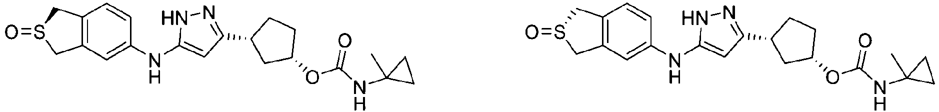

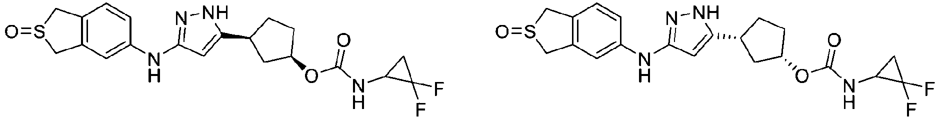

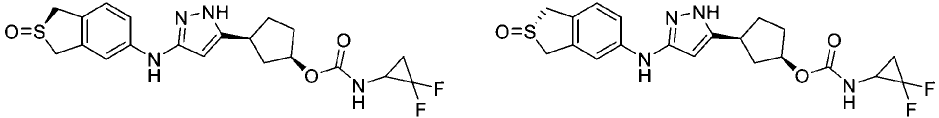

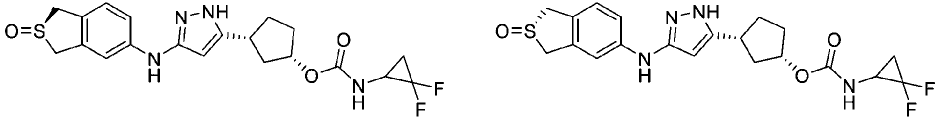

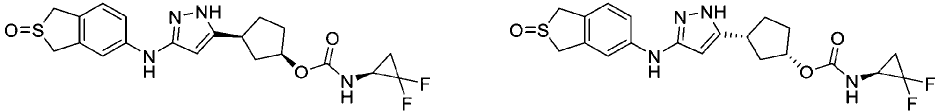

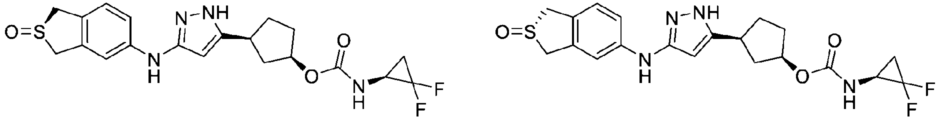

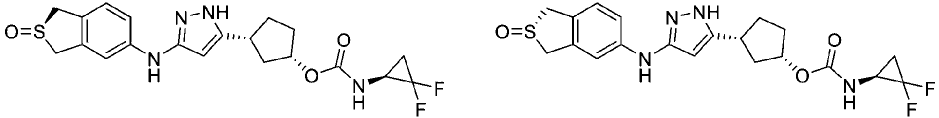

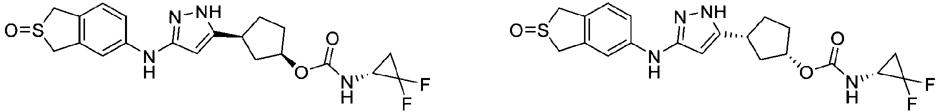

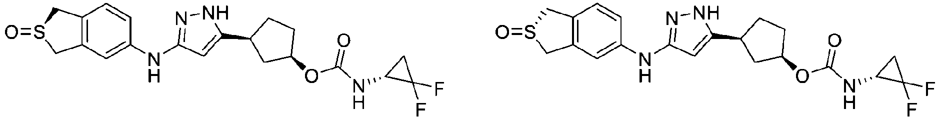

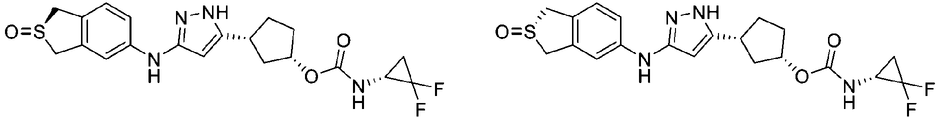

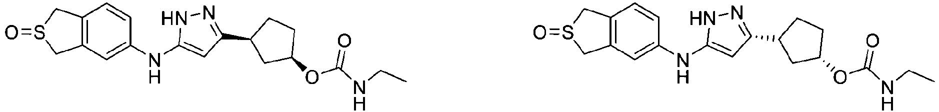

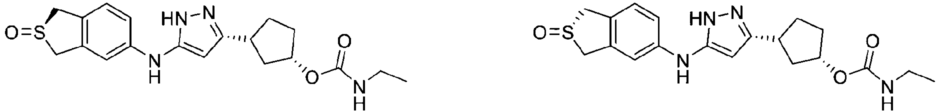

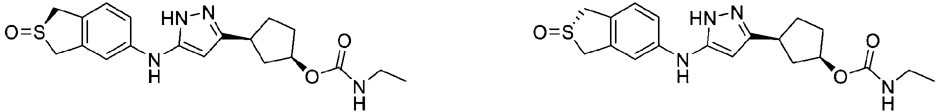

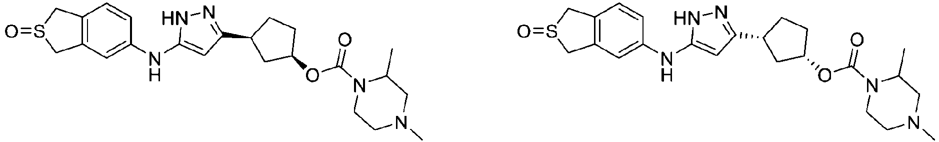

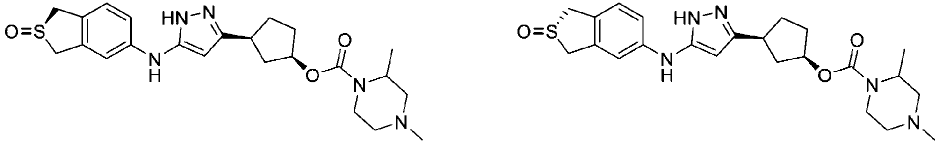

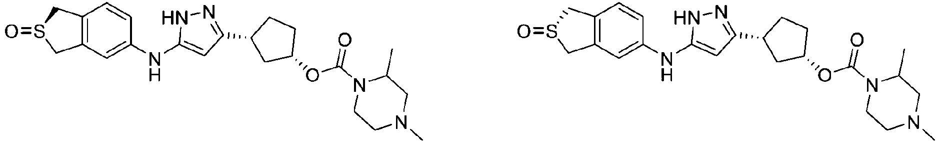

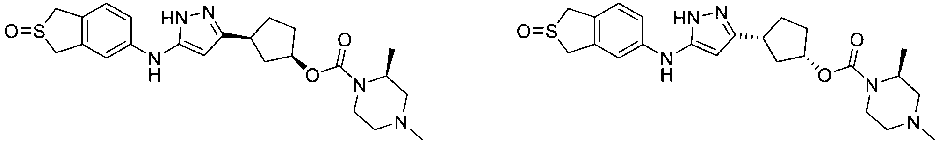

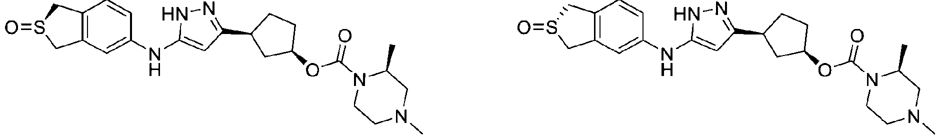

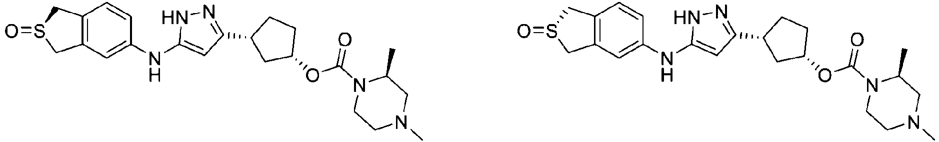

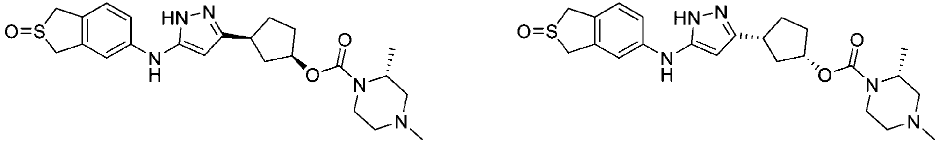

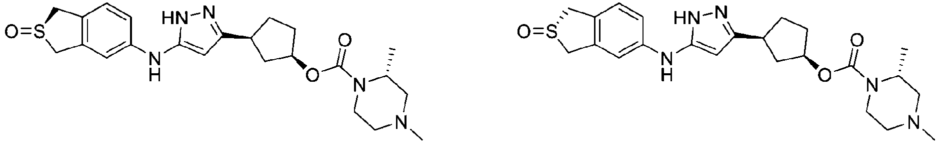

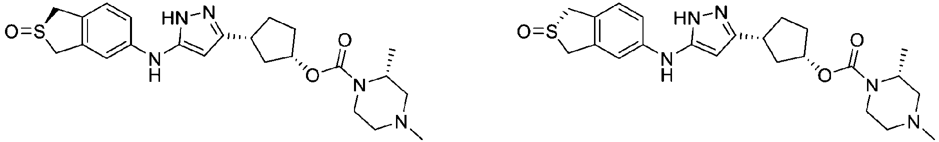

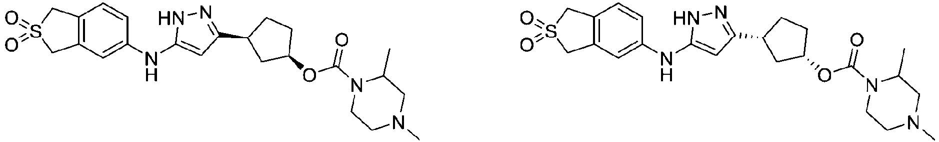

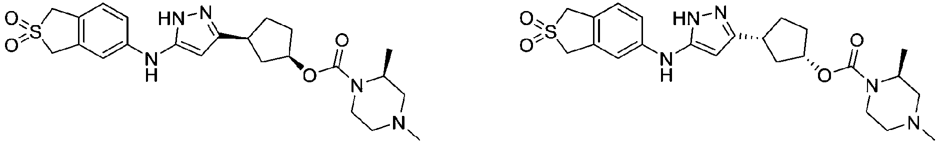

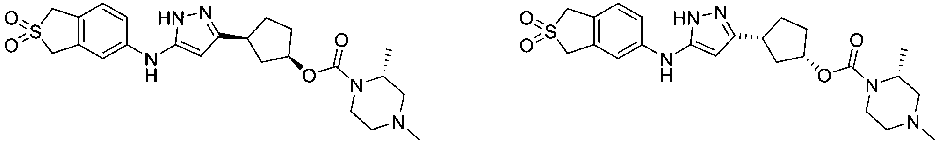

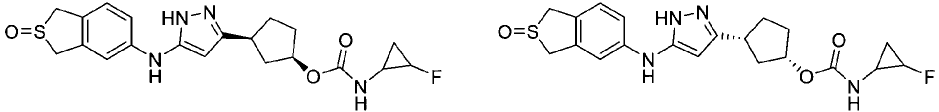

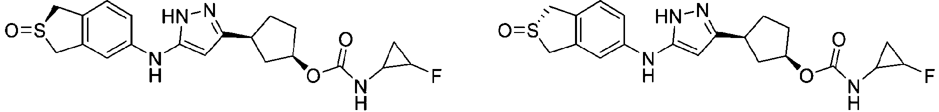

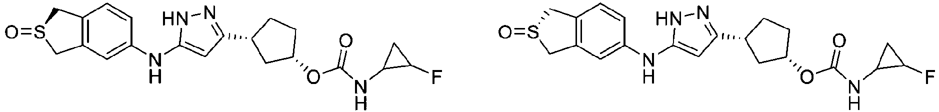

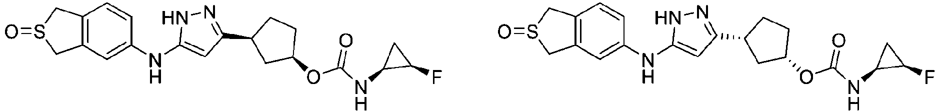

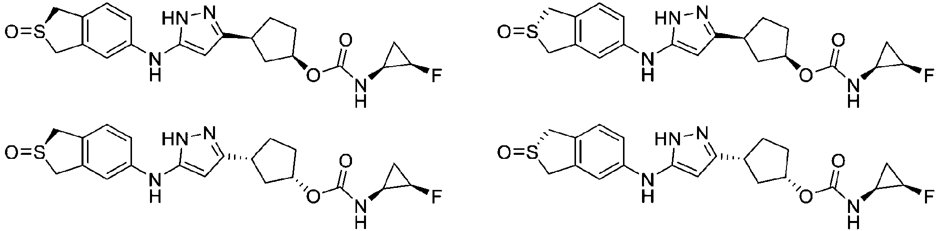

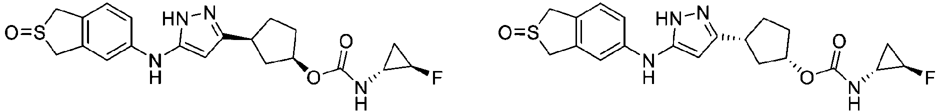

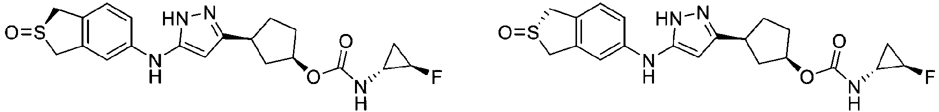

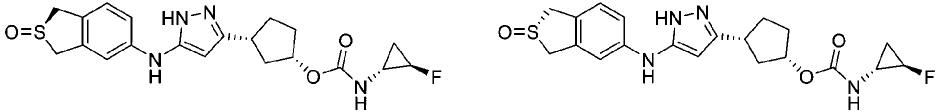

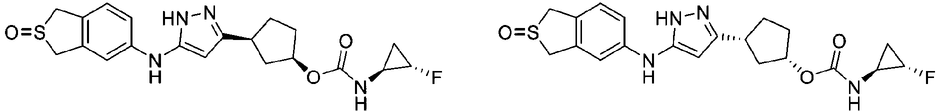

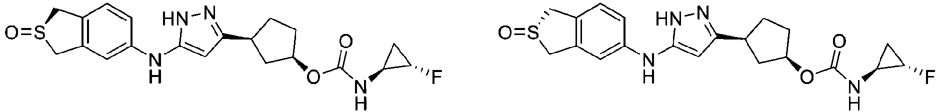

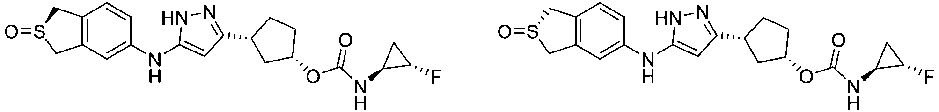

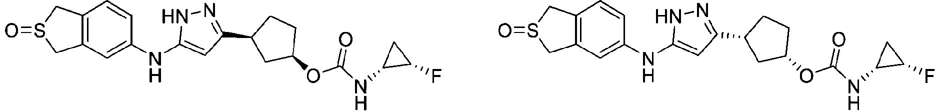

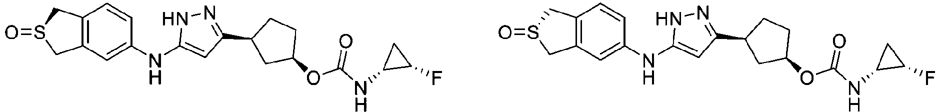

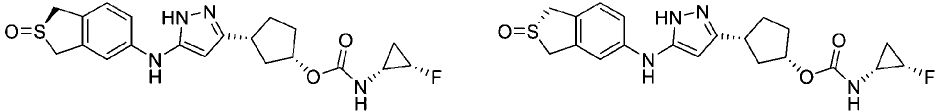

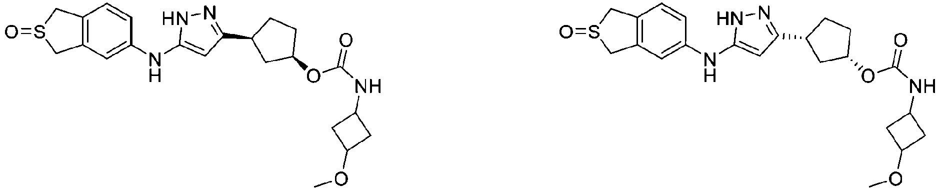

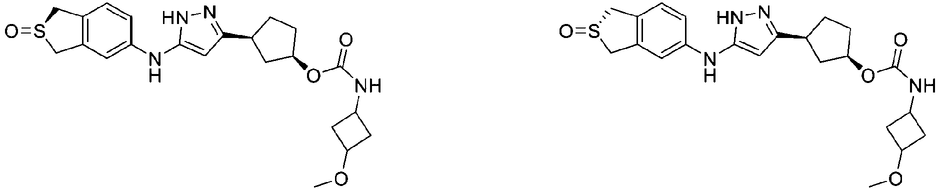

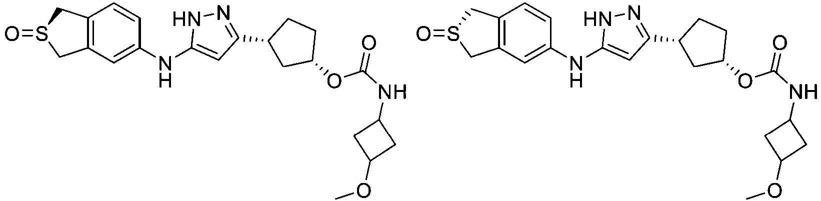

16. A pharmaceutical composition, comprising a therapeutically or prophylactically effective amount of the compound, the isomer thereof, or the pharmaceutically acceptable salt thereof according to any one of claims 1-15, and optionally further comprising a pharmaceutically acceptable excipient.

17. Use of the compound, the isomer thereof, or the pharmaceutically acceptable salt thereof according to any one of claims 1-15, or the pharmaceutical composition according to claim 16 for preparing a medicament for treating or preventing a disease.
